# EUROPEAN PATENT SPECIFICATION

(11) **EP 2 751 264 B1**
(45) Date of publication and mention of the grant of the patent: **27.12.2017**
(21) Application number: 12828073.2
(22) Date of filing: 31.08.2012
(51) Int. Cl.: C12N 9/22, C12P 19/34

(54) **COMPOSITIONS AND METHODS RELATING TO VARIANT DNA POLYMERASES AND SYNTHETIC DNA POLYMERASES**
ZUSAMMENSETZUNGEN UND VERFAHREN IM ZUSAMMENHANG MIT VARIANTEN DNA-POLYMERASEN UND SYNTHETISCHEN DNA POLYMERASEN
COMPOSITIONS ET PROCÉDÉS ASSOCIÉS À DES VARIANTS D'ADN POLYMÉRASES ET D'ADN POLYMÉRASES SYNTHÉTIQUES

(30) Priority: 01.09.2011 US 201161530273 P; 01.03.2012 US 201261605484 P
(43) Date of publication of application: 09.07.2014
(73) Proprietor: NEW ENGLAND BIOLABS, INC., Ipswich, MA 01938-2723 (US)
(72) Inventor: ONG, Jennifer, Salem, MA 01970 (US); EVANS, Jr., Thomas, C., Topsfield, MA 01983 (US); TANNER, Nathan, Peabody, MA 01960 (US)
(74) Representative: Mathys & Squire LLP
(86) International application number: PCT/US2012/053330
(87) International publication number: WO 2013/033528

(56) References cited:
- WO-A2-02/101004
- US-A1- 2003 180 737
- NOTOMI T ET AL: "Loop-mediated isothermal amplification of DNA", NUCLEIC ACIDS RESEARCH, OXFORD UNIVERSITY PRESS, GB, vol. 28, no. 12, 1 December 2000 (2000-12-01), pages i-vii, XP007905272, ISSN: 0305-1048, DOI: 10.1093/NAR/28.12.E63
- DATABASE UNIPROT [Online] 31 May 2011 FARASHAHI ET AL., XP003032842 Database accession no. A2TF40
- DATABASE UNIPROT [Online] 28 June 2011 CAGLAYAN ET AL., XP003032843 Database accession no. B6E9X1

### SEQUENCE LISTING

<110> New England Biolabs
<120> Compositions and Methods Relating to Varients of Bst DNA Polymerase
<130> NEB-349-PCT
<140> 61/530,273
   <141> 2011-09-01
<160> 147
<170> PatentIn version 3.5
<210> 1
   <211> 587
   <212> PRT
   <213> Geobacillus sp. G11MC16
<400> 1
   Ala Glu Gly Glu Lys Pro Leu Glu Glu Met Glu Phe Ala Ile Val Asp
   1               5                   10                  15
   Val Ile Thr Glu Glu Met Leu Ala Asp Lys Ala Ala Leu Val Val Glu
               20                  25                  30
   Val Met Glu Glu Asn Tyr His Asp Ala Pro Ile Val Gly Ile Ala Leu
           35                  40                  45
   Val Asn Glu His Gly Arg Phe Phe Met Arg Pro Glu Thr Ala Leu Ala
       50                  55                  60
   Asp Ser Gln Phe Leu Ala Trp Leu Ala Asp Glu Thr Lys Lys Lys Ser
   65                  70                  75                  80
   Met Phe Asp Ala Lys Arg Ala Val Val Ala Leu Lys Trp Lys Gly Ile
                   85                  90                  95
   Glu Leu Arg Gly Val Ala Phe Asp Leu Leu Leu Ala Ala Tyr Leu Leu
               100                 105                 110
   Asn Pro Ala Gln Asp Ala Gly Asp Ile Ala Ala Val Ala Lys Met Lys
           115                 120                 125
   Gln Tyr Glu Ala Val Arg Ser Asp Glu Ala Val Tyr Gly Lys Gly Val
       130                 135                 140
   Lys Arg Ser Leu Pro Asp Glu Gln Thr Leu Ala Glu His Leu Val Arg
   145                 150                 155                 160
   Lys Ala Ala Ala Ile Trp Ala Leu Glu Gln Pro Phe Met Asp Asp Leu
                   165                 170                 175
   Arg Asn Asn Glu Gln Asp Gln Leu Leu Thr Lys Leu Glu Gln Pro Leu
               180                 185                 190
   Ala Ala Ile Leu Ala Glu Met Glu Phe Thr Gly Val Asn Val Asp Thr
           195                 200                 205
   Lys Arg Leu Glu Gln Met Gly Ser Glu Leu Ala Glu Gln Leu Arg Ala
       210                 215                 220
   Ile Glu Gln Arg Ile Tyr Glu Leu Ala Gly Gln Glu Phe Asn Ile Asn
   225                 230                 235                 240
   Ser Pro Lys Gln Leu Gly Val Ile Leu Phe Glu Lys Leu Gln Leu Pro
                   245                 250                 255
   Val Leu Lys Lys Thr Lys Thr Gly Tyr Ser Thr Ser Ala Asp Val Leu
               260                 265                 270
   Glu Lys Leu Ala Pro His His Glu Ile Val Glu Asn Ile Leu His Tyr
           275                 280                 285
   Arg Gln Leu Gly Lys Leu Gln Ser Thr Tyr Ile Glu Gly Leu Leu Lys
       290                 295                 300
   Val Val Arg Pro Asp Thr Gly Lys Val His Thr Met Phe Asn Gln Ala
   305                 310                 315                 320
   Leu Thr Gln Thr Gly Arg Leu Ser Ser Ala Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Pro Asp Trp Leu Ile Phe Ala Ala Asp Tyr Ser Gln Ile
           355                 360                 365
   Glu Leu Arg Val Leu Ala His Ile Ala Asp Asp Asp Asn Leu Ile Glu
       370                 375                 380
   Ala Phe Gln Arg Asp Leu Asp Ile His Thr Lys Thr Ala Met Asp Ile
   385                 390                 395                 400
   Phe His Val Ser Glu Glu Glu Val Thr Ala Asn Met Arg Arg Gln Ala
                   405                 410                 415
   Lys Ala Val Asn Phe Gly Ile Val Tyr Gly Ile Ser Asp Tyr Gly Leu
               420                 425                 430
   Ala Gln Asn Leu Asn Ile Thr Arg Lys Glu Ala Ala Glu Phe Ile Glu
           435                 440                 445
   Arg Tyr Phe Ala Ser Phe Pro Gly Val Lys Gln Tyr Met Glu Asn Ile
       450                 455                 460
   Val Gln Glu Ala Lys Gln Lys Gly Tyr Val Thr Thr Leu Leu His Arg
   465                 470                 475                 480
   Arg Arg Tyr Leu Pro Asp Ile Thr Ser Arg Asn Phe Asn Val Arg Ser
                   485                 490                 495
   Phe Ala Glu Arg Thr Ala Met Asn Thr Pro Ile Gln Gly Ser Ala Ala
               500                 505                 510
   Asp Ile Ile Lys Lys Ala Met Ile Asp Leu Ala Ala Arg Leu Lys Glu
           515                 520                 525
   Glu Gln Leu Gln Ala Arg Leu Leu Leu Gln Val His Asp Glu Leu Ile
       530                 535                 540
   Leu Glu Ala Pro Lys Glu Glu Ile Glu Arg Leu Cys Glu Leu Val Pro
   545                 550                 555                 560
   Glu Val Met Glu Gln Ala Val Thr Leu Arg Val Pro Leu Lys Val Asp
                   565                 570                 575
   Tyr His Tyr Gly Pro Thr Trp Tyr Asp Ala Lys
               580                 585
<210> 2
   <211> 586
   <212> PRT
   <213> Geobacillus stearothermophilus
<400> 2
   Ala Glu Gly Arg Lys Pro Leu Glu Glu Met Glu Phe Ala Ile Val Asp
   1               5                   10                  15
   Val Ile Thr Glu Glu Met Leu Ala Asp Lys Ala Ala Leu Val Val Glu
               20                  25                  30
   Val Met Glu Glu Asn Tyr His Asp Ala Pro Ile Val Gly Ile Ala Leu
           35                  40                  45
   Val Asn Glu His Gly Arg Phe Phe Met Arg Pro Glu Thr Ala Leu Ala
       50                  55                  60
   Asp Ser Gln Phe Leu Ala Trp Leu Ala Asp Glu Thr Lys Lys Lys Ser
   65                  70                  75                  80
   Met Phe Asp Ala Lys Arg Ala Val Val Ala Leu Lys Trp Lys Gly Ile
                   85                  90                  95
   Asp Val Arg Gly Val Ala Phe Asp Leu Leu Leu Ala Ala Tyr Leu Leu
               100                 105                 110
   Asn Pro Ala Gln Asp Ala Gly Asp Ile Ala Ala Val Ala Lys Met Lys
           115                 120                 125
   Gln Tyr Glu Ala Val Arg Ser Asp Glu Ala Val Tyr Gly Lys Gly Val
       130                 135                 140
   Lys Arg Ser Leu Pro Asp Glu Gln Thr Leu Ala Glu His Leu Val Arg
   145                 150                 155                 160
   Lys Ala Ala Ala Ile Trp Ala Leu Glu Gln Pro Phe Met Asp Asp Leu
                   165                 170                 175
   Arg Asn Asn Glu Gln Asp Gln Leu Leu Thr Lys Leu Glu Gln Pro Leu
               180                 185                 190
   Ala Ala Ile Leu Ala Glu Met Glu Phe Thr Gly Val Asn Val Asp Thr
           195                 200                 205
   Lys Arg Leu Glu Gln Met Gly Ser Glu Leu Ala Glu Gln Leu Arg Ala
       210                 215                 220
   Ile Glu Gln Arg Ile Tyr Glu His Ala Gly Gln Glu Phe Asn Ile Asn
   225                 230                 235                 240
   Ser Pro Lys Gln Leu Gly Val Ile Leu Phe Glu Lys Leu Gln Leu Pro
                   245                 250                 255
   Val Leu Lys Lys Thr Lys Thr Gly Tyr Ser Thr Ser Ala Asp Val Leu
               260                 265                 270
   Glu Lys Leu Ala Pro His His Glu Ile Val Glu Asn Ile Leu His Tyr
           275                 280                 285
   Arg Gln Leu Gly Lys Leu Gln Ser Thr Tyr Ile Glu Gly Leu Leu Lys
       290                 295                 300
   Val Val Arg Pro Asp Thr Gly Lys Val His Thr Met Phe Asn Gln Thr
   305 310 315 320
   Leu Thr Gln Thr Gly Arg Leu Ser Ser Ala Glu Pro Asn Leu Gln Asn
   325 330 335
   Ile Pro Ile Arg Leu Glu Glu Gly Arg Lys Ile Arg Gln Ala Phe Val
   340 345 350
   Pro Ser Glu Pro Asp Trp Leu Ile Phe Ala Ala Asp Tyr Ser Gln Ile
   355 360 365
   Glu Leu Arg Val Leu Ala His Ile Ala Asp Asp Asp Asn Leu Ile Glu
   370 375 380
   Ala Phe Gln Arg Asp Leu Asp Ile His Thr Lys Thr Ala Met Asp Ile
   385 390 395 400
   Phe His Val Ser Glu Glu Glu Val Thr Ala Asn Met Arg Arg Gln Ala
   405 410 415
   Lys Ala Val Asn Phe Gly Ile Val Tyr Gly Ile Ser Asp Tyr Gly Leu
   420 425 430
   Ala Gln Asn Leu Asn Ile Thr Arg Lys Glu Ala Ala Glu Phe Ile Glu
   435 440 445
   Arg Tyr Phe Ala Ser Phe Pro Gly Val Arg Arg Tyr Met Glu Asn Ile
   450 455 460
   Val Gln Glu Ala Lys Gln Lys Gly Tyr Val Thr Thr Leu Leu His Arg
   465 470 475 480
   Arg Arg Tyr Leu Pro Asp Ile Thr Ser Arg Asn Phe Asn Val Arg Ser
   485 490 495
   Phe Ala Glu Arg Thr Ala Met Asn Thr Pro Ile Gln Gly Ser Ala Ala
   500 505 510
   Asp Ile Ile Lys Lys Ala Met Ile Asp Leu Ala Ala Arg Leu Lys Glu
   515 520 525
   Glu Gln Leu Gln Ala Arg Leu Leu Leu Gln Val His Asp Glu Leu Ile
   530 535 540
   Leu Glu Ala Pro Lys Glu Glu Ile Glu Arg Leu Cys Glu Leu Val Pro
   545 550 555 560
   Glu Val Met Glu Gln Ala Val Ser Ser Val Pro Leu Lys Val Asp Tyr
   565 570 575
   His Tyr Gly Pro Thr Trp Tyr Asp Ala Lys
   580 585
<210> 3
   <211> 587
   <212> PRT
   <213> Geobacillus so, MKK (2005)
<400> 3
   Ala Glu Asp Glu Thr Pro Leu Met Glu Met Glu Phe Val Ile Ala Asp
   1               5                   10                  15
   Gly Ile Thr Asp Glu Met Leu Ala Asp Lys Ala Ala Leu Val Val Glu
               20                  25                  30
   Val Met Glu Glu Asn Tyr His Asp Ala Pro Ile Val Gly Ile Ala Leu
           35                  40                  45
   Val Asn Glu His Gly Arg Phe Phe Leu Arg Ala Glu Met Ala Leu Ala
       50                  55                  60
   Asp Ser Gln Phe Leu Ala Trp Leu Ala Asp Glu Thr Lys Lys Lys Ser
   65                  70                  75                  80
   Met Phe Asp Ala Lys Arg Ala Ala Val Ala Leu Lys Trp Lys Gly Ile
                   85                  90                  95
   Glu Leu Arg Gly Val Ala Phe Asp Leu Leu Leu Ala Ala Tyr Leu Leu
               100                 105                 110
   Asn Pro Ala Gln Asp Ala Gly Asp Val Ala Ala Val Ala Lys Met Lys
           115                 120                 125
   Gln Tyr Glu Ala Val Arg Pro Asp Glu Ala Val Tyr Gly Lys Gly Ala
       130                 135                 140
   Lys Arg Ser Leu Pro Asp Glu Pro Thr Leu Ala Glu His Leu Val Arg
   145                 150                 155                 160
   Lys Ala Ala Ala Ile Trp Ala Leu Glu Arg Pro Phe Leu Asp Glu Leu
                   165                 170                 175
   Arg Ser Asn Glu Gln Asp Gly Leu Leu Ile Lys Leu Glu Gln Pro Leu
               180                 185                 190
   Ala Thr Ile Leu Ala Glu Met Glu Phe Thr Gly Ile Lys Val Asp Thr
           195                 200                 205
   Lys Arg Leu Glu Gln Met Gly Ser Glu Leu Ala Glu Gln Leu Arg Ala
       210                 215                 220
   Val Glu Gln Arg Ile Tyr Glu Leu Ala Gly Gln Glu Phe Asn Ile Asn
   225                 230                 235                 240
   Ser Pro Lys Gln Leu Gly Ile Ile Leu Phe Glu Lys Leu Gln Leu Pro
                   245                 250                 255
   Val Leu Lys Lys Thr Lys Thr Gly Tyr Ser Thr Ser Ala Asp Val Leu
               260                 265                 270
   Glu Lys Leu Ala Pro His His Glu Ile Val Glu Asn Ile Leu His Tyr
           275                 280                 285
   Arg Gln Leu Gly Lys Leu Gln Ser Thr Tyr Ile Glu Gly Leu Leu Lys
       290                 295                 300
   Val Val His Pro Asp Thr Gly Lys Val His Thr Met Phe Asn Gln Ala
   305                 310                 315                 320
   Leu Thr Gln Thr Gly Arg Leu Ser Ser Ala Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Pro Asp Trp Leu Ile Phe Ala Ala Asp Tyr Ser Gln Ile
           355                 360                 365
   Glu Leu Arg Val Leu Ala His Ile Ala Asp Asp Asp Asn Leu Ile Glu
       370                 375                 380
   Ala Phe Arg Arg Asp Leu Asp Ile His Thr Lys Thr Ala Met Asp Ile
   385                 390                 395                 400
   Phe His Val Ser Glu Glu Glu Val Thr Ala Thr Met Arg Arg Gln Ala
                   405                 410                 415
   Lys Ala Val Asn Phe Gly Ile Val Tyr Gly Ile Ser Asp Tyr Gly Leu
               420                 425                 430
   Ala Gln Asn Leu Asn Ile Thr Arg Lys Glu Ala Ala Glu Phe Ile Glu
   435 440 445
   Arg Tyr Phe Ala Ser Phe Pro Gly Val Lys Arg Tyr Met Glu Thr Ile
   450 455 460
   Val Gln Glu Ala Lys Gln Lys Gly Tyr Val Thr Thr Leu Leu His Arg
   465 470 475 480
   Arg Arg Tyr Leu Pro Asp Ile Thr Ser Arg Asn Phe Asn Val Arg Ser
   485 490 495
   Phe Ala Glu Arg Thr Ala Met Asn Thr Pro Ile Gln Gly Ser Ala Ala
   500 505 510
   Asp Ile Ile Lys Lys Ala Met Ile Asp Leu Ala Ala Arg Leu Lys Glu
   515 520 525
   Glu Arg Leu Gln Ala Arg Leu Leu Leu Gln Val His Asp Glu Leu Ile
   530 535 540
   Leu Glu Ala Pro Lys Glu Glu Met Glu Arg Leu Cys Gln Leu Val Pro
   545 550 555 560
   Glu Val Met Glu Gln Ala Val Ala Leu Arg Val Pro Leu Lys Val Asp
   565 570 575
   Tyr His Tyr Gly Pro Thr Trp Tyr Asp Ala Lys
   580 585
<210> 4
   <211> 587
   <212> PRT
   <213> Geobacillus sp. LH8
<400> 4
   Ala Glu Asp Glu Thr Pro Leu Met Glu Met Glu Phe Val Ile Ala Asp
   1 5 10 15
   Gly Ile Thr Asp Glu Met Leu Ala Asp Lys Ala Ala Leu Val Val Glu
   20 25 30
   Val Met Glu Glu Asn Tyr His Asp Ala Pro Ile Val Gly Ile Ala Leu
   35 40 45
   Val Asn Glu His Gly Arg Phe Phe Leu Arg Ala Glu Met Ala Leu Ala
   50 55 60
   Asp Ser Gln Phe Leu Ala Trp Leu Ala Asp Glu Thr Lys Lys Lys Ser
   65 70 75 80
   Met Phe Asp Ala Lys Arg Ala Ala Val Ala Leu Lys Trp Lys Gly Ile
   85 90 95
   Glu Leu Arg Gly Val Ala Phe Asp Leu Leu Leu Ala Ala Tyr Leu Leu
   100 105 110
   Asn Pro Ala Gln Asp Ala Gly Asp Val Ala Ala Val Ala Lys Met Lys
   115 120 125
   Gln Tyr Glu Ala Val Arg Pro Asp Glu Ala Val Tyr Gly Lys Gly Ala
   130 135 140
   Lys Arg Ser Leu Pro Asp Glu Pro Thr Leu Ala Glu His Leu Val Arg
   145 150 155 160
   Lys Ala Ala Ala Ile Trp Ala Leu Glu Arg Pro Ser Leu Asp Glu Leu
   165 170 175
   Arg Ser Asn Glu Gln Asp Gly Leu Leu Ile Lys Leu Glu Gln Pro Leu
   180 185 190
   Ala Thr Ile Leu Ala Glu Met Glu Phe Thr Gly Ile Lys Val Asp Thr
   195 200 205
   Lys Arg Leu Glu Gln Met Gly Ser Glu Leu Ala Glu Gln Leu Arg Ala
   210 215 220
   Val Glu Gln Arg Ile Tyr Glu Leu Ala Gly Gln Glu Phe Asn Ile Asn
   225 230 235 240
   Ser Pro Lys Gln Leu Gly Ile Ile Leu Phe Glu Lys Leu Gln Leu Pro
   245 250 255
   Val Leu Lys Lys Thr Lys Thr Gly Tyr Ser Thr Ser Ala Asp Val Leu
   260 265 270
   Glu Lys Leu Ala Pro His His Glu Ile Val Glu Asn Ile Leu His Tyr
   275 280 285
   Arg Gln Leu Gly Lys Leu Gln Ser Thr Tyr Ile Glu Gly Leu Leu Lys
   290 295 300
   Val Val His Pro Asp Thr Gly Lys Val His Thr Met Phe Asn Gln Ala
   305 310 315 320
   Leu Thr Gln Thr Gly Arg Leu Ser Ser Ala Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Pro Asp Trp Leu Ile Phe Ala Ala Asp Tyr Ser Gln Ile
           355                 360                 365
   Glu Leu Arg Val Leu Ala His Ile Ala Asp Asp Asp Asn Leu Ile Glu
       370                 375                 380
   Ala Phe Arg Arg Asp Leu Asp Ile His Thr Lys Thr Ala Met Asp Ile
   385                 390                 395                 400
   Phe His Val Ser Glu Glu Glu Val Thr Ala Thr Met Arg Arg Gln Ala
                   405                 410                 415
   Lys Ala Val Asn Phe Gly Val Val Tyr Gly Ile Ser Asp Tyr Gly Leu
               420                 425                 430
   Ala Gln Asn Leu Asn Ile Thr Arg Lys Glu Ala Ala Glu Phe Ile Glu
           435                 440                 445
   Arg Tyr Phe Ala Ser Phe Pro Gly Val Lys Arg Tyr Met Glu Thr Ile
       450                 455                 460
   Val Gln Glu Ala Lys Gln Lys Gly Tyr Val Thr Thr Leu Leu His Arg
   465                 470                 475                 480
   Arg Arg Tyr Leu Pro Asp Ile Thr Ser Arg Asn Phe Asn Val Arg Ser
                   485                 490                 495
   Phe Ala Glu Arg Thr Ala Met Asn Thr Pro Ile Gln Gly Ser Ala Ala
               500                 505                 510
   Asp Ile Ile Lys Lys Ala Met Ile Asp Leu Ala Ala Arg Leu Lys Glu
           515                 520                 525
   Glu Arg Leu Gln Ala Arg Leu Leu Leu Gln Val His Asp Glu Leu Ile
       530                 535                 540
   Leu Glu Ala Pro Lys Glu Glu Met Glu Arg Leu Cys Gln Leu Val Pro
   545                 550                 555                 560
   Glu Val Met Glu Gln Ala Val Ala Leu Arg Val Pro Leu Lys Val Asp
                   565                 570                 575
   Tyr His Tyr Gly Pro Thr Trp Tyr Asp Ala Lys
                   580                 585
<210> 5
   <211> 587
   <212> PRT
   <213> Geobacillus kaue
<400> 5
   Ala Glu Asp Glu Thr Pro Leu Met Glu Met Glu Phe Val Ala Ala Asp
   1               5                   10                  15
   Gly Ile Thr Asp Glu Met Leu Ala Asp Lys Ala Ala Leu Val Val Glu
               20                  25                  30
   Val Met Glu Glu Asn Tyr His Asp Ala Pro Ile Val Gly Ile Ala Leu
           35                  40                  45
   Val Asn Glu His Gly Arg Phe Phe Leu Arg Ala Glu Met Ala Leu Ala
       50                  55                  60
   Asp Pro Gln Phe Val Ala Trp Leu Ala Asp Glu Thr Lys Lys Lys Ser
   65                  70                  75                  80
   Met Phe Asp Ala Lys Arg Ala Ser Val Ala Leu Lys Trp Lys Gly Ile
                   85                  90                  95
   Glu Leu Arg Gly Val Ala Phe Asp Leu Leu Leu Ala Ala Tyr Leu Leu
               100                 105                 110
   Asn Pro Ala Gln Asp Ala Gly Asp Val Ala Ala Val Ala Lys Met Lys
           115                 120                 125
   Gln Tyr Glu Ala Val Arg Pro Asp Glu Ala Val Tyr Gly Lys Gly Ala
       130                 135                 140
   Lys Arg Ser Leu Pro Asp Glu Pro Thr Leu Ala Glu His Leu Val Arg
   145                 150                 155                 160
   Lys Ala Ala Ala Ile Trp Ala Leu Glu Arg Pro Phe Leu Asp Glu Leu
                   165                 170                 175
   Arg Ser Asn Glu Gln Asp Glu Leu Leu Ile Lys Leu Glu Gln Pro Leu
               180                 185                 190
   Ala Thr Ile Leu Ala Glu Met Glu Phe Thr Gly Val Lys Val Asp Thr
           195                 200                 205
   Lys Arg Leu Glu Gln Met Gly Ser Glu Leu Ala Glu Gln Leu Gly Ala
       210                 215                 220
   Ile Glu Gln Arg Ile Tyr Glu Leu Ala Gly Gln Glu Phe Asn Ile Asn
   225                 230                 235                 240
   Ser Pro Lys Gln Leu Gly Ile Ile Leu Phe Glu Lys Leu Gln Leu Pro
                   245                 250                 255
   Val Leu Lys Lys Thr Lys Thr Gly Tyr Ser Thr Ser Ala Asp Val Leu
               260                 265                 270
   Glu Lys Leu Ala Pro His His Glu Ile Val Glu Asn Ile Leu His Tyr
           275                 280                 285
   Arg Gln Leu Gly Lys Leu Gln Ser Thr Tyr Ile Glu Gly Leu Leu Lys
       290                 295                 300
   Val Val His His Asp Thr Gly Lys Val His Thr Met Phe Asn Gln Ala
   305                 310                 315                 320
   Leu Thr Gln Thr Gly Arg Leu Ser Ser Ala Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Pro Gly Trp Leu Ile Phe Ala Ala Asp Tyr Ser Gln Ile
           355                 360                 365
   Glu Leu Arg Val Leu Ala His Ile Ala Asp Asp Asp Asn Leu Ile Glu
       370                 375                 380
   Ala Phe Arg Arg Asp Leu Asp Ile His Ala Lys Thr Ala Met Asp Ile
   385                 390                 395                 400
   Phe His Val Ser Glu Glu Glu Val Thr Ala Thr Met Arg Arg Gln Ala
                   405                 410                 415
   Lys Ala Val Asn Phe Gly Ile Val Tyr Gly Ile Ser Asp Tyr Gly Leu
               420                 425                 430
   Ala Gln Asn Leu Asn Ile Thr Arg Lys Glu Ala Ala Glu Phe Ile Glu
           435                 440                 445
   Arg Tyr Phe Ala Ser Phe Pro Gly Val Lys Arg Tyr Met Glu Thr Ile
           450                 455                 460
   Val Gln Glu Ala Lys Gln Lys Gly Tyr Val Thr Thr Leu Leu His Arg
   465                 470                 475                 480
   Arg Arg Tyr Leu Pro Asp Ile Thr Ser Arg Asn Phe Asn Val Arg Ser
                   485                 490                 495
   Phe Ala Glu Arg Thr Ala Met Asn Thr Pro Ile Gln Gly Ser Ala Ala
               500                 505                 510
   Asp Ile Ile Lys Lys Ala Met Ile Asp Leu Ala Ala Arg Leu Lys Glu
           515                 520                 525
   Glu Arg Leu Gln Ala Arg Leu Leu Leu Gln Val His Asp Glu Leu Ile
       530                 535                 540
   Leu Glu Ala Pro Lys Glu Glu Met Glu Arg Leu Cys Gln Leu Val Pro
   545                 550                 555                 560
   Glu Val Met Glu Gln Ala Val Glu Leu Arg Val Pro Leu Lys Val Asp
                   565                 570                 575
   Tyr His Tyr Gly Pro Thr Trp Tyr Asp Ala Lys
               580                 585
<210> 6
   <211> 587
   <212> PRT
   <213> Geobacillus anatolicus
<400> 6
   Ala Glu Asp Glu Thr Pro Leu Met Glu Met Glu Phe Val Ala Ala Asp
   1               5                   10                  15
   Gly Ile Thr Asp Glu Met Leu Ala Asp Lys Ala Ala Leu Val Val Glu
               20                  25                  30
   Val Met Glu Glu Asn Tyr His Asp Ala Pro Ile Val Gly Ile Ala Leu
           35                  40                  45
   Val Asn Glu His Gly Arg Phe Phe Leu Arg Ala Glu Met Ala Leu Ala
       50                  55                  60
   Asp Pro Gln Phe Val Ala Trp Leu Ala Asp Glu Thr Lys Lys Lys Ser
   65                  70                  75                  80
   Met Phe Asp Ala Lys Arg Ala Ser Val Ala Leu Lys Trp Lys Gly Ile
                   85                  90                  95
   Glu Leu Arg Gly Val Ala Phe Asp Leu Leu Leu Ala Ala Tyr Leu Leu
               100                 105                 110
   Asn Pro Ala Gln Asp Ala Gly Asp Val Ala Ala Val Ala Lys Met Lys
           115                 120                 125
   Gln Tyr Glu Ala Val Arg Pro Asp Glu Ala Val Tyr Gly Lys Gly Ala
       130                 135                 140
   Lys Arg Ser Leu Pro Asp Glu Pro Thr Leu Ala Glu His Leu Val Arg
   145                 150                 155                 160
   Lys Ala Ala Ala Ile Trp Ala Leu Glu Arg Pro Phe Leu Asp Glu Leu
                   165                 170                 175
   Arg Ser Asn Glu Gln Asp Glu Leu Leu Ile Lys Leu Glu Gln Pro Leu
               180                 185                 190
   Ala Thr Ile Leu Ala Glu Met Glu Phe Thr Gly Val Lys Val Asp Thr
           195                 200                 205
   Lys Arg Leu Glu Gln Met Gly Ser Glu Leu Ala Glu Gln Leu Gly Ala
       210                 215                 220
   Val Glu Gln Arg Ile Tyr Glu Leu Ala Gly Gln Glu Phe Asn Ile Asn
   225                 230                 235                 240
   Ser Pro Lys Gln Leu Gly Ile Ile Leu Phe Glu Lys Leu Gln Leu Pro
                   245                 250                 255
   Val Leu Lys Lys Thr Lys Thr Gly Tyr Ser Thr Ser Ala Asp Val Leu
               260                 265                 270
   Glu Lys Leu Ala Pro His His Glu Ile Val Glu Asn Ile Leu His Tyr
           275                 280                 285
   Arg Gln Leu Gly Lys Leu Gln Ser Thr Tyr Ile Glu Gly Leu Leu Lys
       290                 295                 300
   Val Val His His Asp Thr Gly Lys Val His Thr Met Phe Asn Gln Ala
   305                 310                 315                 320
   Leu Thr Gln Thr Gly Arg Leu Ser Ser Ala Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Pro Gly Trp Leu Ile Phe Ala Ala Asp Tyr Ser Gln Ile
           355                 360                 365
   Glu Leu Arg Val Leu Ala His Ile Ala Asp Asp Asp Asn Leu Ile Glu
       370                 375                 380
   Ala Phe Arg Arg Asp Leu Asp Ile His Thr Lys Thr Ala Met Asp Ile
   385                 390                 395                 400
   Phe His Val Ser Glu Glu Glu Val Thr Ala Thr Met Arg Arg Gln Ala
                   405                 410                 415
   Lys Ala Val Asn Phe Gly Ile Val Tyr Gly Ile Ser Asp Tyr Gly Leu
               420                 425                 430
   Ala Gln Asn Leu Asn Ile Thr Arg Lys Glu Ala Ala Glu Phe Ile Glu
           435                 440                 445
   Arg Tyr Phe Ala Ser Phe Pro Gly Val Lys Arg Tyr Met Glu Thr Ile
       450                 455                 460
   Val Gln Glu Ala Lys Gln Lys Gly Tyr Val Thr Thr Leu Leu His Arg
   465                 470                 475                 480
   Arg Arg Tyr Phe Pro Asp Ile Thr Ser Arg Asn Phe Asn Val Arg Ser
                   485                 490                 495
   Phe Ala Glu Arg Thr Ala Met Asn Thr Pro Ile Gln Gly Ser Ala Ala
               500                 505                 510
   Asp Ile Ile Lys Lys Ala Met Ile Asp Leu Ala Ala Arg Leu Lys Glu
           515                 520                 525
   Glu Arg Leu Gln Ala Arg Leu Leu Leu Gln Val His Asp Glu Leu Ile
       530                 535                 540
   Leu Glu Ala Pro Lys Glu Glu Met Glu Arg Leu Cys Gln Leu Val Pro
   545                 550                 555                 560
   Glu Val Met Glu Gln Ala Val Glu Leu Arg Val Pro Leu Lys Val Asp
                   565                 570                 575
   Tyr His Tyr Gly Pro Thr Trp Tyr Asp Pro Lys
               580                 585
<210> 7
   <211> 587
   <212> PRT
   <213> Geobacillus bogazici
<400> 7
   Ala Glu Asp Glu Thr Pro Leu Met Glu Met Glu Phe Val Ala Ala Asp
   1               5                   10                  15
   Gly Ile Thr Asp Glu Met Leu Ala Asp Lys Ala Ala Leu Val Val Glu
               20                  25                  30
   Val Met Glu Glu Asn Tyr His Asp Ala Pro Ile Val Gly Ile Ala Leu
           35                  40                  45
   Val Asn Glu His Gly Arg Phe Phe Leu Arg Ala Glu Met Ala Leu Ala
       50                  55                  60
   Asp Pro Gln Phe Val Ala Trp Leu Ala Asp Glu Thr Lys Lys Lys Ser
   65                  70                  75                  80
   Met Phe Asp Ala Lys Arg Ala Ser Val Ala Leu Lys Trp Lys Gly Ile
                   85                  90                  95
   Glu Leu Arg Gly Val Ala Phe Asp Leu Leu Leu Ala Ala Tyr Leu Leu
               100                 105                 110
   Asn Pro Ala Gln Asp Ala Gly Asp Val Ala Ala Val Ala Lys Met Lys
           115                 120                 125
   Gln Tyr Glu Ala Val Arg Pro Asp Glu Ala Val Tyr Gly Lys Gly Ala
       130                 135                 140
   Lys Arg Ser Leu Pro Asp Glu Pro Thr Leu Ala Glu His Leu Val Arg
   145                 150                 155                 160
   Lys Ala Ala Ala Ile Trp Ala Leu Glu Arg Pro Phe Leu Asp Glu Leu
                   165                 170                 175
   Arg Ser Asn Glu Gln Asp Glu Leu Leu Ile Lys Leu Glu Gln Pro Leu
               180                 185                 190
   Ala Thr Ile Leu Ala Glu Met Glu Phe Thr Gly Val Lys Val Asp Thr
           195                 200                 205
   Lys Arg Leu Glu Gln Met Gly Ser Glu Leu Ala Glu Gln Leu Gly Ala
       210                 215                 220
   Val Glu Gln Arg Ile Tyr Glu Leu Ala Gly Gln Glu Phe Asn Ile Asn
   225                 230                 235                 240
   Ser Pro Lys Gln Leu Gly Ile Ile Leu Phe Glu Lys Leu Gln Leu Pro
                   245                 250                 255
   Val Leu Lys Lys Thr Lys Thr Gly Tyr Ser Thr Ser Ala Asp Val Leu
               260                 265                 270
   Glu Lys Leu Ala Pro His His Glu Ile Val Glu Asn Ile Leu His Tyr
           275                 280                 285
   Arg Gln Leu Gly Lys Leu Gln Ser Thr Tyr Ile Glu Gly Leu Leu Lys
       290                 295                 300
   Val Val His His Asp Thr Gly Lys Val His Thr Met Phe Asn Gln Ala
   305                 310                 315                 320
   Leu Thr Gln Thr Gly Arg Leu Ser Ser Ala Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Pro Gly Trp Leu Ile Phe Ala Ala Asp Tyr Ser Gln Ile
           355                 360                 365
   Glu Leu Arg Val Leu Ala His Ile Ala Asp Asp Asp Asn Leu Ile Glu
       370                 375                 380
   Ala Phe Arg Arg Asp Leu Asp Ile His Thr Lys Thr Ala Met Asp Ile
   385                 390                 395                 400
   Phe His Val Ser Glu Glu Glu Val Thr Ala Thr Met Arg Arg Gln Ala
                   405                 410                 415
   Lys Ala Val Asn Phe Gly Ile Val Tyr Gly Ile Ser Asp Tyr Gly Leu
               420                 425                 430
   Ala Gln Asn Leu Asn Ile Thr Arg Lys Glu Ala Ala Glu Phe Ile Glu
           435                 440                 445
   Arg Tyr Phe Ala Ser Phe Pro Gly Val Lys Arg Tyr Met Glu Asn Ile
       450                 455                 460
   Val Gln Glu Ala Lys Gln Lys Gly Tyr Val Thr Thr Leu Leu His Arg
   465                 470                 475                 480
   Arg Arg Tyr Leu Pro Asp Ile Thr Ser Arg Asn Phe Asn Val Arg Ser
                   485                 490                 495
   Phe Ala Glu Arg Met Ala Met Asn Thr Pro Ile Gln Gly Ser Ala Ala
               500                 505                 510
   Asp Ile Ile Lys Lys Ala Met Ile Asp Leu Ala Ala Arg Leu Lys Glu
           515                 520                 525
   Glu Arg Leu Gln Ala Arg Leu Leu Leu Gln Val His Asp Glu Leu Ile
       530                 535                 540
   Leu Glu Ala Pro Lys Glu Glu Met Glu Arg Leu Cys Arg Leu Val Pro
   545                 550                 555                 560
   Glu Val Met Glu Gln Ala Val Glu Leu Arg Val Pro Leu Lys Val Asp
                   565                 570                 575
   Tyr His Tyr Gly Pro Thr Trp Tyr Asp Pro Lys
               580                 585
<210> 8
   <211> 587
   <212> PRT
   <213> Geobacillus kaue
<400> 8
   Ala Glu Asp Glu Thr Pro Leu Met Glu Met Glu Phe Val Ala Ala Asp
   1               5                   10                  15
   Gly Ile Thr Asp Glu Met Leu Ala Asp Lys Ala Ala Leu Val Val Glu
               20                  25                  30
   Val Met Glu Glu Asn Tyr His Asp Ala Pro Ile Val Gly Ile Ala Leu
           35                  40                  45
   Val Asn Glu His Gly Arg Phe Ser Leu Arg Ala Glu Met Ala Leu Ala
       50                  55                  60
   Asp Pro Gln Phe Val Ala Trp Leu Ala Asp Glu Thr Lys Lys Lys Ser
   65                  70                  75                  80
   Met Phe Asp Ala Lys Arg Ala Ser Val Ala Leu Lys Trp Lys Gly Ile
                   85                  90                  95
   Glu Leu Arg Gly Val Ala Phe Asp Leu Leu Leu Ala Ala Tyr Leu Leu
               100                 105                 110
   Asn Pro Ala Gln Asp Ala Gly Asp Val Ala Ala Val Ala Lys Met Lys
           115                 120                 125
   Gln Tyr Glu Ala Val Arg Pro Asp Glu Ala Val Tyr Gly Lys Gly Ala
       130                 135                 140
   Lys Arg Ser Leu Pro Asp Glu Pro Thr Leu Ala Glu His Leu Val Arg
   145                 150                 155                 160
   Lys Ala Ala Ala Ile Trp Ala Leu Glu Arg Pro Phe Leu Asp Glu Leu
                   165                 170                 175
   Arg Ser Asn Glu Gln Asp Glu Leu Leu Ile Lys Leu Glu Gln Pro Leu
               180                 185                 190
   Ala Thr Ile Leu Ala Glu Met Glu Phe Thr Gly Val Lys Val Asp Thr
           195                 200                 205
   Lys Arg Leu Glu Gln Met Gly Ser Glu Leu Ala Glu Gln Leu Gly Ala
       210                 215                 220
   Ile Glu Gln Arg Ile Tyr Glu Leu Ala Gly Gln Glu Phe Asn Ile Asn
   225                 230                 235                 240
   Ser Pro Lys Gln Leu Gly Ile Ile Leu Phe Glu Lys Leu Gln Leu Pro
                   245                 250                 255
   Val Leu Lys Lys Thr Lys Thr Gly Tyr Ser Thr Ser Ala Asp Val Leu
               260                 265                 270
   Glu Lys Leu Ala Pro His His Glu Ile Val Glu Asn Ile Leu His Tyr
           275                 280                 285
   Arg Gln Leu Gly Lys Leu Gln Ser Thr Tyr Ile Glu Gly Leu Leu Lys
       290                 295                 300
   Val Val His His Asp Thr Gly Lys Val His Thr Met Phe Asn Gln Ala
   305                 310                 315                 320
   Leu Thr Gln Thr Gly Arg Leu Ser Ser Ala Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Pro Gly Trp Leu Ile Phe Ala Ala Asp Tyr Ser Gln Ile
               355                 360                 365
   Glu Leu Arg Val Leu Ala His Ile Ala Asp Asp Asp Asn Leu Ile Glu
       370                 375                 380
   Ala Phe Arg Arg Asp Leu Asp Ile His Thr Lys Thr Ala Met Asp Ile
   385                 390                 395                 400
   Phe His Val Ser Glu Glu Glu Val Thr Ala Thr Met Arg Arg Gln Ala
                   405                 410                 415
   Lys Ala Val Asn Phe Gly Ile Val Tyr Gly Ile Ser Asp Tyr Gly Leu
               420                 425                 430
   Ala Gln Asn Leu Asn Ile Thr Arg Lys Glu Ala Ala Glu Phe Ile Glu
           435                 440                 445
   Arg Tyr Phe Ala Ser Phe Pro Gly Val Lys Arg Tyr Met Glu Thr Ile
       450                 455                 460
   Val Gln Glu Ala Lys Gln Lys Gly Tyr Val Thr Thr Leu Leu His Arg
   465                 470                 475                 480
   Arg Arg Tyr Phe Pro Asp Ile Thr Ser Arg Asn Phe Asn Val Arg Ser
                   485                 490                 495
   Phe Ala Glu Arg Thr Ala Met Asn Thr Pro Ile Gln Gly Ser Ala Ala
               500                 505                 510
   Asp Ile Ile Lys Lys Ala Met Ile Asp Leu Ala Ala Arg Leu Lys Glu
           515                 520                 525
   Glu Arg Leu Gln Ala Arg Leu Leu Leu Gln Val His Asp Glu Leu Ile
       530                 535                 540
   Leu Glu Ala Pro Lys Glu Glu Met Glu Arg Leu Cys Gln Leu Val Pro
   545                 550                 555                 560
   Glu Val Met Glu Gln Ala Val Glu Leu Arg Val Pro Leu Lys Val Asp
                   565                 570                 575
   Tyr His Tyr Gly Pro Thr Trp Tyr Asp Pro Lys
               580                 585
<210> 9
   <211> 587
   <212> PRT
   <213> Anoxybacillus sp. NB
<400> 9
   Ala Glu Asp Glu Thr Pro Leu Met Glu Met Glu Phe Val Ala Ala Asp
   1               5                   10                  15
   Gly Ile Thr Asp Glu Met Leu Ala Asp Lys Ala Ala Leu Val Val Glu
               20                  25                  30
   Val Met Glu Glu Asn Tyr His Asp Ala Pro Ile Val Gly Ile Ala Leu
           35                  40                  45
   Val Asn Glu His Gly Arg Phe Ser Leu Arg Ala Glu Met Ala Leu Ala
       50                  55                  60
   Asp Pro Gln Phe Val Ala Trp Leu Ala Asp Glu Thr Lys Lys Lys Ser
   65                  70                  75                  80
   Met Phe Asp Ala Lys Arg Ala Ser Val Ala Leu Lys Trp Lys Gly Ile
                   85                  90                  95
   Glu Leu Arg Gly Val Ala Phe Asp Leu Leu Leu Ala Ala Tyr Leu Leu
               100                 105                 110
   Asn Pro Ala Gln Asp Ala Gly Asp Val Ala Ala Val Ala Lys Met Lys
           115                 120                 125
   Gln Tyr Glu Ala Val Arg Pro Asp Glu Ala Val Tyr Gly Lys Gly Ala
       130                 135                 140
   Lys Arg Ser Leu Pro Asp Glu Pro Thr Leu Ala Glu His Leu Val Arg
   145                 150                 155                 160
   Lys Ala Ala Ala Ile Trp Ala Leu Glu Arg Pro Phe Leu Asp Glu Leu
                   165                 170                 175
   Arg Ser Asn Glu Gln Asp Glu Leu Leu Ile Lys Leu Glu Gln Pro Leu
               180                 185                 190
   Ala Thr Ile Leu Ala Glu Met Glu Phe Thr Gly Val Lys Val Asp Thr
           195                 200                 205
   Lys Arg Leu Glu Gln Met Gly Ser Glu Leu Ala Glu Gln Leu Gly Ala
       210                 215                 220
   Val Glu Gln Arg Ile Tyr Glu Leu Ala Gly Gln Glu Phe Asn Ile Asn
   225                 230                 235                 240
   Ser Pro Lys Gln Leu Gly Ile Ile Leu Phe Glu Lys Leu Gln Leu Pro
                   245                 250                 255
   Val Leu Lys Lys Thr Lys Thr Gly Tyr Ser Thr Ser Ala Asp Val Leu
               260                 265                 270
   Glu Lys Leu Ala Pro His His Glu Ile Val Glu Asn Ile Leu His Tyr
           275                 280                 285
   Arg Gln Leu Gly Lys Leu Gln Ser Thr Tyr Ile Glu Gly Leu Leu Lys
       290                 295                 300
   Val Val His His Asp Thr Gly Lys Val His Thr Met Phe Asn Gln Ala
   305                 310                 315                 320
   Leu Thr Gln Thr Gly Arg Leu Ser Ser Ala Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Pro Gly Trp Leu Ile Phe Ala Ala Asp Tyr Ser Gln Ile
           355                 360                 365
   Glu Leu Arg Val Leu Ala His Ile Ala Asp Asp Asp Asn Leu Ile Glu
       370                 375                 380
   Ala Phe Arg Arg Asp Leu Asp Ile His Thr Lys Thr Ala Met Asp Ile
   385                 390                 395                 400
   Phe His Val Ser Glu Glu Glu Val Thr Ala Thr Met Arg Arg Gln Ala
                   405                 410                 415
   Lys Ala Val Asn Phe Gly Ile Val Tyr Gly Ile Ser Asp Tyr Gly Leu
               420                 425                 430
   Ala Gln Asn Leu Asn Ile Thr Arg Lys Glu Ala Ala Glu Phe Ile Glu
           435                 440                 445
   Arg Tyr Phe Ala Ser Phe Pro Gly Val Lys Arg Tyr Met Glu Thr Ile
       450                 455                 460
   Val Gln Glu Ala Lys Gln Lys Gly Tyr Val Thr Thr Leu Leu His Arg
   465                 470                 475                 480
   Arg Arg Tyr Phe Pro Asp Ile Thr Ser Arg Asn Phe Asn Val Arg Ser
                   485                 490                 495
   Phe Ala Glu Arg Thr Ala Met Asn Thr Pro Ile Gln Gly Ser Ala Ala
   500 505 510
   Asp Ile Ile Lys Lys Ala Met Ile Asp Leu Ala Ala Arg Leu Lys Glu
   515 520 525
   Glu Arg Leu Gln Ala Arg Leu Leu Leu Gln Val His Asp Glu Leu Ile
   530 535 540
   Leu Glu Ala Pro Lys Glu Glu Met Glu Arg Leu Cys Gln Leu Val Pro
   545 550 555 560
   Glu Val Met Glu Gln Ala Val Glu Leu Arg Val Pro Leu Lys Val Asp
   565 570 575
   Tyr His Tyr Gly Pro Thr Trp Tyr Asp Pro Lys
   580 585
<210> 10
   <211> 587
   <212> PRT
   <213> Geobacillus stearothermophilus
<400> 10
   Asp Glu Gly Glu Lys Pro Leu Ala Gly Met Asp Phe Ala Ile Ala Asp
   1 5 10 15
   Ser Val Thr Asp Glu Met Leu Ala Asp Lys Ala Ala Leu Val Val Glu
   20 25 30
   Val Val Gly Asp Asn Tyr His His Ala Pro Ile Val Gly Ile Ala Leu
   35 40 45
   Ala Asn Glu Arg Gly Arg Phe Phe Leu Arg Pro Glu Thr Ala Leu Ala
   50 55 60
   Asp Pro Lys Phe Leu Ala Trp Leu Gly Asp Glu Thr Lys Lys Lys Thr
   65 70 75 80
   Met Phe Asp Ser Lys Arg Ala Ala Val Ala Leu Lys Trp Lys Gly Ile
   85 90 95
   Glu Leu Arg Gly Val Val Phe Asp Leu Leu Leu Ala Ala Tyr Leu Leu
   100 105 110
   Asp Pro Ala Gln Ala Ala Gly Asp Val Ala Ala Val Ala Lys Met His
   115 120 125
   Gln Tyr Glu Ala Val Arg Ser Asp Glu Ala Val Tyr Gly Lys Gly Ala
   130 135 140
   Lys Arg Thr Val Pro Asp Glu Pro Thr Leu Ala Glu His Leu Val Arg
   145 150 155 160
   Lys Ala Ala Ala Ile Trp Ala Leu Glu Glu Pro Leu Met Asp Glu Leu
   165 170 175
   Arg Arg Asn Glu Gln Asp Arg Leu Leu Thr Glu Leu Glu Gln Pro Leu
   180 185 190
   Ala Gly Ile Leu Ala Asn Met Glu Phe Thr Gly Val Lys Val Asp Thr
   195 200 205
   Lys Arg Leu Glu Gln Met Gly Ala Glu Leu Thr Glu Gln Leu Gln Ala
   210 215 220
   Val Glu Arg Arg Ile Tyr Glu Leu Ala Gly Gln Glu Phe Asn Ile Asn
   225 230 235 240
   Ser Pro Lys Gln Leu Gly Thr Val Leu Phe Asp Lys Leu Gln Leu Pro
   245 250 255
   Val Leu Lys Lys Thr Lys Thr Gly Tyr Ser Thr Ser Ala Asp Val Leu
   260 265 270
   Glu Lys Leu Ala Pro His His Glu Ile Val Glu His Ile Leu His Tyr
   275 280 285
   Arg Gln Leu Gly Lys Leu Gln Ser Thr Tyr Ile Glu Gly Leu Leu Lys
   290 295 300
   Val Val His Pro Val Thr Gly Lys Val His Thr Met Phe Asn Gln Ala
   305 310 315 320
   Leu Thr Gln Thr Gly Arg Leu Ser Ser Val Glu Pro Asn Leu Gln Asn
   325 330 335
   Ile Pro Ile Arg Leu Glu Glu Gly Arg Lys Ile Arg Gln Ala Phe Val
   340 345 350
   Pro Ser Glu Pro Asp Trp Leu Ile Phe Ala Ala Asp Tyr Ser Gln Ile
   355 360 365
   Glu Leu Arg Val Leu Ala His Ile Ala Glu Asp Asp Asn Leu Ile Glu
   370 375 380
   Ala Phe Arg Arg Gly Leu Asp Ile His Thr Lys Thr Ala Met Asp Ile
   385 390 395 400
   Phe His Val Ser Glu Glu Asp Val Thr Ala Asn Met Arg Arg Gln Ala
   405 410 415
   Lys Ala Val Asn Phe Gly Ile Val Tyr Gly Ile Ser Asp Tyr Gly Leu
   420 425 430
   Ala Gln Asn Leu Asn Ile Thr Arg Lys Glu Ala Ala Glu Phe Ile Glu
   435 440 445
   Arg Tyr Phe Ala Ser Phe Pro Gly Val Lys Gln Tyr Met Asp Asn Ile
   450 455 460
   Val Gln Glu Ala Lys Gln Lys Gly Tyr Val Thr Thr Leu Leu His Arg
   465 470 475 480
   Arg Arg Tyr Leu Pro Asp Ile Thr Ser Arg Asn Phe Asn Val Arg Ser
   485 490 495
   Phe Ala Glu Arg Thr Ala Met Asn Thr Pro Ile Gln Gly Ser Ala Ala
   500 505 510
   Asp Ile Ile Lys Lys Ala Met Ile Asp Leu Ser Val Arg Leu Arg Glu
   515 520 525
   Glu Arg Leu Gln Ala Arg Leu Leu Leu Gln Val His Asp Glu Leu Ile
   530 535 540
   Leu Glu Ala Pro Lys Glu Glu Ile Glu Arg Leu Cys Arg Leu Val Pro
   545 550 555 560
   Glu Val Met Glu Gln Ala Val Thr Leu Arg Val Pro Leu Lys Val Asp
   565 570 575
   Tyr His Tyr Gly Pro Thr Trp Tyr Asp Ala Lys
   580 585
<210> 11
   <211> 587
   <212> PRT
   <213> Geobacillus stearothermophilus
<400> 11
   Asp Glu Gly Glu Lys Pro Leu Ala Gly Met Asp Phe Ala Ile Ala Asp
   1 5 10 15
   Ser Val Thr Asp Glu Met Leu Ala Asp Lys Ala Ala Leu Val Val Glu
               20                  25                  30
   Val Val Gly Asp Asn Tyr His His Ala Pro Ile Val Gly Ile Ala Leu
           35                  40                  45
   Ala Asn Glu Arg Gly Arg Phe Phe Leu Arg Pro Glu Thr Ala Leu Ala
       50                  55                  60
   Asp Pro Lys Phe Leu Ala Trp Leu Gly Asp Glu Thr Lys Lys Lys Thr
   65                  70                  75                  80
   Met Phe Asp Ser Lys Arg Ala Ala Val Ala Leu Lys Trp Lys Gly Ile
                   85                  90                  95
   Glu Leu Arg Gly Val Val Phe Asp Leu Leu Leu Ala Ala Tyr Leu Leu
               100                 105                 110
   Asp Pro Ala Gln Ala Ala Gly Asp Val Ala Ala Val Ala Lys Met His
           115                 120                 125
   Gln Tyr Glu Ala Val Arg Ser Asp Glu Ala Val Tyr Gly Lys Gly Ala
       130                 135                 140
   Lys Arg Thr Val Pro Asp Glu Pro Thr Leu Ala Glu His Leu Ala Arg
   145                 150                 155                 160
   Lys Ala Ala Ala Ile Trp Ala Leu Glu Glu Pro Leu Met Asp Glu Leu
                   165                 170                 175
   Arg Arg Asn Glu Gln Asp Arg Leu Leu Thr Glu Leu Glu Gln Pro Leu
               180                 185                 190
   Ala Gly Ile Leu Ala Asn Met Glu Phe Thr Gly Val Lys Val Asp Thr
           195                 200                 205
   Lys Arg Leu Glu Gln Met Gly Ala Glu Leu Thr Glu Gln Leu Gln Ala
       210                 215                 220
   Val Glu Arg Arg Ile Tyr Glu Leu Ala Gly Gln Glu Phe Asn Ile Asn
   225                 230                 235                 240
   Ser Pro Lys Gln Leu Gly Thr Val Leu Phe Asp Lys Leu Gln Leu Pro
                   245                 250                 255
   Val Leu Lys Lys Thr Lys Thr Gly Tyr Ser Thr Ser Ala Asp Val Leu
   260 265 270
   Glu Lys Leu Ala Pro His His Glu Ile Val Glu His Ile Leu His Tyr
   275 280 285
   Arg Gln Leu Gly Lys Leu Gln Ser Thr Tyr Ile Glu Gly Leu Leu Lys
   290 295 300
   Val Val His Pro Val Thr Gly Lys Val His Thr Met Phe Asn Gln Ala
   305 310 315 320
   Leu Thr Gln Thr Gly Arg Leu Ser Ser Val Glu Pro Asn Leu Gln Asn
   325 330 335
   Ile Pro Ile Arg Leu Glu Glu Gly Arg Lys Ile Arg Gln Ala Phe Val
   340 345 350
   Pro Ser Glu Pro Asp Trp Leu Ile Phe Ala Ala Asp Tyr Ser Gln Ile
   355 360 365
   Glu Leu Arg Val Leu Ala His Ile Ala Glu Asp Asp Asn Leu Ile Glu
   370 375 380
   Ala Phe Arg Arg Gly Leu Asp Ile His Thr Lys Thr Ala Met Asp Ile
   385 390 395 400
   Phe His Val Ser Glu Glu Asp Val Thr Ala Asn Met Arg Arg Gln Ala
   405 410 415
   Lys Ala Val Asn Phe Gly Ile Val Tyr Gly Ile Ser Asp Tyr Gly Leu
   420 425 430
   Ala Gln Asn Leu Asn Ile Thr Arg Lys Glu Ala Ala Glu Phe Ile Glu
   435 440 445
   Arg Tyr Phe Ala Ser Phe Pro Gly Val Lys Gln Tyr Met Asp Asn Ile
   450 455 460
   Val Gln Glu Ala Lys Gln Lys Gly Tyr Val Thr Thr Leu Leu His Arg
   465 470 475 480
   Arg Arg Tyr Leu Pro Asp Ile Thr Ser Arg Asn Phe Asn Val Arg Ser
   485 490 495
   Phe Ala Glu Arg Thr Ala Met Asn Thr Pro Ile Gln Gly Ser Ala Ala
   500 505 510
   Asp Ile Ile Lys Lys Ala Met Ile Asp Leu Ser Val Arg Leu Arg Glu
           515                 520                 525
   Glu Arg Leu Gln Ala Arg Leu Leu Leu Gln Val His Asp Glu Leu Ile
       530                 535                 540
   Leu Glu Ala Pro Lys Glu Glu Ile Glu Arg Leu Cys Arg Leu Val Pro
   545                 550                 555                 560
   Glu Val Met Glu Gln Ala Val Ala Leu Arg Val Pro Leu Lys Val Asp
                   565                 570                 575
   Tyr His Tyr Gly Pro Thr Trp Tyr Asp Ala Lys
               580                 585
<210> 12
   <211> 587
   <212> PRT
   <213> Bacillus sp. G(2006)
<400> 12
   Ser Glu Glu Glu Lys Pro Leu Ala Lys Met Ala Phe Thr Leu Ala Asp
   1               5                   10                  15
   Arg Val Thr Glu Glu Met Leu Ala Asp Lys Ala Ala Leu Val Val Glu
               20                  25                  30
   Val Val Glu Glu Asn Tyr His Asp Ala Pro Ile Val Gly Ile Ala Val
           35                  40                  45
   Val Asn Glu His Gly Arg Phe Phe Leu Arg Pro Glu Thr Ala Leu Ala
       50                  55                  60
   Asp Pro Gln Phe Val Ala Trp Leu Gly Asp Glu Thr Lys Lys Lys Ser
   65                  70                  75                  80
   Met Phe Asp Ser Lys Arg Ala Ala Val Ala Leu Lys Trp Lys Gly Ile
                   85                  90                  95
   Glu Leu Cys Gly Val Ser Phe Asp Leu Leu Leu Ala Ala Tyr Leu Leu
               100                 105                 110
   Asp Pro Ala Gln Gly Val Asp Asp Val Ala Ala Ala Ala Lys Met Lys
           115                 120                 125
   Gln Tyr Glu Ala Val Arg Ser Asp Glu Ala Val Tyr Gly Lys Gly Ala
       130                 135                 140
   Lys Arg Ala Val Pro Asp Glu Pro Val Leu Ala Glu His Leu Val Arg
   145                 150                 155                 160
   Lys Ala Ala Ala Ile Trp Ala Leu Glu Arg Pro Phe Leu Asp Glu Leu
                   165                 170                 175
   Arg Arg Asn Glu Gln Asp Arg Leu Leu Val Glu Leu Glu Gln Pro Leu
               180                 185                 190
   Ser Ser Ile Leu Ala Glu Met Glu Phe Ala Gly Val Lys Val Asp Thr
           195                 200                 205
   Lys Arg Leu Glu Gln Met Gly Glu Glu Leu Ala Glu Gln Leu Arg Thr
       210                 215                 220
   Val Glu Gln Arg Ile Tyr Glu Leu Ala Gly Gln Glu Phe Asn Ile Asn
   225                 230                 235                 240
   Ser Pro Lys Gln Leu Gly Val Ile Leu Phe Glu Lys Leu Gln Leu Pro
                   245                 250                 255
   Ile Leu Lys Lys Thr Lys Thr Gly Tyr Ser Thr Ser Ala Asp Val Leu
               260                 265                 270
   Glu Lys Leu Ala Pro Tyr His Glu Ile Val Glu Asn Ile Leu His Tyr
           275                 280                 285
   Arg Gln Leu Gly Lys Leu Gln Ser Thr Tyr Ile Glu Gly Leu Leu Lys
       290                 295                 300
   Val Val Arg Pro Asp Thr Lys Lys Val His Thr Ile Phe Asn Gln Ala
   305                 310                 315                 320
   Leu Thr Gln Thr Gly Arg Leu Ser Ser Thr Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Ser Asp Trp Leu Ile Phe Ala Ala Asp Tyr Ser Gln Ile
           355                 360                 365
   Glu Leu Arg Val Leu Ala His Ile Ala Glu Asp Asp Asn Leu Met Glu
       370                 375                 380
   Ala Phe Arg Arg Asp Leu Asp Ile His Thr Lys Thr Ala Met Asp Ile
   385                 390                 395                 400
   Phe Gln Val Ser Glu Asp Glu Val Thr Pro Asn Met Arg Arg Gln Ala
   405 410 415
   Lys Ala Val Asn Phe Gly Ile Val Tyr Gly Ile Ser Asp Tyr Gly Leu
   420 425 430
   Ala Gln Asn Leu Asn Ile Ser Arg Lys Glu Ala Ala Glu Phe Ile Glu
   435 440 445
   Arg Tyr Phe Glu Ser Phe Pro Gly Val Lys Arg Tyr Met Glu Asn Ile
   450 455 460
   Val Gln Glu Ala Lys Gln Lys Gly Tyr Val Thr Thr Leu Leu His Arg
   465 470 475 480
   Arg Arg Tyr Leu Pro Asp Ile Thr Ser Arg Asn Phe Asn Val Arg Ser
   485 490 495
   Phe Ala Glu Arg Met Ala Met Asn Thr Pro Ile Gln Gly Ser Ala Ala
   500 505 510
   Asp Ile Ile Lys Lys Ala Met Ile Asp Leu Asn Ala Arg Leu Lys Glu
   515 520 525
   Glu Arg Leu Gln Ala Arg Leu Leu Leu Gln Val His Asp Glu Leu Ile
   530 535 540
   Leu Glu Ala Pro Lys Glu Glu Met Glu Arg Leu Cys Arg Leu Val Pro
   545 550 555 560
   Glu Val Met Glu Gln Ala Val Thr Leu Arg Val Pro Leu Lys Val Asp
   565 570 575
   Tyr His Tyr Gly Pro Thr Trp Tyr Asp Ala Lys
   580 585
<210> 13
   <211> 587
   <212> PRT
   <213> Bacillus caldolyticus
<400> 13
   Ser Glu Glu Glu Lys Pro Leu Ala Lys Met Ala Phe Thr Leu Ala Asp
   1 5 10 15
   Arg Val Thr Glu Glu Met Leu Ala Asp Lys Ala Ala Leu Val Val Glu
   20 25 30
   Val Val Glu Glu Asn Tyr His Asp Ala Pro Ile Val Gly Ile Ala Val
   35 40 45
   Val Asn Glu His Gly Arg Phe Phe Leu Arg Pro Glu Thr Ala Leu Ala
   50 55 60
   Asp Pro Gln Phe Val Ala Trp Leu Gly Asp Glu Thr Lys Lys Lys Ser
   65 70 75 80
   Met Phe Asp Ser Lys Arg Ala Ala Val Ala Leu Lys Trp Lys Gly Ile
   85 90 95
   Glu Leu Cys Gly Val Ser Phe Asp Leu Leu Leu Ala Ala Tyr Leu Leu
   100 105 110
   Asp Pro Ala Gln Gly Val Asp Asp Val Ala Ala Ala Ala Lys Met Lys
   115 120 125
   Gln Tyr Glu Ala Val Arg Ser Asp Glu Ala Val Tyr Gly Lys Gly Ala
   130 135 140
   Lys Arg Ala Val Pro Asp Glu Pro Val Leu Ala Glu His Leu Val Arg
   145 150 155 160
   Lys Ala Ala Ala Ile Trp Ala Leu Glu Arg Pro Phe Leu Asp Glu Leu
   165 170 175
   Arg Arg Asn Glu Gln Asp Arg Leu Leu Val Glu Leu Glu Gln Pro Leu
   180 185 190
   Ser Ser Ile Leu Ala Glu Met Glu Phe Ala Gly Val Lys Val Asp Thr
   195 200 205
   Lys Arg Leu Glu Gln Met Gly Glu Glu Leu Ala Glu Gln Leu Arg Thr
   210 215 220
   Val Glu Gln Arg Ile Tyr Glu Leu Ala Gly Gln Glu Phe Asn Ile Asn
   225 230 235 240
   Ser Pro Lys Gln Leu Gly Val Ile Leu Phe Glu Lys Leu Gln Leu Pro
   245 250 255
   Val Leu Lys Lys Thr Lys Thr Gly Tyr Ser Thr Ser Ala Asp Val Leu
   260 265 270
   Glu Lys Leu Ala Pro Tyr His Glu Ile Val Glu Asn Ile Leu His Tyr
   275 280 285
   Arg Gln Leu Gly Lys Leu Gln Ser Thr Tyr Ile Glu Gly Leu Leu Lys
       290                 295                 300
   Val Val Arg Pro Asp Thr Lys Lys Val His Thr Ile Phe Asn Gln Ala
   305                 310                 315                 320
   Leu Thr Gln Thr Gly Arg Leu Ser Ser Thr Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Ser Asp Trp Leu Ile Phe Ala Ala Asp Tyr Ser Gln Ile
           355                 360                 365
   Glu Leu Arg Val Leu Ala His Ile Ala Glu Asp Asp Asn Leu Met Glu
       370                 375                 380
   Ala Phe Arg Arg Asp Leu Asp Ile His Thr Lys Thr Ala Met Asp Ile
   385                 390                 395                 400
   Phe Gln Val Ser Glu Asp Glu Val Thr Pro Asn Met Arg Arg Gln Ala
                   405                 410                 415
   Lys Ala Val Asn Phe Gly Ile Val Tyr Gly Ile Ser Asp Tyr Gly Leu
               420                 425                 430
   Ala Gln Asn Leu Asn Ile Ser Arg Lys Glu Ala Ala Glu Phe Ile Glu
           435                 440                 445
   Arg Tyr Phe Glu Ser Phe Pro Gly Val Lys Arg Tyr Met Glu Asn Ile
       450                 455                 460
   Val Gln Glu Ala Lys Gln Lys Gly Tyr Val Thr Thr Leu Leu His Arg
   465                 470                 475                 480
   Arg Arg Tyr Leu Pro Asp Ile Thr Ser Arg Asn Phe Asn Val Arg Ser
                   485                 490                 495
   Phe Ala Glu Arg Met Ala Met Asn Thr Pro Ile Gln Gly Ser Ala Ala
               500                 505                 510
   Asp Ile Ile Lys Lys Ala Met Ile Asp Leu Asn Ala Arg Leu Lys Glu
           515                 520                 525
   Glu Arg Leu Gln Ala Arg Leu Leu Leu Gln Val His Asp Glu Leu Ile
   530 535 540
   Leu Glu Ala Pro Lys Glu Glu Met Glu Arg Leu Cys Arg Leu Val Pro
   545 550 555 560
   Glu Val Met Glu Gln Ala Val Thr Leu Arg Val Pro Leu Lys Val Asp
   565 570 575
   Tyr His Tyr Gly Ser Thr Trp Tyr Asp Ala Lys
   580 585
<210> 14
   <211> 587
   <212> PRT
   <213> Geobacillus sp. C56-T3
<400> 14
   Ser Glu Glu Glu Lys Pro Leu Ala Lys Met Ala Phe Thr Leu Ala Asp
   1               5                   10                  15
   Arg Val Thr Glu Glu Met Leu Ala Asp Lys Ala Ala Leu Val Val Glu
               20                  25                  30
   Val Val Glu Glu Asn Tyr His Asp Ala Pro Ile Val Gly Ile Ala Val
           35                  40                  45
   Val Asn Glu His Gly Arg Phe Phe Leu Arg Pro Glu Thr Ala Leu Ala
       50                  55                  60
   Asp Pro Gln Phe Val Ala Trp Leu Gly Asp Glu Thr Lys Lys Lys Ser
   65                  70                  75                  80
   Met Phe Asp Ser Lys Arg Ala Ala Val Ala Leu Lys Trp Lys Gly Ile
                   85                  90                  95
   Glu Leu Cys Gly Val Ser Phe Asp Leu Leu Leu Ala Ala Tyr Leu Leu
               100                 105                 110
   Asp Pro Ala Gln Gly Val Asp Asp Val Ala Ala Ala Ala Lys Met Lys
           115                 120                 125
   Gln Tyr Glu Ala Val Arg Pro Asp Glu Ala Val Tyr Gly Lys Gly Ala
       130                 135                 140
   Lys Arg Ala Val Pro Asp Glu Pro Val Leu Ala Glu His Leu Val Arg
   145                 150                 155                 160
   Lys Ala Ala Ala Ile Trp Ala Leu Glu Arg Pro Phe Leu Asp Glu Leu
   165 170 175
   Arg Arg Asn Glu Gln Asp Arg Leu Leu Val Glu Leu Glu Gln Pro Leu
   180 185 190
   Ser Ser Ile Leu Ala Glu Met Glu Phe Ala Gly Val Lys Val Asp Thr
   195 200 205
   Lys Arg Leu Glu Gln Met Gly Glu Glu Leu Ala Glu Gln Leu Arg Thr
   210 215 220
   Val Glu Gln Arg Ile Tyr Glu Leu Ala Gly Gln Glu Phe Asn Ile Asn
   225 230 235 240
   Ser Pro Lys Gln Leu Gly Val Ile Leu Phe Glu Lys Leu Gln Leu Pro
   245 250 255
   Val Leu Lys Lys Thr Lys Thr Gly Tyr Ser Thr Ser Ala Asp Val Leu
   260 265 270
   Glu Lys Leu Ala Pro Tyr His Glu Ile Val Glu Asn Ile Leu His Tyr
   275 280 285
   Arg Gln Leu Gly Lys Leu Gln Ser Thr Tyr Ile Glu Gly Leu Leu Lys
   290 295 300
   Val Val Arg Pro Asp Thr Lys Lys Val His Thr Ile Phe Asn Gln Ala
   305 310 315 320
   Leu Thr Gln Thr Gly Arg Leu Ser Ser Thr Glu Pro Asn Leu Gln Asn
   325 330 335
   Ile Pro Ile Arg Leu Glu Glu Gly Arg Lys Ile Arg Gln Ala Phe Val
   340 345 350
   Pro Ser Glu Ser Asp Trp Leu Ile Phe Ala Ala Asp Tyr Ser Gln Ile
   355 360 365
   Glu Leu Arg Val Leu Ala His Ile Ala Glu Asp Asp Asn Leu Met Glu
   370 375 380
   Ala Phe Arg Arg Asp Leu Asp Ile His Thr Lys Thr Ala Met Asp Ile
   385 390 395 400
   Phe Gln Val Ser Glu Asp Glu Val Thr Pro Asn Met Arg Arg Gln Ala
   405 410 415
   Lys Ala Val Asn Phe Gly Ile Val Tyr Gly Ile Ser Asp Tyr Gly Leu
   420 425 430
   Ala Gln Asn Leu Asn Ile Ser Arg Lys Glu Ala Ala Glu Phe Ile Glu
   435 440 445
   Arg Tyr Phe Glu Ser Phe Pro Gly Val Lys Arg Tyr Met Glu Asn Ile
   450 455 460
   Val Gln Glu Ala Lys Gln Lys Gly Tyr Val Thr Thr Leu Leu His Arg
   465 470 475 480
   Arg Arg Tyr Leu Pro Asp Ile Thr Ser Arg Asn Phe Asn Val Arg Ser
   485 490 495
   Phe Ala Glu Arg Met Ala Met Asn Thr Pro Ile Gln Gly Ser Ala Ala
   500 505 510
   Asp Ile Ile Lys Lys Ala Met Ile Asp Leu Asn Ala Arg Leu Lys Glu
   515 520 525
   Glu Arg Leu Gln Ala Arg Leu Leu Leu Gln Val His Asp Glu Leu Ile
   530 535 540
   Leu Glu Ala Pro Lys Glu Glu Met Glu Arg Leu Cys Arg Leu Val Pro
   545 550 555 560
   Glu Val Met Glu Gln Ala Val Thr Leu Arg Val Pro Leu Lys Val Asp
   565 570 575
   Tyr His Tyr Gly Ser Thr Trp Tyr Asp Ala Lys
   580 585
<210> 15
   <211> 587
   <212> PRT
   <213> Geobacillus sp. Y412MC61
<400> 15
   Ser Glu Glu Glu Lys Pro Leu Ala Lys Met Ala Phe Thr Leu Ala Asp
   1 5 10 15
   Arg Val Thr Glu Glu Met Leu Ala Asp Lys Ala Ala Leu Val Val Glu
   20 25 30
   Val Val Glu Glu Asn Tyr His Asp Ala Pro Ile Val Gly Ile Ala Val
   35 40 45
   Val Asn Glu His Gly Arg Phe Phe Leu Arg Pro Glu Thr Ala Leu Ala
       50                  55                  60
   Asp Pro Gln Phe Val Ala Trp Leu Gly Asp Glu Thr Lys Lys Lys Ser
   65                  70                  75                  80
   Met Phe Asp Ser Lys Arg Ala Ala Val Ala Leu Lys Trp Lys Gly Ile
                   85                  90                  95
   Glu Leu Cys Gly Val Ser Phe Asp Leu Leu Leu Ala Ala Tyr Leu Leu
               100                 105                 110
   Asp Pro Ala Gln Gly Val Asp Asp Val Ala Ala Ala Ala Lys Met Lys
           115                 120                 125
   Gln Tyr Glu Ala Val Arg Pro Asp Glu Ala Val Tyr Gly Lys Gly Ala
       130                 135                 140
   Lys Arg Ala Val Pro Asp Glu Pro Val Leu Ala Glu His Leu Val Arg
   145                 150                 155                 160
   Lys Ala Ala Ala Ile Trp Ala Leu Glu Arg Pro Phe Leu Asp Glu Leu
                   165                 170                 175
   Arg Arg Asn Glu Gln Asp Arg Leu Leu Val Glu Leu Glu Gln Pro Leu
               180                 185                 190
   Ser Ser Ile Leu Ala Glu Met Glu Phe Ala Gly Val Lys Val Asp Thr
           195                 200                 205
   Lys Arg Leu Glu Gln Met Gly Glu Glu Leu Ala Glu Gln Leu Gly Thr
       210                 215                 220
   Val Glu Gln Arg Ile Tyr Glu Leu Ala Gly Gln Glu Phe Asn Ile Asn
   225                 230                 235                 240
   Ser Pro Lys Gln Leu Gly Val Ile Leu Phe Glu Lys Leu Gln Leu Pro
                   245                 250                 255
   Val Leu Lys Lys Thr Lys Thr Gly Tyr Ser Thr Ser Ala Asp Val Leu
               260                 265                 270
   Glu Lys Leu Ala Pro Tyr His Glu Ile Val Glu Asn Ile Leu His Tyr
           275                 280                 285
   Arg Gln Leu Gly Lys Leu Gln Ser Thr Tyr Ile Glu Gly Leu Leu Lys
       290                 295                 300
   Val Val Arg Pro Asp Thr Lys Lys Val His Thr Ile Phe Asn Gln Ala
   305                 310                 315                 320
   Leu Thr Gln Thr Gly Arg Leu Ser Ser Thr Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Ser Asp Trp Leu Ile Phe Ala Ala Asp Tyr Ser Gln Ile
           355                 360                 365
   Glu Leu Arg Val Leu Ala His Ile Ala Glu Asp Asp Asn Leu Met Glu
       370                 375                 380
   Ala Phe Arg Arg Asp Leu Asp Ile His Thr Lys Thr Ala Met Asp Ile
   385                 390                 395                 400
   Phe Gln Val Ser Glu Asp Glu Val Thr Pro Asn Met Arg Arg Gln Ala
                   405                 410                 415
   Lys Ala Val Asn Phe Gly Ile Val Tyr Gly Ile Ser Asp Tyr Gly Leu
               420                 425                 430
   Ala Gln Asn Leu Asn Ile Ser Arg Lys Glu Ala Ala Glu Phe Ile Glu
           435                 440                 445
   Arg Tyr Phe Glu Ser Phe Pro Gly Val Lys Arg Tyr Met Glu Asn Ile
       450                 455                 460
   Val Gln Glu Ala Lys Gln Lys Gly Tyr Val Thr Thr Leu Leu His Arg
   465                 470                 475                 480
   Arg Arg Tyr Leu Pro Asp Ile Thr Ser Arg Asn Phe Asn Val Arg Ser
                   485                 490                 495
   Phe Ala Glu Arg Met Ala Met Asn Thr Pro Ile Gln Gly Ser Ala Ala
               500                 505                 510
   Asp Ile Ile Lys Lys Ala Met Ile Asp Leu Asn Ala Arg Leu Lys Glu
           515                 520                 525
   Glu Arg Leu Gln Ala Arg Leu Leu Leu Gln Val His Asp Glu Leu Ile
       530                 535                 540
   Leu Glu Ala Pro Lys Glu Glu Met Glu Arg Leu Cys Arg Leu Val Pro
   545                 550                 555                 560
   Glu Val Met Glu Gln Ala Val Thr Leu Arg Val Pro Leu Lys Val Asp
   565 570 575
   Tyr His Tyr Gly Ser Thr Trp Tyr Asp Ala Lys
   580 585
<210> 16
   <211> 587
   <212> PRT
   <213> Geobacillus kaustophilus HTA426
<400> 16
   Ser Glu Glu Glu Lys Pro Leu Ala Lys Met Ala Phe Thr Leu Ala Asp
   1 5 10 15
   Arg Val Thr Glu Glu Met Leu Ala Asp Lys Ala Ala Leu Val Val Glu
   20 25 30
   Val Val Glu Glu Asn Tyr His Asp Ala Pro Ile Val Gly Ile Ala Val
   35 40 45
   Val Asn Glu His Gly Arg Phe Phe Leu Arg Pro Glu Thr Ala Leu Ala
   50 55 60
   Asp Pro Gln Phe Val Ala Trp Leu Gly Asp Glu Thr Lys Lys Lys Ser
   65 70 75 80
   Met Phe Asp Ser Lys Arg Ala Ala Val Ala Leu Lys Trp Lys Gly Ile
   85 90 95
   Glu Leu Cys Gly Val Ser Phe Asp Leu Leu Leu Ala Ala Tyr Leu Leu
   100 105 110
   Asp Pro Ala Gln Gly Val Asp Asp Val Ala Ala Ala Ala Lys Met Lys
   115 120 125
   Gln Tyr Glu Ala Val Arg Pro Asp Glu Ala Val Tyr Gly Lys Gly Ala
   130 135 140
   Lys Arg Ala Val Pro Asp Glu Pro Val Leu Ala Glu His Leu Val Arg
   145 150 155 160
   Lys Ala Ala Ala Ile Trp Glu Leu Glu Arg Pro Phe Leu Asp Glu Leu
   165 170 175
   Arg Arg Asn Glu Gln Asp Arg Leu Leu Val Glu Leu Glu Gln Pro Leu
   180 185 190
   Ser Ser Ile Leu Ala Glu Met Glu Phe Ala Gly Val Lys Val Asp Thr
           195                 200                 205
   Lys Arg Leu Glu Gln Met Gly Lys Glu Leu Ala Glu Gln Leu Gly Thr
       210                 215                 220
   Val Glu Gln Arg Ile Tyr Glu Leu Ala Gly Gln Glu Phe Asn Ile Asn
   225                 230                 235                 240
   Ser Pro Lys Gln Leu Gly Val Ile Leu Phe Glu Lys Leu Gln Leu Pro
                   245                 250                 255
   Val Leu Lys Lys Thr Lys Thr Gly Tyr Ser Thr Ser Ala Asp Val Leu
               260                 265                 270
   Glu Lys Leu Ala Pro Tyr His Glu Ile Val Glu Asn Ile Leu His Tyr
           275                 280                 285
   Arg Gln Leu Gly Lys Leu Gln Ser Thr Tyr Ile Glu Gly Leu Leu Lys
       290                 295                 300
   Val Val Arg Pro Asp Thr Lys Lys Val His Thr Ile Phe Asn Gln Ala
   305                 310                 315                 320
   Leu Thr Gln Thr Gly Arg Leu Ser Ser Thr Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Ser Asp Trp Leu Ile Phe Ala Ala Asp Tyr Ser Gln Ile
           355                 360                 365
   Glu Leu Arg Val Leu Ala His Ile Ala Glu Asp Asp Asn Leu Met Glu
       370                 375                 380
   Ala Phe Arg Arg Asp Leu Asp Ile His Thr Lys Thr Ala Met Asp Ile
   385                 390                 395                 400
   Phe Gln Val Ser Glu Asp Glu Val Thr Pro Asn Met Arg Arg Gln Ala
                   405                 410                 415
   Lys Ala Val Asn Phe Gly Ile Val Tyr Gly Ile Ser Asp Tyr Gly Leu
               420                 425                 430
   Ala Gln Asn Leu Asn Ile Ser Arg Lys Glu Ala Ala Glu Phe Ile Glu
                   435                 440                 445
   Arg Tyr Phe Glu Ser Phe Pro Gly Val Lys Arg Tyr Met Glu Asn Ile
       450                 455                 460
   Val Gln Glu Ala Lys Gln Lys Gly Tyr Val Thr Thr Leu Leu His Arg
   465                 470                 475                 480
   Arg Arg Tyr Leu Pro Asp Ile Thr Ser Arg Asn Phe Asn Val Arg Ser
                   485                 490                 495
   Phe Ala Glu Arg Met Ala Met Asn Thr Pro Ile Gln Gly Ser Ala Ala
               500                 505                 510
   Asp Ile Ile Lys Lys Ala Met Ile Asp Leu Asn Ala Arg Leu Lys Glu
           515                 520                 525
   Glu Arg Leu Gln Ala Arg Leu Leu Leu Gln Val His Asp Glu Leu Ile
       530                 535                 540
   Leu Glu Ala Pro Lys Glu Glu Met Glu Arg Leu Cys Arg Leu Val Pro
   545                 550                 555                 560
   Glu Val Met Glu Gln Ala Val Thr Leu Arg Val Pro Leu Lys Val Asp
                   565                 570                 575
   Tyr His Tyr Gly Ser Thr Trp Tyr Asp Ala Lys
               580                 585
<210> 17
   <211> 588
   <212> PRT
   <213> Bacillus caldotenax
<400> 17
   Ser Glu Glu Glu Lys Pro Leu Ala Lys Met Ala Phe Thr Leu Ala Asp
   1               5                   10                  15
   Arg Val Thr Glu Glu Met Leu Ala Asp Lys Ala Ala Leu Val Val Glu
               20                  25                  30
   Val Val Glu Glu Asn Tyr His Asp Ala Pro Ile Val Gly Ile Ala Val
           35                  40                  45
   Val Asn Glu His Gly Arg Phe Phe Leu Arg Pro Glu Thr Ala Leu Ala
       50                  55                  60
   Asp Pro Gln Phe Val Ala Trp Leu Gly Asp Glu Thr Lys Lys Lys Ser
   65 70 75 80
   Met Phe Asp Ser Lys Arg Ala Ala Val Ala Leu Lys Trp Lys Gly Ile
   85 90 95
   Glu Leu Cys Gly Val Ser Phe Asp Leu Leu Leu Ala Ala Tyr Leu Leu
   100 105 110
   Asp Pro Ala Gln Gly Val Asp Asp Val Ala Ala Ala Ala Lys Met Lys
   115 120 125
   Gln Tyr Glu Ala Val Arg Pro Asp Glu Ala Val Tyr Gly Lys Gly Ala
   130 135 140
   Lys Arg Ala Val Pro Asp Glu Pro Val Leu Ala Glu His Leu Val Arg
   145 150 155 160
   Lys Ala Ala Ala Ile Trp Ala Leu Glu Arg Pro Phe Leu Asp Glu Leu
   165 170 175
   Arg Arg Asn Glu Gln Asp Arg Leu Leu Val Glu Leu Glu Gln Pro Leu
   180 185 190
   Ser Ser Ile Leu Ala Glu Met Glu Phe Ala Gly Val Lys Val Asp Thr
   195 200 205
   Lys Arg Leu Glu Gln Met Gly Glu Glu Leu Ala Glu Gln Leu Arg Thr
   210 215 220
   Val Glu Gln Arg Ile Tyr Glu Leu Ala Gly Gln Glu Phe Asn Ile Asn
   225 230 235 240
   Ser Pro Lys Gln Leu Gly Val Ile Leu Phe Glu Lys Leu Gln Leu Pro
   245 250 255
   Val Leu Lys Lys Ser Lys Thr Gly Tyr Ser Thr Ser Ala Asp Val Leu
   260 265 270
   Glu Lys Leu Ala Pro Tyr His Glu Ile Val Glu Asn Ile Leu Gln His
   275 280 285
   Tyr Arg Gln Leu Gly Lys Leu Gln Ser Thr Tyr Ile Glu Gly Leu Leu
   290 295 300
   Lys Val Val Arg Pro Asp Thr Lys Lys Val His Thr Ile Phe Asn Gln
   305 310 315 320
   Ala Leu Thr Gln Thr Gly Arg Leu Ser Ser Thr Glu Pro Asn Leu Gln
   325 330 335
   Asn Ile Pro Ile Arg Leu Glu Glu Gly Arg Lys Ile Arg Gln Ala Phe
   340 345 350
   Val Pro Ser Glu Ser Asp Trp Leu Ile Phe Ala Ala Asp Tyr Ser Gln
   355 360 365
   Ile Glu Leu Arg Val Leu Ala His Ile Ala Glu Asp Asp Asn Leu Met
   370 375 380
   Glu Ala Phe Arg Arg Asp Leu Asp Ile His Thr Lys Thr Ala Met Asp
   385 390 395 400
   Ile Phe Gln Val Ser Glu Asp Glu Val Thr Pro Asn Met Arg Arg Gln
   405 410 415
   Ala Lys Ala Val Asn Phe Gly Ile Val Tyr Gly Ile Ser Asp Tyr Gly
   420 425 430
   Leu Ala Gln Asn Leu Asn Ile Ser Arg Lys Glu Ala Ala Glu Phe Ile
   435 440 445
   Glu Arg Tyr Phe Glu Ser Phe Pro Gly Val Lys Arg Tyr Met Glu Asn
   450 455 460
   Ile Val Gln Glu Ala Lys Gln Lys Gly Tyr Val Thr Thr Leu Leu His
   465 470 475 480
   Arg Arg Arg Tyr Leu Pro Asp Ile Thr Ser Arg Asn Phe Asn Val Arg
   485 490 495
   Ser Phe Ala Glu Arg Met Ala Met Asn Thr Pro Ile Gln Gly Ser Ala
   500 505 510
   Ala Asp Ile Ile Lys Lys Ala Met Ile Asp Leu Asn Ala Arg Leu Lys
   515 520 525
   Glu Glu Arg Leu Gln Ala Arg Leu Leu Leu Gln Val His Asp Glu Leu
   530 535 540
   Ile Leu Glu Ala Pro Lys Glu Glu Met Glu Arg Leu Cys Arg Leu Val
   545 550 555 560
   Pro Glu Val Met Glu Gln Ala Val Thr Leu Arg Val Pro Leu Lys Val
   565 570 575
   Asp Tyr His Tyr Gly Ser Thr Trp Tyr Asp Ala Lys
   580 585
<210> 18
   <211> 589
   <212> PRT
   <213> Geobacillus stearothermophilus
<400> 18
   Asp Glu Gly Glu Lys Pro Leu Ala Gly Met Asp Phe Ala Ile Ala Asp
   1 5 10 15
   Ser Val Thr Asp Glu Met Leu Ala Asp Lys Ala Ala Leu Val Val Glu
   20 25 30
   Val Val Gly Asp Asn Tyr His His Ala Pro Ile Val Gly Ile Ala Leu
   35 40 45
   Ala Asn Glu Arg Gly Arg Phe Phe Leu Arg Pro Glu Thr Ala Val Ala
   50 55 60
   Asp Pro Lys Phe Leu Ala Trp Leu Gly Asp Glu Thr Lys Lys Lys Thr
   65 70 75 80
   Met Phe Asp Ser Lys Arg Ala Ala Val Ala Leu Asn Gly Lys Gly Ile
   85 90 95
   Glu Leu Ala Gly Val Gly Val Val Phe Asp Leu Leu Leu Ala Ala Tyr
   100 105 110
   Leu Leu Asp Pro Ala Gln Ala Ala Gly Asp Val Ala Ala Val Ala Lys
   115 120 125
   Met His Gln Tyr Glu Ala Val Arg Ser Asp Glu Ala Val Tyr Gly Lys
   130 135 140
   Gly Ala Lys Arg Thr Val Pro Asp Glu Pro Thr Leu Ala Glu Gln Leu
   145 150 155 160
   Val Arg Lys Ala Ala Ala Ile Trp Ala Leu Glu Glu Pro Leu Met Asp
   165 170 175
   Glu Leu Arg Arg Asn Glu Gln Asp Arg Leu Leu Thr Glu Leu Glu His
   180 185 190
   Ala Leu Ala Gly Ile Leu Ala Asn Met Glu Phe Thr Gly Val Lys Val
   195 200 205
   Asp Thr Lys Arg Leu Glu Gln Met Gly Ala Glu Leu Thr Glu Gln Leu
       210                 215                 220
   Gln Ala Val Glu Arg Arg Ile Tyr Glu Leu Ala Gly Gln Glu Phe Asn
   225                 230                 235                 240
   Ile Asn Ser Pro Lys Gln Leu Gly Thr Val Leu Phe Asp Lys Leu Gln
                   245                 250                 255
   Leu Pro Val Leu Lys Lys Thr Lys Thr Gly Tyr Ser Thr Ser Ala Asp
               260                 265                 270
   Val Leu Glu Lys Leu Ala Pro His His Glu Ile Val Glu His Ile Leu
           275                 280                 285
   His Tyr Arg Gln Leu Gly Lys Leu Gln Ser Thr Tyr Ile Glu Gly Leu
       290                 295                 300
   Leu Lys Val Val His Pro Val Thr Gly Lys Val His Thr Met Phe Asn
   305                 310                 315                 320
   Gln Ala Leu Thr Gln Thr Gly Arg Leu Ser Ser Val Glu Pro Asn Leu
                   325                 330                 335
   Gln Asn Ile Pro Ile Arg Leu Glu Glu Gly Arg Lys Ile Arg Gln Ala
               340                 345                 350
   Phe Val Pro Ser Glu Pro Asp Trp Leu Ile Phe Ala Ala Asp Tyr Ser
           355                 360                 365
   Gln Ile Glu Leu Arg Val Leu Ala His Ile Ala Glu Asp Asp Asn Leu
       370                 375                 380
   Ile Glu Ala Phe Arg Arg Trp Leu Asp Ile His Thr Lys Thr Ala Met
   385                 390                 395                 400
   Asp Ile Phe His Val Ser Glu Glu Asp Val Thr Ala Asn Met Arg Arg
                   405                 410                 415
   Gln Ala Lys Ala Val Asn Phe Gly Ile Val Tyr Gly Ile Ser Asp Tyr
               420                 425                 430
   Gly Leu Ala Gln Asn Leu Asn Ile Thr Arg Lys Glu Ala Ala Glu Phe
           435                 440                 445
   Ile Glu Arg Tyr Phe Ala Ser Phe Pro Gly Val Lys Gln Tyr Met Asp
       450                 455                 460
   Asn Ile Val Gln Glu Ala Lys Gln Lys Gly Tyr Val Thr Thr Leu Leu
   465 470 475 480
   His Arg Arg Arg Tyr Leu Pro Asp Ile Thr Ser Arg Asn Phe Asn Val
   485 490 495
   Arg Thr Phe Ala Glu Arg Thr Ala Met Asn Thr Pro Ile Gln Gly Ser
   500 505 510
   Ala Ala Asp Ile Ile Lys Lys Ala Met Ile Asp Leu Ser Val Ser Val
   515 520 525
   Arg Glu Glu Arg Leu Gln Ala Arg Leu Leu Leu Gln Gly His Asp Glu
   530 535 540
   Leu Ile Leu Glu Ala Pro Lys Glu Glu Ile Gly Arg Leu Cys Arg Leu
   545 550 555 560
   Val Pro Glu Val Met Glu Gln Ala Val Thr Leu Arg Val Pro Leu Lys
   565 570 575
   Val Asp Tyr His Tyr Gly Pro Thr Trp Tyr Asp Ala Lys
   580 585
<210> 19
   <211> 587
   <212> PRT
   <213> Geobacillus sp. WCH70
<400> 19
   Glu Glu Thr Glu Val Ala Leu Ser Asp Ile Asp Tyr Thr Ile Val Asp
   1 5 10 15
   Glu Val Thr Glu Ser Ile Leu Ser Asp Glu Ala Ala Leu Val Val Glu
   20 25 30
   Val Leu Glu Ser Asn Tyr His Lys Ala Pro Ile Leu Gly Phe Ala Ile
   35 40 45
   Ala Asn Glu His Gly Asn Phe Phe Ile Arg Thr Asp Thr Ala Leu Ser
   50 55 60
   Ser Ser Leu Phe Thr Gln Trp Leu Glu Asp Glu Ser Lys Lys Lys Ser
   65 70 75 80
   Val Phe Asp Gly Lys Arg Ala Ile Val Ser Leu Lys Trp Gln Gly Ile
   85 90 95
   Gln Leu Arg Gly Val Gln Phe Asp Leu Leu Ile Ala Ser Tyr Leu Leu
               100                 105                 110
   Asn Pro Ser Gln Ser Thr Glu Asp Val Ala Ser Ile Ala Lys Thr Lys
           115                 120                 125
   Gln Tyr Thr Asp Val Gln Ser Asp Glu Ala Ile Tyr Gly Lys Gly Ala
       130                 135                 140
   Lys Gln Lys Ile Pro Asp Glu Pro Val Leu Ala Glu His Leu Val Arg
   145                 150                 155                 160
   Lys Ala Ala Ala Ile Arg Ala Leu Glu Gln Asp Phe Ile Cys Asp Leu
                   165                 170                 175
   Gln Glu Asn Glu Gln Tyr Ser Leu Phe Thr Asp Leu Glu Leu Pro Leu
               180                 185                 190
   Ser Ser Ile Leu Ala Glu Met Glu Phe Thr Gly Val Lys Ile Asp Val
           195                 200                 205
   Lys Arg Leu Lys Glu Met Gly Glu Glu Leu Thr Glu Gln Leu Lys Glu
       210                 215                 220
   Ile Glu Gln Glu Ile Tyr Glu Leu Ala Gly Gln Glu Phe Asn Ile Asn
   225                 230                 235                 240
   Ser Pro Lys Gln Leu Gly Val Ile Leu Phe Glu Lys Leu Gln Leu Pro
                   245                 250                 255
   Val Leu Lys Lys Thr Lys Thr Gly Tyr Ser Thr Ser Ala Glu Val Leu
               260                 265                 270
   Glu Lys Leu Ala Pro Gln His Glu Ile Val Glu Lys Ile Leu His Tyr
           275                 280                 285
   Arg Gln Leu Gly Lys Leu Gln Ser Thr Tyr Ile Glu Gly Leu Leu Lys
       290                 295                 300
   Val Val His His Asp Thr Asn Lys Val His Thr Ile Phe Asn Gln Ala
   305                 310                 315                 320
   Leu Thr Gln Thr Gly Arg Leu Ser Ser Thr Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Glu Pro Asp Trp Val Ile Phe Ser Ala Asp Tyr Ser Gln Ile
           355                 360                 365
   Glu Leu Arg Val Leu Ala His Ile Ala Asn Asp Glu Asn Leu Ile Asp
       370                 375                 380
   Ala Phe Arg His Asp Leu Asp Ile His Thr Lys Thr Ala Met Asp Ile
   385                 390                 395                 400
   Phe His Val Asn Glu Asp Glu Val Thr Pro Asn Met Arg Arg Gln Ala
                   405                 410                 415
   Lys Ala Val Asn Phe Gly Ile Val Tyr Gly Ile Ser Asp Tyr Gly Leu
               420                 425                 430
   Ser Gln Asn Leu Asn Ile Thr Arg Lys Glu Ala Ser Glu Phe Ile Lys
           435                 440                 445
   Arg Tyr Phe Glu Ile Phe Pro Gly Val Lys Gln Tyr Met Lys Asp Ile
       450                 455                 460
   Val Gln Glu Ala Lys Gln Lys Gly Tyr Val Thr Thr Leu Leu His Arg
   465                 470                 475                 480
   Arg Arg Tyr Leu Pro Asp Ile Thr Ser Arg Asn Phe Asn Leu Arg Ser
                   485                 490                 495
   Phe Ala Glu Arg Thr Ala Met Asn Thr Pro Ile Gln Gly Ser Ala Ala
               500                 505                 510
   Asp Ile Ile Lys Lys Ala Met Ile Asp Leu Ser Asn Arg Leu Lys Lys
           515                 520                 525
   Glu Asn Met Lys Ala Arg Met Leu Leu Gln Val His Asp Glu Leu Ile
       530                 535                 540
   Leu Glu Ala Pro Lys Glu Glu Val Glu Arg Leu Gln Gln Ile Val Pro
   545                 550                 555                 560
   Glu Val Met Glu Asn Ala Val Gln Leu Arg Val Pro Leu Lys Val Asp
                   565                 570                 575
   Tyr His Phe Gly Pro Thr Trp Tyr Asp Ala Lys
               580                 585
<210> 20
   <211> 587
   <212> PRT
   <213> Geobacillus caldoxylosilyticus
<400> 20
   Lys Lys Glu Glu Ala Val Leu Pro Asn Ile Asp Tyr Thr Ile Val Glu
   1               5                   10                  15
   Glu Val Ser Glu Ser Val Leu Ala Asp Arg Ser Ala Leu Val Val Glu
               20                  25                  30
   Val Leu Glu Ser Asn Tyr His Lys Ala Pro Ile Leu Gly Phe Ala Leu
           35                  40                  45
   Ala Asn Glu His Gly His Phe Phe Ile Arg Thr Asp Thr Ala Leu Ser
       50                  55                  60
   Ser Pro Leu Phe Thr Ala Trp Leu Glu Asp Glu Thr Lys Lys Lys Ser
   65                  70                  75                  80
   Val Phe Asp Gly Lys Arg Ala Ile Val Ala Leu Lys Trp Lys Gly Ile
                   85                  90                  95
   Glu Leu Arg Gly Ile Glu Phe Asp Leu Leu Ile Ala Ser Tyr Leu Leu
               100                 105                 110
   Asn Pro Ser Gln Ser Thr Glu Asp Val Ala Ser Val Ala Lys Thr Lys
           115                 120                 125
   Gln Tyr Met Ala Val Gln Ser Asp Glu Ala Val Tyr Gly Lys Gly Ala
       130                 135                 140
   Lys Gln Thr Val Pro Glu Glu Lys Ala Leu Ala Glu His Leu Val Arg
   145                 150                 155                 160
   Lys Ala Ala Ala Ile Arg Ala Leu Glu Lys Glu Phe Ile His Asp Leu
                   165                 170                 175
   Gln Glu Asn Glu Gln Tyr Ala Leu Phe Thr Asp Leu Glu Leu Pro Leu
               180                 185                 190
   Ser Ser Ile Leu Ala Glu Met Glu Phe Thr Gly Val Lys Val Asp Val
           195                 200                 205
   Glu Arg Leu Lys Glu Met Gly Glu Glu Leu Thr Glu Gln Leu Arg Ala
       210                 215                 220
   Val Glu Gln Glu Ile Tyr Glu Leu Ala Gly Gln Glu Phe Asn Ile Asn
   225                 230                 235                 240
   Ser Pro Lys Gln Leu Gly Val Ile Leu Phe Glu Lys Leu Gln Leu Pro
                   245                 250                 255
   Val Leu Lys Lys Thr Lys Thr Gly Tyr Ser Thr Ser Ala Asp Val Leu
               260                 265                 270
   Glu Lys Leu Ala Pro Gln His Glu Ile Val Glu Lys Ile Leu His Tyr
           275                 280                 285
   Arg Gln Leu Gly Lys Leu Gln Ser Thr Tyr Ile Glu Gly Leu Leu Lys
       290                 295                 300
   Val Val His Cys Asp Thr His Lys Val His Thr Ile Phe Asn Gln Ala
   305                 310                 315                 320
   Leu Thr Gln Thr Gly Arg Leu Ser Ser Thr Glu Pro Asn Leu Gln Asn
                   325                 330                 335
   Ile Pro Ile Arg Leu Glu Glu Gly Arg Lys Ile Arg Gln Ala Phe Val
               340                 345                 350
   Pro Ser Lys Pro Asp Trp Val Ile Phe Ser Ala Asp Tyr Ser Gln Ile
           355                 360                 365
   Glu Leu Arg Val Leu Ala His Ile Ala Asn Asp Glu Asn Leu Ile Asp
       370                 375                 380
   Ala Phe Arg His Asp Leu Asp Ile His Thr Lys Thr Ala Met Asp Ile
   385                 390                 395                 400
   Phe His Val Thr Ala Asp Glu Val Thr Pro Asn Met Arg Arg Gln Ala
                   405                 410                 415
   Lys Ala Val Asn Phe Gly Ile Val Tyr Gly Ile Ser Asp Tyr Gly Leu
               420                 425                 430
   Ser Gln Asn Leu Asn Ile Pro Arg Lys Glu Ala Ala Glu Phe Ile Arg
           435                 440                 445
   Arg Tyr Phe Glu Ile Phe Pro Gly Val Lys Gln Tyr Met Glu Asn Ile
       450                 455                 460
   Val Gln Glu Ala Lys Gln Lys Gly Tyr Val Thr Thr Leu Leu His Arg
   465                 470                 475                 480
   Arg Arg Tyr Leu Pro Asp Ile Thr Ser Arg Asn Phe Asn Leu Arg Ser
   485 490 495
   Phe Ala Glu Arg Thr Ala Met Asn Thr Pro Ile Gln Gly Ser Ala Ala
   500 505 510
   Asp Ile Ile Lys Lys Ala Met Ile Asp Leu Ala Asp Arg Leu Lys Gln
   515 520 525
   Glu Lys Leu Gln Ala Arg Met Leu Leu Gln Val His Asp Glu Leu Ile
   530 535 540
   Leu Glu Ala Pro Lys Glu Glu Val Lys Arg Leu Gln Gln Ile Val Pro
   545 550 555 560
   Glu Val Met Glu Asn Ala Ile Gln Leu Lys Val Pro Leu Leu Ile Asp
   565 570 575
   Phe Arg Phe Gly Pro Thr Trp Asp Asp Ala Lys
   580 585
<210> 21
   <211> 587
   <212> PRT
   <213> Geobacillus sp. Y4.1MC1
<400> 21
   Glu Lys Thr Ala Val Ala Leu Pro Lys Ile Arg Tyr Glu Ile Val Asp
   1 5 10 15
   Glu Val Thr Glu Ala Ile Leu Ser Asp Glu Ala Ala Leu Val Val Glu
   20 25 30
   Val Leu Glu Ser Asn Tyr His Lys Ala Pro Ile Leu Gly Phe Ala Ile
   35 40 45
   Ala Asn Glu His Gly Asn Phe Phe Ile Arg Thr Asp Thr Ala Leu Ser
   50 55 60
   Ser Ser Leu Phe Thr Thr Trp Leu Glu Asp Glu Ser Lys Lys Lys Ser
   65 70 75 80
   Val Phe Asp Gly Lys Arg Ala Ile Val Ser Leu Lys Trp Gln Gly Val
   85 90 95
   His Leu Arg Gly Ile Gln Phe Asp Leu Leu Ile Ala Ser Tyr Leu Leu
   100 105 110
   Asn Pro Ser Gln Ser Thr Glu Asp Val Ala Ser Ile Ala Lys Thr Lys
           115                 120                 125
   Gln Tyr Ala Gly Val Gln Ser Asp Glu Ala Val Tyr Gly Lys Gly Ala
       130                 135                 140
   Lys Gln Lys Val Pro Asp Glu Gln Val Leu Ala Glu His Leu Val Arg
   145                 150                 155                 160
   Lys Ala Ala Ala Ile Arg Ala Leu Glu Gln Gly Phe Ile His Asp Leu
                   165                 170                 175
   Gln Glu Asn Glu Gln Tyr Ser Leu Phe Thr Asp Leu Glu Leu Pro Leu
               180                 185                 190
   Ser Thr Ile Leu Ala Glu Met Glu Phe Ala Gly Val Lys Val Asp Val
           195                 200                 205
   Glu Arg Leu Lys Glu Met Gly Glu Glu Leu Ala Glu Gln Leu Lys Glu
       210                 215                 220
   Val Glu Gln Glu Ile Tyr Arg Leu Ala Gly Gln Glu Phe Asn Ile Asn
   225                 230                 235                 240
   Ser Pro Lys Gln Leu Gly Val Ile Leu Phe Glu Lys Leu Gln Leu Pro
                   245                 250                 255
   Val Leu Lys Lys Thr Lys Thr Gly Tyr Ser Thr Ser Ala Glu Val Leu
               260                 265                 270
   Glu Lys Leu Ala Pro Gln His Glu Ile Val Glu Lys Ile Leu His Tyr
           275                 280                 285
   Arg Gln Leu Gly Lys Leu Gln Ser Thr Tyr Ile Glu Gly Leu Leu Lys
       290                 295                 300
   Val Val His Arg Asp Thr Asn Lys Val His Thr Ile Phe Asn Gln Ala
   305                 310                 315                 320
   Leu Thr Gln Thr Gly Arg Leu Ser Ser Thr Glu Pro Asn Leu Gln Asn
                   325                 330                 335
   Ile Pro Ile Arg Leu Glu Glu Gly Arg Lys Ile Arg Gln Ala Phe Ile
               340                 345                 350
   Pro Ser Glu Pro Asp Trp Val Ile Phe Ser Ala Asp Tyr Ser Gln Ile
           355                 360                 365
   Glu Leu Arg Val Leu Ala His Ile Ala Asn Asp Glu Asn Leu Ile Asp
       370                 375                 380
   Ala Phe Arg His Gly Leu Asp Ile His Thr Lys Thr Ala Met Asp Ile
   385                 390                 395                 400
   Phe His Val Ser Lys Glu Glu Val Thr Pro Asn Met Arg Arg Gln Ala
                   405                 410                 415
   Lys Ala Val Asn Phe Gly Ile Val Tyr Gly Ile Ser Asp Tyr Gly Leu
               420                 425                 430
   Ser Gln Asn Leu Asn Ile Thr Arg Lys Glu Ala Ala Glu Phe Ile Lys
           435                 440                 445
   Arg Tyr Phe Glu Ile Phe Pro Gly Val Lys Gln Tyr Met Lys Asp Ile
       450                 455                 460
   Val Gln Glu Ala Lys Gln Lys Gly Tyr Val Thr Thr Leu Leu His Arg
   465                 470                 475                 480
   Arg Arg Tyr Leu Pro Asp Ile Thr Ser Arg Asn Phe Asn Leu Arg Ser
                   485                 490                 495
   Phe Ala Glu Arg Thr Ala Met Asn Thr Pro Ile Gln Gly Ser Ala Ala
               500                 505                 510
   Asp Ile Ile Lys Lys Ala Met Ile Asp Leu Ser Lys Arg Leu Gln Lys
           515                 520                 525
   Glu Asn Met Lys Ala Arg Met Leu Leu Gln Val His Asp Glu Leu Ile
       530                 535                 540
   Leu Glu Ala Pro Lys Asp Glu Ile Glu Arg Leu Gln Gln Ile Val Pro
   545                 550                 555                 560
   Glu Val Met Glu Asn Ala Val Gln Leu Arg Val Pro Leu Lys Val Asp
                   565                 570                 575
   Tyr His Phe Gly Pro Thr Trp Tyr Asp Ala Lys
               580                 585
<210> 22
   <211> 587
   <212> PRT
   <213> Geobacillus thermoglucosidasius C56-YS93
<400> 22
   Glu Lys Thr Ala Val Ala Leu Pro Glu Ile Gly Tyr Lys Ile Val Asp
   1               5                   10                  15
   Glu Val Thr Glu Ala Ile Leu Ser Asp Glu Ala Ala Leu Val Val Glu
               20                  25                  30
   Val Leu Glu Ser Asn Tyr His Lys Ala Pro Ile Leu Gly Phe Ala Ile
           35                  40                  45
   Ala Asn Glu His Gly Asn Phe Phe Ile Arg Thr Asp Thr Ala Leu Ser
       50                  55                  60
   Ser Ser Leu Phe Thr Thr Trp Leu Glu Asp Glu Ser Lys Lys Lys Ser
   65                  70                  75                  80
   Val Phe Asp Gly Lys Arg Ala Ile Val Ser Leu Lys Trp Gln Gly Val
                   85                  90                  95
   His Leu Arg Gly Ile Gln Phe Asp Leu Leu Ile Ala Ser Tyr Leu Leu
               100                 105                 110
   Asn Pro Ser Gln Ser Thr Glu Asp Val Ala Ser Ile Ala Lys Thr Lys
           115                 120                 125
   Gln Tyr Val Gly Val Gln Ser Asp Glu Ala Val Tyr Gly Lys Gly Ala
       130                 135                 140
   Lys Gln Lys Val Pro Asp Glu Gln Val Leu Ala Glu His Leu Val Arg
   145                 150                 155                 160
   Lys Ala Ala Ala Ile Arg Ala Leu Glu Gln Gly Phe Ile His Asp Leu
                   165                 170                 175
   Gln Glu Asn Glu Gln Tyr Ser Leu Phe Thr Asp Leu Glu Leu Pro Leu
               180                 185                 190
   Ser Thr Ile Leu Ala Glu Met Glu Phe Ala Gly Val Lys Val Asp Val
           195                 200                 205
   Glu Arg Leu Lys Glu Met Gly Glu Glu Leu Ala Glu Gln Leu Lys Glu
       210                 215                 220
   Val Glu Gln Glu Ile Tyr Arg Leu Ala Gly Gln Glu Phe Asn Ile Asn
   225                 230                 235                 240
   Ser Pro Lys Gln Leu Gly Val Ile Leu Phe Glu Lys Leu Gln Leu Pro
                   245                 250                 255
   Val Leu Lys Lys Thr Lys Thr Gly Tyr Ser Thr Ser Ala Glu Val Leu
               260                 265                 270
   Glu Lys Leu Ala Pro Gln His Glu Ile Val Glu Lys Ile Leu His Tyr
           275                 280                 285
   Arg Gln Leu Gly Lys Leu Gln Ser Thr Tyr Ile Glu Gly Leu Leu Lys
       290                 295                 300
   Val Val His Arg Asp Thr Asn Lys Val His Thr Ile Phe Asn Gln Ala
   305                 310                 315                 320
   Leu Thr Gln Thr Gly Arg Leu Ser Ser Thr Glu Pro Asn Leu Gln Asn
                   325                 330                 335
   Ile Pro Ile Arg Leu Glu Glu Gly Arg Lys Ile Arg Gln Ala Phe Ile
               340                 345                 350
   Pro Ser Glu Pro Asp Trp Val Ile Phe Ser Ala Asp Tyr Ser Gln Ile
           355                 360                 365
   Glu Leu Arg Val Leu Ala His Ile Ala Asn Asp Glu Asn Leu Ile Asp
       370                 375                 380
   Ala Phe Arg His Gly Leu Asp Ile His Thr Lys Thr Ala Met Asp Ile
   385                 390                 395                 400
   Phe His Val Ser Lys Glu Glu Val Thr Pro Asn Met Arg Arg Gln Ala
                   405                 410                 415
   Lys Ala Val Asn Phe Gly Ile Val Tyr Gly Ile Ser Asp Tyr Gly Leu
               420                 425                 430
   Ser Gln Asn Leu Asn Ile Thr Arg Lys Glu Ala Ala Glu Phe Ile Lys
           435                 440                 445
   Arg Tyr Phe Glu Ile Phe Pro Gly Val Lys Gln Tyr Met Lys Asp Ile
       450                 455                 460
   Val Gln Glu Ala Lys Gln Lys Gly Tyr Val Thr Thr Leu Leu His Arg
   465                 470                 475                 480
   Arg Arg Tyr Leu Pro Asp Ile Thr Ser Arg Asn Phe Asn Leu Arg Ser
                   485                 490                 495
   Phe Ala Glu Arg Thr Ala Met Asn Thr Pro Ile Gln Gly Ser Ala Ala
   500 505 510
   Asp Ile Ile Lys Lys Ala Met Ile Asp Leu Ser Lys Arg Leu Gln Lys
   515 520 525
   Glu Asn Met Lys Ala Arg Met Leu Leu Gln Val His Asp Glu Leu Ile
   530 535 540
   Leu Glu Ala Pro Lys Asp Glu Ile Glu Arg Leu Gln Gln Ile Val Pro
   545 550 555 560
   Glu Val Met Glu Asn Ala Val Gln Leu Arg Val Pro Leu Lys Val Asp
   565 570 575
   Tyr His Phe Gly Pro Thr Trp Tyr Asp Ala Lys
   580 585
<210> 23
   <211> 587
   <212> PRT
   <213> Anoxybacillus flavithermus WK1
<400> 23
   Ser Gln Glu Gln Leu Ser Leu Thr Asp Ile Ser Phe Val Thr Val Gln
   1 5 10 15
   Thr Ile Asp Glu His Met Leu Thr Lys Glu Gly Ala Leu Val Val Glu
   20 25 30
   Val Leu Asp Glu Asn Tyr His Gln Ala Pro Ile Val Gly Phe Ala Leu
   35 40 45
   Val Asn Glu Arg Gly His Phe Phe Ile Pro Thr Asp Ile Ala Leu Ala
   50 55 60
   Ser Ser Arg Phe Lys Arg Trp Leu Glu Asp Glu Gln Cys Lys Lys Ser
   65 70 75 80
   Val Phe Asp Ala Lys Arg Ala Ile Val Ala Leu Lys Trp Asn Gly Ile
   85 90 95
   Glu Leu Lys Gly Val Asp Phe Asp Leu Leu Leu Ala Ala Tyr Leu Leu
   100 105 110
   Asn Pro Thr Asp Ala Asn Gly Asp Val Ala Ala Val Ala Lys Thr Lys
   115 120 125
   Gln Tyr Thr Asp Val Gln Ser Asp Glu Glu Val Tyr Gly Lys Gly Ala
   130 135 140
   Lys Gln Ala Ile Pro Pro Thr Asn Val Leu Ala Glu His Leu Val Arg
   145 150 155 160
   Lys Ala Lys Ala Ile Ala Ser Leu Lys Glu Thr Tyr Ile Gln Glu Leu
   165 170 175
   Lys Arg Asn Glu Gln Phe Glu Leu Leu Val His Leu Glu Leu Pro Leu
   180 185 190
   Thr Phe Ile Leu Ala Gln Met Glu Phe Tyr Gly Val Lys Val Asp Val
   195 200 205
   Asp Arg Leu Glu Gln Met Gly Lys Glu Phe Thr Ala Gln Leu Glu Gln
   210 215 220
   Ile Glu Gln Arg Ile Tyr Glu Leu Ala Gly Thr Thr Phe Asn Ile Asn
   225 230 235 240
   Ser Pro Lys Gln Leu Gly Thr Ile Leu Phe Glu Lys Leu Gln Leu Pro
   245 250 255
   Ile Val Lys Lys Thr Lys Thr Gly Tyr Ser Thr Ser Ala Asp Val Leu
   260 265 270
   Glu Lys Leu Ala Pro Tyr His Glu Ile Ile Glu Gln Ile Leu His Tyr
   275 280 285
   Arg Gln Leu Gly Lys Leu Gln Ser Thr Tyr Val Glu Gly Leu Met Lys
   290 295 300
   Val Val Arg Lys Asp Thr Gly Lys Val His Thr Ile Phe Gln Gln Ala
   305 310 315 320
   Leu Thr Gln Thr Gly Arg Leu Ser Ser Thr Glu Pro Asn Leu Gln Asn
   325 330 335
   Ile Pro Ile Arg Ile Glu Glu Gly Arg Lys Ile Arg Gln Ala Phe Val
   340 345 350
   Pro Ser Ser Asp Asp Trp Val Ile Phe Ala Ala Asp Tyr Ser Gln Ile
   355 360 365
   Glu Leu Arg Val Leu Ala His Ile Ala Asn Asp Glu Asn Leu Ile Ala
   370 375 380
   Ala Phe His His Asp Leu Asp Ile His Thr Lys Thr Ala Met Asp Ile
   385                 390                 395                 400
   Phe His Val Lys Glu Asp Glu Val Thr Ala His Met Arg Arg Gln Ala
                   405                 410                 415
   Lys Ala Val Asn Phe Gly Ile Val Tyr Gly Ile Ser Asp Tyr Gly Leu
               420                 425                 430
   Ser Gln Asn Leu Gly Ile Thr Arg Lys Glu Ala Ala Glu Phe Ile Glu
           435                 440                 445
   Arg Tyr Phe Arg Ser Tyr Pro Gly Val Lys Arg Tyr Met Glu Glu Val
       450                 455                 460
   Val Gln Asp Ala Lys Gln Lys Gly Tyr Val Thr Thr Leu Leu His Arg
   465                 470                 475                 480
   Arg Arg Tyr Leu Pro Asp Ile Thr Ser Gly Asn Phe Asn Val Arg Ser
                   485                 490                 495
   Phe Ala Glu Arg Thr Ala Met Asn Thr Pro Ile Gln Gly Ser Ala Ala
               500                 505                 510
   Asp Ile Ile Lys Lys Ala Met Ile Asp Leu Ala Asn Arg Leu His Glu
           515                 520                 525
   Glu Arg Leu Gln Thr Arg Leu Leu Leu Gln Val His Asp Glu Leu Ile
       530                 535                 540
   Leu Glu Ala Pro Lys Glu Glu Ile Glu Leu Leu Lys Lys Ile Val Pro
   545                 550                 555                 560
   Asp Val Met Glu Asn Ala Val Ser Leu Arg Val Pro Leu Lys Val Asp
                   565                 570                 575
   Tyr His Phe Gly Pro Thr Trp Tyr Asp Ala Lys
               580                 585
<210> 24
   <211> 31
   <212> PRT
   <213> Artificial Sequence
<220>
   <223> Synthetic construct
<400> 24
   Ala Glu Gly Glu Lys Pro Leu Glu Glu Met Glu Phe Ala Ile Val Asp
   1               5                   10                  15
   Val Ile Thr Glu Glu Met Leu Ala Asp Lys Ala Ala Leu Val Val
               20                  25                  30
<210> 25
   <211> 31
   <212> PRT
   <213> Artificial Sequence
<220>
   <223> Synthetic construct
<400> 25
   Ala Glu Glu Glu Lys Pro Leu Glu Asp Ile Glu Phe Glu Ile Ala Asp
   1               5                   10                  15
   Glu Val Thr Glu Glu Met Leu Ala Asp Glu Ala Ala Leu Val Val
               20                  25                  30
<210> 26
   <211> 31
   <212> PRT
   <213> Artificial Sequence
<220>
   <223> Synthetic construct
<400> 26
   Ala Glu Glu Glu Val Pro Leu Glu Glu Met Glu Phe Val Ile Ala Asp
   1               5                   10                  15
   Glu Ile Thr Glu Glu Met Leu Ala Asp Lys Ala Ala Leu Val Val
               20                  25                  30
<210> 27
   <211> 31
   <212> PRT
   <213> Artificial Sequence
<220>
   <223> Synthetic construct
<400> 27
   Ala Glu Glu Glu Val Pro Leu Glu Glu Met Glu Phe Thr Ile Ala Asp
   1               5                   10                  15
   Glu Ile Thr Glu Glu Met Leu Ala Asp Lys Ala Ala Leu Val Val
               20                  25                  30
<210> 28
   <211> 31
   <212> PRT
   <213> Artificial Sequence
<220>
   <223> Synthetic construct
<400> 28
   Ala Glu Glu Glu Lys Pro Leu Glu Glu Met Glu Phe Ala Ile Ala Asp
   1               5                   10                  15
   Glu Val Thr Glu Glu Met Leu Ala Asp Lys Ala Ala Leu Val Val
               20                  25                  30
<210> 29
   <211> 31
   <212> PRT
   <213> Artificial Sequence
<220>
   <223> Synthetic construct
<400> 29
   Ala Glu Asp Glu Lys Pro Leu Glu Glu Ile Glu Phe Ala Ile Ala Asp
   1               5                   10                  15
   Glu Ile Thr Glu Glu Met Leu Ala Asp Lys Ala Ala Leu Val Val
               20                  25                  30
<210> 30
   <211> 31
   <212> PRT
   <213> Artificial Sequence
<220>
   <223> Synthetic construct
<400> 30
   Ala Glu Glu Glu Lys Pro Leu Ala Glu Met Glu Phe Thr Ile Ala Asp
   1               5                   10                  15
   Glu Val Thr Glu Glu Met Leu Ala Asp Lys Ala Ala Leu Val Val
               20                  25                  30
<210> 31
   <211> 31
   <212> PRT
   <213> Artificial Sequence
<220>
   <223> Synthetic construct
<400> 31
   Ala Glu Glu Glu Val Pro Leu Ala Glu Met Glu Phe Val Ile Ala Asp
   1               5                   10                  15
   Glu Ile Thr Glu Glu Met Leu Ala Asp Lys Ala Ala Leu Val Val
               20                  25                  30
<210> 32
   <211> 31
   <212> PRT
   <213> Artificial Sequence
<220>
   <223> Synthetic construct
<400> 32
   Ala Glu Gly Glu Lys Pro Leu Ala Glu Met Glu Phe Ala Ile Val Asp
   1               5                   10                  15
   Glu Ile Thr Glu Glu Met Leu Ala Asp Lys Ala Ala Leu Val Val
               20                  25                  30
<210> 33
   <211> 31
   <212> PRT
   <213> Artificial Sequence
<220>
   <223> Synthetic construct
<400> 33
   Ala Glu Glu Glu Ala Pro Leu Glu Asp Ile Glu Phe Asp Ile Ala Asp
   1               5                   10                  15
   Glu Val Thr Glu Glu Met Leu Ala Asp Lys Ala Ala Leu Val Val
               20                  25                  30
<210> 34
   <211> 31
   <212> PRT
   <213> Artificial Sequence
<220>
   <223> Synthetic construct
<400> 34
   Ala Glu Glu Glu Lys Pro Leu Ala Glu Met Glu Phe Ala Ile Ala Asp
   1               5                   10                  15
   Glu Val Thr Glu Glu Met Leu Ala Asp Lys Ala Ala Leu Val Val
               20                  25                  30
<210> 35
   <211> 31
   <212> PRT
   <213> Artificial Sequence
<220>
   <223> Synthetic construct
<400> 35
   Glu Glu Glu Glu Val Pro Leu Glu Glu Ile Glu Phe Ala Ile Ala Asp
   1               5                   10                  15
   Glu Val Thr Glu Glu Met Leu Ala Asp Lys Ala Ala Leu Val Val
               20                  25                  30
<210> 36
   <211> 31
   <212> PRT
   <213> Artificial Sequence
<220>
   <223> Synthetic construct
<400> 36
   Ala Glu Asp Glu Lys Pro Leu Ala Glu Met Glu Phe Val Ile Ala Asp
   1               5                   10                  15
   Gly Ile Thr Asp Glu Met Leu Ala Asp Lys Ala Ala Leu Val Val
               20                  25                  30
<210> 37
   <211> 31
   <212> PRT
   <213> Artificial Sequence
<220>
   <223> Synthetic construct
<400> 37
   Ala Glu Lys Glu Leu Pro Leu Met Glu Met Glu Phe Ala Asp Ala Asp
   1               5                   10                  15
   Thr Ile Thr Met Glu Met Leu Ala Asp Lys Ala Ala Leu Val Val
               20                  25                  30
<210> 38
   <211> 31
   <212> PRT
   <213> Artificial Sequence
<220>
   <223> Synthetic construct
<400> 38
   Ala Glu Glu Glu Lys Pro Leu Ala Glu Met Glu Phe Val Ile Ala Asp
   1               5                   10                  15
   Gly Ile Thr Glu Glu Met Leu Ala Asp Lys Ala Ala Leu Val Val
               20                  25                  30
<210> 39
   <211> 31
   <212> PRT
   <213> Artificial Sequence
<220>
   <223> Synthetic construct
<400> 39
   Ala Glu Glu Glu Lys Pro Leu Ala Glu Met Glu Phe Ala Ile Ala Asp
   1               5                   10                  15
   Glu Ile Thr Glu Glu Met Leu Ala Asp Lys Ala Ala Leu Val Val
               20                  25                  30
<210> 40
   <211> 80
   <212> PRT
   <213> Artificial Sequence
<220>
   <223> Synthetic construct
<400> 40
   Glu Val Met Glu Glu Asn Tyr His Asp Ala Pro Ile Val Gly Ile Ala
   1               5                   10                  15
   Leu Val Asn Glu His Gly Arg Phe Phe Met Arg Pro Glu Thr Ala Leu
               20                  25                  30
   Ala Asp Ser Gln Phe Leu Ala Trp Leu Ala Asp Glu Thr Lys Lys Lys
           35                  40                  45
   Ser Met Phe Asp Ala Lys Arg Ala Val Val Ala Leu Lys Trp Lys Gly
       50                  55                  60
   Ile Glu Leu Arg Gly Val Ala Phe Asp Leu Leu Leu Ala Ala Tyr Leu
   65                  70                  75                  80
<210> 41
   <211> 80
   <212> PRT
   <213> Artificial Sequence
<220>
   <223> Synthetic construct
<400> 41
   Glu Val Gln Glu Glu Asn Tyr His Asp Ala Pro Ile Val Gly Ile Ala
   1               5                   10                  15
   Ile Val Asn Glu His Gly Arg Phe Phe Leu Arg Ala Glu Thr Ala Leu
               20                  25                  30
   Ala Asp Phe Gln Phe Val Ala Trp Leu Glu Asp Glu Thr Lys Lys Lys
           35                  40                  45
   Ser Met Phe Asp Ala Lys Arg Ala Val Val Ala Leu Lys Trp Lys Gly
       50                  55                  60
   Ile Glu Leu Val Gly Val Asp Phe Asp Leu Leu Leu Ala Ala Tyr Leu
   65                  70                  75                  80
<210> 42
   <211> 80
   <212> PRT
   <213> Artificial Sequence
<220>
   <223> Synthetic construct
<400> 42
   Glu Val Leu Glu Glu Asn Tyr His Asp Ala Pro Ile Val Gly Ile Ala
   1               5                   10                  15
   Leu Val Asn Glu His Gly Arg Phe Phe Leu Arg Pro Glu Thr Ala Leu
               20                  25                  30
   Ala Asp Pro Gln Phe Val Ala Trp Leu Glu Asp Glu Thr Lys Lys Lys
           35                  40                  45
   Ser Met Phe Asp Ala Lys Arg Ala Ala Val Ala Leu Lys Trp Lys Gly
       50                  55                  60
   Ile Glu Leu Arg Gly Val Ala Phe Asp Leu Leu Leu Ala Ala Tyr Leu
   65                  70                  75                  80
<210> 43
   <211> 80
   <212> PRT
   <213> Artificial Sequence
<220>
   <223> Synthetic construct
<400> 43
   Glu Val Met Glu Glu Asn Tyr His Asp Ala Pro Ile Val Gly Ile Ala
   1               5                   10                  15
   Leu Val Asn Glu His Gly Arg Phe Phe Leu Arg Ala Glu Thr Ala Leu
               20                  25                  30
   Ala Asp Pro Gln Phe Val Ala Trp Leu Ala Asp Glu Thr Lys Lys Lys
           35                  40                  45
   Ser Met Phe Asp Ala Lys Arg Ala Ala Val Ala Leu Lys Trp Lys Gly
       50                  55                  60
   Ile Glu Leu Arg Gly Val Ala Phe Asp Leu Leu Leu Ala Ala Tyr Leu
   65                  70                  75                  80
<210> 44
   <211> 80
   <212> PRT
   <213> Artificial Sequence
<220>
   <223> Synthetic construct
<400> 44
   Glu Val Met Glu Glu Asn Tyr His Asp Ala Pro Ile Val Gly Ile Ala
   1               5                   10                  15
   Leu Val Asn Glu His Gly Arg Phe Phe Leu Arg Pro Glu Thr Ala Leu
               20                  25                  30
   Ala Asp Pro Gln Phe Leu Ala Trp Leu Ala Asp Glu Thr Lys Lys Lys
           35                  40                  45
   Ser Met Phe Asp Ala Lys Arg Ala Ala Val Ala Leu Lys Trp Lys Gly
       50                  55                  60
   Ile Glu Leu Arg Gly Val Ala Phe Asp Leu Leu Leu Ala Ala Tyr Leu
   65                  70                  75                  80
<210> 45
   <211> 80
   <212> PRT
   <213> Artificial Sequence
<220>
   <223> Synthetic construct
<400> 45
   Glu Val Leu Glu Glu Asn Tyr His Asp Ala Pro Ile Val Gly Phe Ala
   1               5                   10                  15
   Ile Val Asn Glu His Gly Arg Phe Phe Leu Arg Thr Glu Leu Ala Leu
               20                  25                  30
   Ala Asp Phe Gln Phe Val Ala Trp Leu Glu Asp Glu Thr Lys Lys Lys
           35                  40                  45
   Ser Met Phe Asp Arg Lys Arg Ala Val Val Ala Leu Lys Trp Lys Gly
       50                  55                  60
   Ile Glu Leu Val Gly Val Asp Phe Asp Leu Leu Leu Ala Ala Tyr Leu
   65                  70                  75                  80
<210> 46
   <211> 80
   <212> PRT
   <213> Artificial Sequence
<220>
   <223> Synthetic construct
<400> 46
   Glu Val Gln Glu Asp Asn Tyr His Asp Ala Pro Ile Val Gly Phe Ala
   1 5 10 15
   Ile Val Asn Glu His Gly Arg Phe Phe Ile Arg Thr Glu Thr Ala Leu
   20 25 30
   Ala Ser Glu Ala Phe Lys Ala Trp Leu Ala Asp Glu Thr Lys Lys Lys
   35 40 45
   Ser Val Phe Asp Ala Lys Arg Ala Ile Val Ala Leu Lys Trp Lys Gly
   50 55 60
   Ile Glu Leu Arg Gly Val Asp Phe Asp Leu Leu Leu Ala Ala Tyr Leu
   65 70 75 80
<210> 47
   <211> 80
   <212> PRT
   <213> Artificial Sequence
<220>
   <223> Synthetic construct
<400> 47
   Glu Val Met Glu Glu Asn Tyr His Asp Ala Pro Ile Val Gly Ile Ala
   1 5 10 15
   Leu Val Asn Glu His Gly Arg Phe Phe Leu Arg Pro Glu Thr Ala Leu
   20 25 30
   Ala Asp Pro Gln Phe Leu Ala Trp Leu Ala Asp Glu Thr Lys Lys Lys
   35 40 45
   Ser Met Phe Asp Ala Lys Arg Ala Ile Val Ala Leu Lys Trp Lys Gly
   50 55 60
   Ile Glu Leu Arg Gly Val Ala Phe Asp Leu Leu Leu Ala Ala Tyr Leu
   65 70 75 80
<210> 48
   <211> 80
   <212> PRT
   <213> Artificial Sequence
<220>
   <223> Synthetic construct
<400> 48
   Glu Val Met Glu Glu Asn Tyr His Asp Ala Pro Ile Val Gly Ile Ala
   1               5                   10                  15
   Asn Val Asn Glu His Gly Arg Phe Phe Leu Arg Thr Glu Leu Ala Leu
               20                  25                  30
   Ala Asp Phe Gln Phe Val Ala Trp Leu Glu Asp Glu Thr Lys Lys Lys
           35                  40                  45
   Ser Met Phe Asp Arg Lys Arg Ala Ala Val Ala Leu Lys Trp Lys Gly
       50                  55                  60
   Ile Glu Leu Val Gly Val Asp Phe Asp Leu Leu Leu Ala Ala Tyr Leu
   65                  70                  75                  80
<210> 49
   <211> 80
   <212> PRT
   <213> Artificial Sequence
<220>
   <223> Synthetic construct
<400> 49
   Glu Val Leu Glu Glu Asn Tyr His Asp Ala Pro Ile Val Gly Phe Ala
   1               5                   10                  15
   Leu Val Asn Glu His Gly Arg Phe Phe Ile Arg Thr Glu Thr Ala Leu
               20                  25                  30
   Ala Ser Ser Gln Phe Lys Ala Trp Leu Glu Asp Glu Thr Lys Lys Lys
           35                  40                  45
   Ser Val Phe Asp Ala Lys Arg Ala Ile Val Ala Leu Lys Trp Lys Gly
       50                  55                  60
   Ile Glu Leu Arg Gly Val Asp Phe Asp Leu Leu Leu Ala Ala Tyr Leu
   65                  70                  75                  80
<210> 50
   <211> 80
   <212> PRT
   <213> Artificial Sequence
<220>
   <223> Synthetic construct
<400> 50
   Glu Val Leu Glu Glu Asn Tyr His Asp Ala Pro Ile Val Gly Phe Ala
   1               5                   10                  15
   Leu Val Asn Glu His Gly Arg Phe Phe Leu Arg Pro Glu Thr Ala Leu
               20                  25                  30
   Ala Asp Glu Gln Phe Val Ala Trp Leu Ala Asp Glu Thr Lys Lys Lys
           35                  40                  45
   Ser Val Phe Asp Ala Lys Arg Ala Val Val Ala Leu Lys Trp Lys Gly
       50                  55                  60
   Ile Glu Leu Arg Gly Val Asp Phe Asp Leu Leu Leu Ala Ala Tyr Leu
   65                  70                  75                  80
<210> 51
   <211> 80
   <212> PRT
   <213> Artificial Sequence
<220>
   <223> Synthetic construct
<400> 51
   Glu Val Met Glu Glu Asn Tyr His Asp Ala Pro Ile Val Gly Ile Ala
   1               5                   10                  15
   Leu Val Asn Glu His Gly Arg Phe Phe Leu Arg Pro Glu Thr Ala Leu
               20                  25                  30
   Ala Asp Pro Gln Phe Val Ala Trp Leu Ala Asp Glu Thr Lys Lys Lys
           35                  40                  45
   Ser Met Phe Asp Ala Lys Arg Ala Ala Val Ala Leu Lys Trp Lys Gly
       50                  55                  60
   Ile Glu Leu Arg Gly Val Ala Phe Asp Leu Leu Leu Ala Ala Tyr Leu
   65                  70                  75                  80
<210> 52
   <211> 80
   <212> PRT
   <213> Artificial Sequence
<220>
   <223> Synthetic construct
<400> 52
   Glu Val Met Glu Glu Asn Tyr His Asp Ala Pro Ile Val Gly Ile Ala
   1               5                   10                  15
   Leu Val Asn Glu His Gly Arg Phe Phe Leu Arg Pro Glu Thr Ala Leu
               20                  25                  30
   Ala Ser Pro Gln Phe Lys Ala Trp Leu Ala Asp Glu Thr Lys Lys Lys
           35                  40                  45
   Ser Met Phe Asp Ala Lys Arg Ala Ile Val Ala Leu Lys Trp Lys Gly
       50                  55                  60
   Ile Glu Leu Arg Gly Val Ala Phe Asp Leu Leu Leu Ala Ala Tyr Leu
   65                  70                  75                  80
<210> 53
   <211> 80
   <212> PRT
   <213> Artificial Sequence
<220>
   <223> Synthetic construct
<400> 53
   Glu Val Leu Glu Glu Asn Tyr His Asp Ala Pro Ile Val Gly Phe Ala
   1               5                   10                  15
   Ile Val Asn Glu His Gly Arg Phe Phe Ile Arg Pro Glu Thr Ala Leu
               20                  25                  30
   Ala Ser Ser Gln Phe Lys Ala Trp Leu Glu Asp Glu Thr Lys Lys Lys
           35                  40                  45
   Ser Met Phe Asp Ala Lys Arg Ala Ala Val Ala Leu Lys Trp Lys Gly
       50                  55                  60
   Ile Glu Leu Arg Gly Val Ala Phe Asp Leu Leu Leu Ala Ala Tyr Leu
   65                  70                  75                  80
<210> 54
   <211> 80
   <212> PRT
   <213> Artificial Sequence
<220>
   <223> Synthetic construct
<400> 54
   Glu Val Met Glu Glu Asn Tyr His Asp Ala Pro Ile Val Gly Ile Ala
   1               5                   10                  15
   Leu Val Asn Glu His Gly Arg Phe Phe Leu Arg Thr Glu Thr Ala Leu
               20                  25                  30
   Ala Asp Pro Gln Phe Lys Ala Trp Leu Ala Asp Glu Thr Lys Lys Lys
           35                  40                  45
   Ser Met Phe Asp Ala Lys Arg Ala Ile Val Ala Leu Lys Trp Lys Gly
       50                  55                  60
   Ile Glu Leu Arg Gly Val Asp Phe Asp Leu Leu Leu Ala Ala Tyr Leu
   65                  70                  75                  80
<210> 55
   <211> 80
   <212> PRT
   <213> Artificial Sequence
<220>
   <223> Synthetic construct
<400> 55
   Glu Val Gln Glu Asp Asn Tyr His Asp Ala Pro Ile Val Gly Phe Ala
   1               5                   10                  15
   Asn Val Asn Glu His Gly Arg Phe Phe Leu Arg Thr Glu Thr Ala Leu
               20                  25                  30
   Ala Ser Glu Ala Phe Ala Ala Trp Leu Glu Asp Glu Thr Lys Lys Lys
           35                  40                  45
   Ser Val Phe Asp Ala Lys Arg Ala Val Val Ala Leu Lys Trp Lys Gly
       50                  55                  60
   Ile Glu Leu Val Gly Val Ala Phe Asp Leu Leu Leu Ala Ala Tyr Leu
   65                  70                  75                  80
<210> 56
   <211> 80
   <212> PRT
   <213> Artificial Sequence
<220>
   <223> Synthetic construct
<400> 56
   Glu Val Leu Glu Glu Asn Tyr His Asp Ala Pro Ile Val Gly Ile Ala
   1 5 10 15
   Leu Val Asn Glu His Gly Arg Phe Phe Leu Arg Pro Glu Thr Ala Leu
   20 25 30
   Ala Asp Ser Gln Phe Leu Ala Trp Leu Glu Asp Glu Thr Lys Lys Lys
   35 40 45
   Ser Met Phe Asp Ala Lys Arg Ala Ala Val Ala Leu Lys Trp Lys Gly
   50 55 60
   Ile Glu Leu Arg Gly Val Ala Phe Asp Leu Leu Leu Ala Ala Tyr Leu
   65 70 75 80
<210> 57
   <211> 80
   <212> PRT
   <213> Artificial Sequence
<220>
   <223> Synthetic construct
<400> 57
   Leu Asn Pro Ala Gln Asp Ala Gly Asp Ile Ala Ala Val Ala Lys Met
   1 5 10 15
   Lys Gln Tyr Glu Ala Val Arg Ser Asp Glu Ala Val Tyr Gly Lys Gly
   20 25 30
   Val Lys Arg Ser Leu Pro Asp Glu Gln Thr Leu Ala Glu His Leu Val
   35 40 45
   Arg Lys Ala Ala Ala Ile Trp Ala Leu Glu Gln Pro Phe Met Asp Asp
   50 55 60
   Leu Arg Asn Asn Glu Gln Asp Gln Leu Leu Thr Lys Leu Glu Gln Pro
   65 70 75 80
<210> 58
   <211> 80
   <212> PRT
   <213> Artificial Sequence
<220>
   <223> Synthetic construct
<400> 58
   Leu Asn Pro Ala Gln Asp Ala Gly Asp Val Ala Ala Val Ala Lys Met
   1               5                   10                  15
   Lys Gln Tyr Glu Ala Val Arg Ser Asp Glu Ala Val Tyr Gly Lys Gly
               20                  25                  30
   Ala Lys Arg Ser Leu Pro Asp Glu Pro Val Leu Ala Glu His Leu Val
           35                  40                  45
   Arg Lys Ala Ala Ala Ile Trp Ala Leu Glu Arg Pro Phe Leu Asp Glu
       50                  55                  60
   Leu Arg Glu Asn Glu Gln Asp Glu Leu Leu Thr Asp Leu Glu Gln Pro
   65                  70                  75                  80
<210> 59
   <211> 80
   <212> PRT
   <213> Artificial Sequence
<220>
   <223> Synthetic construct
<400> 59
   Leu Asn Pro Ala Gln Ala Ala Gly Asp Val Ala Ala Val Ala Lys Met
   1               5                   10                  15
   Lys Gln Tyr Glu Ala Val Arg Ser Asp Glu Ala Val Tyr Gly Lys Gly
               20                  25                  30
   Ala Lys Arg Ala Val Pro Asp Glu Pro Val Leu Ala Glu His Leu Val
           35                  40                  45
   Arg Lys Ala Ala Ala Ile Trp Ala Leu Glu Glu Pro Phe Ile Asp Glu
       50                  55                  60
   Leu Arg Arg Asn Glu Gln Asp Arg Leu Leu Thr Asp Leu Glu Gln Pro
   65                  70                  75                  80
<210> 60
   <211> 80
   <212> PRT
   <213> Artificial Sequence
<220>
   <223> Synthetic construct
<400> 60
   Leu Asn Pro Ala Gln Asp Ala Gly Asp Val Ala Ala Val Ala Lys Met
   1 5 10 15
   Lys Gln Tyr Glu Ala Val Arg Ser Asp Glu Ala Val Tyr Gly Lys Gly
   20 25 30
   Ala Lys Arg Ala Val Pro Asp Glu Pro Thr Leu Ala Glu His Leu Val
   35 40 45
   Arg Lys Ala Ala Ala Ile Trp Ala Leu Glu Arg Pro Phe Leu Asp Glu
   50 55 60
   Leu Arg Arg Asn Glu Gln Asp Glu Leu Leu Ile Lys Leu Glu Gln Pro
   65 70 75 80
<210> 61
   <211> 80
   <212> PRT
   <213> Artificial Sequence
<220>
   <223> Synthetic construct
<400> 61
   Leu Ala Pro Ala Gln Asp Asp Gly Asp Ala Ala Ala Lys Ala Lys Met
   1 5 10 15
   Lys Gln Tyr Glu Ala Val Arg Glu Asp Glu Ala Val Tyr Gly Lys Gly
   20 25 30
   Ala Lys Arg Pro Asp Pro Asp Glu Leu Ala Leu Ala Glu His Leu Val
   35 40 45
   Arg Lys Ala Ala Ala Ile Trp Ala Leu Glu Arg Pro Phe Leu Asp Glu
   50 55 60
   Leu Arg Glu Asn Glu Gln Asp Leu Leu Leu Leu Glu Leu Glu Gln Pro
   65 70 75 80
<210> 62
   <211> 80
   <212> PRT
   <213> Artificial Sequence
<220>
   <223> Synthetic construct
<400> 62
   Leu Asn Pro Ala Gln Asp Ala Gly Asp Val Ala Ala Val Ala Lys Met
   1 5 10 15
   Lys Gln Tyr Glu Ala Val Arg Ser Asp Glu Ala Val Tyr Gly Lys Gly
   20 25 30
   Ala Lys Arg Ser Leu Pro Asp Glu Pro Thr Leu Ala Glu His Leu Val
   35 40 45
   Arg Lys Ala Ala Ala Ile Trp Ala Leu Glu Arg Pro Phe Leu Asp Glu
   50 55 60
   Leu Arg Glu Asn Glu Gln Asp Glu Leu Leu Ile Lys Leu Glu Gln Pro
   65 70 75 80
<210> 63
   <211> 80
   <212> PRT
   <213> Artificial Sequence
<220>
   <223> Synthetic construct
<400> 63
   Leu Asn Pro Ala Gln Thr Ala Asp Asp Val Ala Ala Val Ala Lys Met
   1 5 10 15
   Lys Gln Tyr His Ala Val Arg Ser Asp Glu Ala Val Tyr Gly Lys Gly
   20 25 30
   Ala Lys Arg Ala Val Pro Asp Glu Pro Val Leu Ala Glu His Leu Val
   35 40 45
   Arg Lys Ala Ala Ala Ile Trp Ala Leu Glu Glu Pro Phe Leu Asp Glu
   50 55 60
   Leu Arg Lys Asn Glu Gln Asp Glu Leu Phe Thr Glu Leu Glu Leu Pro
   65 70 75 80
<210> 64
   <211> 80
   <212> PRT
   <213> Artificial Sequence
<220>
   <223> Synthetic construct
<400> 64
   Leu Asn Pro Ala Gln Asp Ala Gly Asp Val Ala Ala Val Ala Lys Met
   1               5                   10                  15
   Lys Gln Tyr Glu Ala Val Arg Ser Asp Glu Ala Val Tyr Gly Lys Gly
               20                  25                  30
   Ala Lys Arg Ser Leu Pro Asp Glu Pro Thr Leu Ala Glu His Leu Val
           35                  40                  45
   Arg Lys Ala Ala Ala Ile Trp Ala Leu Glu Gln Pro Phe Ile Asp Glu
       50                  55                  60
   Leu Arg Arg Asn Glu Gln Asp Glu Leu Leu Thr Lys Leu Glu Gln Pro
   65                  70                  75                  80
<210> 65
   <211> 80
   <212> PRT
   <213> Artificial Sequence
<220>
   <223> Synthetic construct
<400> 65
   Leu Asn Pro Ala Gln Ser Ala Glu Asp Val Ala Ala Val Ala Lys Met
   1               5                   10                  15
   Lys Gln Tyr Glu Ala Val Arg Ser Asp Glu Ala Val Tyr Gly Lys Gly
               20                  25                  30
   Ala Lys Arg Ala Val Pro Asp Glu Pro Val Leu Ala Glu His Leu Val
           35                  40                  45
   Arg Lys Ala Ala Ala Ile Trp Ala Leu Glu Glu Pro Phe Ile Asp Glu
       50                  55                  60
   Leu Arg Glu Asn Glu Gln Asp Glu Leu Phe Thr Asp Leu Glu Gln Pro
   65                  70                  75                  80
<210> 66
   <211> 80
   <212> PRT
   <213> Artificial Sequence
<220>
   <223> Synthetic construct
<400> 66
   Leu Asn Pro Ala Gln Asp Ala Gly Asp Val Ala Ala Val Ala Lys Met
   1               5                   10                  15
   Lys Gln Tyr Glu Ala Val Arg Ser Asp Glu Ala Val Tyr Gly Lys Gly
               20                  25                  30
   Ala Lys Arg Ala Leu Pro Asp Glu Pro Thr Leu Ala Glu His Leu Val
           35                  40                  45
   Arg Lys Ala Ala Ala Ile Trp Ala Leu Glu Glu Pro Phe Leu Asp Glu
       50                  55                  60
   Leu Arg Glu Asn Glu Gln Asp Glu Leu Leu Thr Glu Leu Glu Gln Pro
   65                  70                  75                  80
<210> 67
   <211> 80
   <212> PRT
   <213> Artificial Sequence
<220>
   <223> Synthetic construct
<400> 67
   Leu Asn Pro Ala Gln Ser Ala Gly Asp Val Ala Ala Val Ala Lys Met
   1               5                   10                  15
   Lys Gln Tyr Glu Ala Val Arg Ser Asp Glu Ala Val Tyr Gly Lys Gly
               20                  25                  30
   Ala Lys Arg Ala Val Pro Asp Glu Pro Thr Leu Ala Glu His Leu Val
           35                  40                  45
   Arg Lys Ala Ala Ala Ile Trp Ala Leu Glu Glu Pro Phe Ile Asp Glu
       50                  55                  60
   Leu Arg Glu Asn Glu Gln Asp Glu Leu Phe Thr Glu Leu Glu Met Pro
   65                  70                  75                  80
<210> 68
   <211> 80
   <212> PRT
   <213> Artificial Sequence
<220>
   <223> Synthetic construct
<400> 68
   Leu Asn Pro Ala Gln Asp Ala Gly Asp Val Ala Ala Val Ala Lys Met
   1               5                   10                  15
   Lys Gln Tyr Glu Ala Val Arg Pro Asp Glu Ala Val Tyr Gly Lys Gly
               20                  25                  30
   Ala Lys Arg Ser Leu Pro Asp Glu Pro Thr Leu Ala Glu His Leu Val
           35                  40                  45
   Arg Lys Ala Ala Ala Ile Trp Ala Leu Glu Arg Pro Phe Leu Asp Glu
       50                  55                  60
   Leu Arg Arg Asn Glu Gln Asp Arg Leu Leu Ile Lys Leu Glu Gln Pro
   65                  70                  75                  80
<210> 69
   <211> 80
   <212> PRT
   <213> Artificial Sequence
<220>
   <223> Synthetic construct
<400> 69
   Leu Asn Pro Ala Gln Asp Ala Gly Asp Val Ala Ala Val Ala Lys Met
   1               5                   10                  15
   Lys Gln Tyr Glu Ala Val Arg Ser Asp Glu Ala Val Tyr Gly Lys Gly
               20                  25                  30
   Ala Lys Arg Ser Leu Pro Asp Glu Pro Thr Leu Ala Glu His Leu Val
           35                  40                  45
   Arg Lys Ala Ala Ala Ile Trp Ala Leu Glu Arg Pro Phe Leu Asp Glu
       50                  55                  60
   Leu Arg Arg Asn Glu Gln Asp Glu Leu Leu Thr Lys Leu Glu Gln Pro
   65                  70                  75                  80
<210> 70
   <211> 80
   <212> PRT
   <213> Artificial Sequence
<220>
   <223> Synthetic construct
<400> 70
   Leu Asn Pro Ala Gln Asp Ala Gly Asp Val Ala Ala Val Ala Lys Met
   1               5                   10                  15
   Lys Gln Tyr Glu Ala Val Arg Ser Asp Glu Ala Val Tyr Gly Lys Gly
               20                  25                  30
   Ala Lys Arg Ala Val Pro Asp Glu Pro Val Leu Ala Glu His Leu Val
           35                  40                  45
   Arg Lys Ala Ala Ala Ile Trp Ala Leu Glu Arg Pro Phe Leu Asp Glu
       50                  55                  60
   Leu Arg Asn Asn Glu Gln Asp Glu Leu Leu Thr Glu Leu Glu Gln Pro
   65                  70                  75                  80
<210> 71
   <211> 80
   <212> PRT
   <213> Artificial Sequence
<220>
   <223> Synthetic construct
<400> 71
   Leu Asn Pro Ala Gln Asp Ala Gly Asp Val Ala Ala Val Ala Lys Met
   1               5                   10                  15
   Lys Gln Tyr Glu Ala Val Arg Ser Asp Glu Ala Val Tyr Gly Lys Gly
               20                  25                  30
   Ala Lys Arg Ser Val Pro Asp Glu Pro Thr Leu Ala Glu His Leu Val
           35                  40                  45
   Arg Lys Ala Ala Ala Ile Trp Ala Leu Glu Gln Pro Phe Leu Asp Glu
       50                  55                  60
   Leu Arg Arg Asn Glu Gln Asp Arg Leu Leu Thr Lys Leu Glu Gln Pro
   65                  70                  75                  80
<210> 72
   <211> 80
   <212> PRT
   <213> Artificial Sequence
<220>
   <223> Synthetic construct
<400> 72
   Leu Asn Pro Ala Gln Ser Ala Gly Asp Val Ala Ala Val Ala Lys Met
   1               5                   10                  15
   Lys Gln Tyr Glu Ala Val Arg Ser Asp Glu Ala Val Tyr Gly Lys Gly
               20                  25                  30
   Ala Lys Arg Ala Val Pro Asp Glu Pro Thr Leu Ala Glu His Leu Val
           35                  40                  45
   Arg Lys Ala Ala Ala Ile Trp Ala Leu Glu Gln Pro Phe Leu Asp Glu
       50                  55                  60
   Leu Arg Glu Asn Glu Gln Asp Glu Leu Leu Thr Lys Leu Glu Gln Pro
   65                  70                  75                  80
<210> 73
   <211> 80
   <212> PRT
   <213> Artificial Sequence
<220>
   <223> Synthetic construct
<400> 73
   Leu Ala Ala Ile Leu Ala Glu Met Glu Phe Thr Gly Val Asn Val Asp
   1               5                   10                  15
   Thr Lys Arg Leu Glu Gln Met Gly Ser Glu Leu Ala Glu Gln Leu Arg
               20                  25                  30
   Ala Ile Glu Gln Arg Ile Tyr Glu Leu Ala Gly Gln Glu Phe Asn Ile
           35                  40                  45
   Asn Ser Pro Lys Gln Leu Gly Val Ile Leu Phe Glu Lys Leu Gln Leu
       50                  55                  60
   Pro Val Leu Lys Lys Thr Lys Thr Gly Tyr Ser Thr Ser Ala Asp Val
   65                  70                  75                  80
<210> 74
   <211> 80
   <212> PRT
   <213> Artificial Sequence
<220>
   <223> Synthetic construct
<400> 74
   Leu Ala Thr Ile Leu Ala Glu Met Glu Phe Thr Gly Val Lys Val Asp
   1               5                   10                  15
   Thr Lys Arg Leu Glu Gln Met Gly Ser Glu Leu Ala Glu Gln Leu Arg
               20                  25                  30
   Ala Val Glu Gln Arg Ile Tyr Glu Leu Ala Gly Gln Glu Phe Asn Ile
           35                  40                  45
   Asn Ser Pro Lys Gln Leu Gly Val Ile Leu Phe Glu Lys Leu Gln Leu
       50                  55                  60
   Pro Val Leu Lys Lys Thr Lys Thr Gly Tyr Ser Thr Ser Ala Asp Val
   65                  70                  75                  80
<210> 75
   <211> 80
   <212> PRT
   <213> Artificial Sequence
<220>
   <223> Synthetic construct
<400> 75
   Leu Ala Thr Ile Leu Ala Glu Met Glu Phe Thr Gly Val Lys Val Asp
   1               5                   10                  15
   Thr Lys Arg Leu Glu Gln Met Gly Glu Glu Leu Ala Glu Gln Leu Gly
               20                  25                  30
   Ala Val Glu Gln Arg Ile Tyr Glu Leu Ala Gly Gln Glu Phe Asn Ile
           35                  40                  45
   Asn Ser Pro Lys Gln Leu Gly Val Ile Leu Phe Glu Lys Leu Gln Leu
       50                  55                  60
   Pro Val Leu Lys Lys Thr Lys Thr Gly Tyr Ser Thr Ser Ala Asp Val
   65                  70                  75                  80
<210> 76
   <211> 80
   <212> PRT
   <213> Artificial Sequence
<220>
   <223> Synthetic construct
<400> 76
   Leu Ile Leu Ile Leu Ala Glu Met Glu Phe Thr Gly Val Asp Val Asp
   1 5 10 15
   Thr Lys Arg Leu Glu Gln Met Gly Leu Glu Leu Ala Glu Gln Leu Val
   20 25 30
   Glu Gln Glu Gln Arg Ile Tyr Glu Leu Ala Gly Gln Glu Phe Asn Ile
   35 40 45
   Asn Ser Pro Lys Gln Leu Gly Leu Ile Leu Phe Glu Lys Leu Gln Leu
   50 55 60
   Pro Val Leu Lys Lys Thr Lys Thr Gly Tyr Ser Thr Ser Ala Asp Val
   65 70 75 80
<210> 77
   <211> 80
   <212> PRT
   <213> Artificial Sequence
<220>
   <223> Synthetic construct
<400> 77
   Leu Ser Leu Ile Leu Ala Glu Met Glu Phe Thr Gly Val Asp Val Asp
   1 5 10 15
   Thr Lys Arg Leu Glu Gln Met Gly Leu Glu Leu Ala Glu Gln Leu Gly
   20 25 30
   Ala Gln Glu Gln Arg Ile Tyr Glu Leu Ala Gly Gln Glu Phe Asn Ile
   35 40 45
   Asn Ser Pro Lys Gln Leu Gly Val Ile Leu Phe Glu Lys Leu Gln Leu
   50 55 60
   Pro Val Leu Lys Lys Thr Lys Thr Gly Tyr Ser Thr Ser Ala Asp Val
   65 70 75 80
<210> 78
   <211> 78
   <212> PRT
   <213> Artificial Sequence
<220>
   <223> Synthetic construct
<400> 78
   Leu Ala Leu Ile Leu Ala Glu Met Glu Phe Thr Gly Val Lys Val Asp
   1               5                   10                  15
   Thr Lys Arg Leu Glu Gln Met Gly Glu Glu Leu Ala Glu Gln Leu Lys
               20                  25                  30
   Glu Val Glu Gln Arg Ile Tyr Glu Leu Ala Gly Gln Glu Phe Asn Ile
           35                  40                  45
   Asn Ser Pro Lys Gln Leu Gly Val Ile Leu Phe Glu Lys Leu Gln Leu
       50                  55                  60
   Pro Val Leu Lys Lys Thr Lys Thr Gly Tyr Ser Thr Ser Ala
   65                  70                  75
<210> 79
   <211> 80
   <212> PRT
   <213> Artificial Sequence
<220>
   <223> Synthetic construct
<400> 79
   Leu Ile Leu Ile Leu Ala Glu Met Glu Phe Thr Gly Val Asp Val Asp
   1               5                   10                  15
   Thr Lys Arg Leu Glu Gln Met Gly Ser Glu Leu Ala Glu Gln Leu Gly
               20                  25                  30
   Ala Ile Glu Gln Arg Ile Tyr Glu Leu Ala Gly Gln Glu Phe Asn Ile
           35                  40                  45
   Asn Ser Pro Lys Gln Leu Gly Leu Ile Leu Phe Glu Lys Leu Gln Leu
       50                  55                  60
   Pro Val Leu Lys Lys Thr Lys Thr Gly Tyr Ser Thr Ser Ala Asp Val
   65                  70                  75                  80
<210> 80
   <211> 80
   <212> PRT
   <213> Artificial Sequence
<220>
   <223> Synthetic construct
<400> 80
   Leu Ala Ala Ile Leu Ala Glu Met Glu Phe Thr Gly Val Lys Val Asp
   1               5                   10                  15
   Thr Lys Arg Leu Glu Gln Met Gly Glu Glu Leu Ala Glu Gln Leu Lys
               20                  25                  30
   Glu Val Glu Gln Arg Ile Tyr Glu Leu Ala Gly Gln Glu Phe Asn Ile
           35                  40                  45
   Asn Ser Pro Lys Gln Leu Gly Val Ile Leu Phe Glu Lys Leu Gln Leu
       50                  55                  60
   Pro Val Leu Lys Lys Thr Lys Thr Gly Tyr Ser Thr Ser Ala Asp Val
   65                  70                  75                  80
<210> 81
   <211> 80
   <212> PRT
   <213> Artificial Sequence
<220>
   <223> Synthetic construct
<400> 81
   Leu Ile Leu Ile Leu Ala Glu Met Glu Phe Thr Gly Val Asp Val Asp
   1               5                   10                  15
   Thr Lys Arg Leu Glu Gln Met Gly Leu Glu Leu Ala Glu Gln Leu Val
               20                  25                  30
   Ala Gln Glu Gln Arg Ile Tyr Glu Leu Ala Gly Gln Glu Phe Asn Ile
           35                  40                  45
   Asn Ser Pro Lys Gln Leu Gly Val Ile Leu Phe Glu Lys Leu Gln Leu
       50                  55                  60
   Pro Val Leu Lys Lys Thr Lys Thr Gly Tyr Ser Thr Ser Ala Asp Val
   65                  70                  75                  80
<210> 82
   <211> 80
   <212> PRT
   <213> Artificial Sequence
<220>
   <223> Synthetic construct
<400> 82
   Leu Ser Leu Ile Leu Ala Glu Met Glu Phe Thr Gly Val Asp Val Asp
   1               5                   10                  15
   Thr Lys Arg Leu Glu Gln Met Gly Leu Glu Leu Ala Glu Gln Leu Val
               20                  25                  30
   Glu Ile Glu Gln Arg Ile Tyr Glu Leu Ala Gly Gln Glu Phe Asn Ile
           35                  40                  45
   Asn Ser Pro Lys Gln Leu Gly Leu Ile Leu Phe Glu Lys Leu Gln Leu
       50                  55                  60
   Pro Val Leu Lys Lys Thr Lys Thr Gly Tyr Ser Thr Ser Ala Asp Val
   65                  70                  75                  80
<210> 83
   <211> 80
   <212> PRT
   <213> Artificial Sequence
<220>
   <223> Synthetic construct
<400> 83
   Leu Ala Leu Ile Leu Ala Glu Met Glu Phe Thr Gly Val Lys Val Asp
   1               5                   10                  15
   Thr Lys Arg Leu Glu Gln Met Gly Glu Glu Leu Ala Glu Gln Leu Lys
               20                  25                  30
   Ala Ile Glu Gln Arg Ile Tyr Glu Leu Ala Gly Gln Glu Phe Asn Ile
           35                  40                  45
   Asn Ser Pro Lys Gln Leu Gly Val Ile Leu Phe Glu Lys Leu Gln Leu
       50                  55                  60
   Pro Val Leu Lys Lys Thr Lys Thr Gly Tyr Ser Thr Ser Ala Asp Val
   65                  70                  75                  80
<210> 84
   <211> 80
   <212> PRT
   <213> Artificial Sequence
<220>
   <223> Synthetic construct
<400> 84
   Leu Ala Leu Ile Leu Ala Glu Met Glu Phe Thr Gly Val Lys Val Asp
   1 5 10 15
   Thr Lys Arg Leu Glu Gln Met Gly Glu Glu Leu Ala Glu Gln Leu Lys
   20 25 30
   Glu Ile Glu Gln Arg Ile Tyr Glu Leu Ala Gly Gln Glu Phe Asn Ile
   35 40 45
   Asn Ser Pro Lys Gln Leu Gly Val Ile Leu Phe Glu Lys Leu Gln Leu
   50 55 60
   Pro Val Leu Lys Lys Thr Lys Thr Gly Tyr Ser Thr Ser Ala Asp Val
   65 70 75 80
<210> 85
   <211> 80
   <212> PRT
   <213> Artificial Sequence
<220>
   <223> Synthetic construct
<400> 85
   Leu Ala Leu Ile Leu Ala Glu Met Glu Phe Thr Gly Val Lys Val Asp
   1 5 10 15
   Thr Lys Arg Leu Glu Gln Met Gly Glu Glu Leu Ala Glu Gln Leu Lys
   20 25 30
   Ala Val Glu Gln Arg Ile Tyr Glu Leu Ala Gly Gln Glu Phe Asn Ile
   35 40 45
   Asn Ser Pro Lys Gln Leu Gly Val Ile Leu Phe Glu Lys Leu Gln Leu
   50 55 60
   Pro Val Leu Lys Lys Thr Lys Thr Gly Tyr Ser Thr Ser Ala Asp Val
   65 70 75 80
<210> 86
   <211> 80
   <212> PRT
   <213> Artificial Sequence
<220>
   <223> Synthetic construct
<400> 86
   Leu Ala Leu Ile Leu Ala Glu Met Glu Phe Thr Gly Val Lys Val Asp
   1               5                   10                  15
   Thr Lys Arg Leu Glu Gln Met Gly Glu Glu Leu Ala Glu Gln Leu Lys
               20                  25                  30
   Glu Ile Glu Gln Arg Ile Tyr Glu Leu Ala Gly Gln Glu Phe Asn Ile
           35                  40                  45
   Asn Ser Pro Lys Gln Leu Gly Val Ile Leu Phe Glu Lys Leu Gln Leu
       50                  55                  60
   Pro Val Leu Lys Lys Thr Lys Thr Gly Tyr Ser Thr Ser Ala Asp Val
   65                  70                  75                  80
<210> 87
   <211> 80
   <212> PRT
   <213> Artificial Sequence
<220>
   <223> Synthetic construct
<400> 87
   Leu Ser Ser Ile Leu Ala Glu Met Glu Phe Thr Gly Val Lys Val Asp
   1               5                   10                  15
   Thr Lys Arg Leu Glu Gln Met Gly Glu Glu Leu Ala Glu Gln Leu Arg
               20                  25                  30
   Ala Val Glu Gln Arg Ile Tyr Glu Leu Ala Gly Gln Glu Phe Asn Ile
           35                  40                  45
   Asn Ser Pro Lys Gln Leu Gly Val Ile Leu Phe Glu Lys Leu Gln Leu
       50                  55                  60
   Pro Val Leu Lys Lys Thr Lys Thr Gly Tyr Ser Thr Ser Ala Asp Val
   65                  70                  75                  80
<210> 88
   <211> 80
   <212> PRT
   <213> Artificial Sequence
<220>
   <223> Synthetic construct
<400> 88
   Leu Glu Lys Leu Ala Pro His His Glu Ile Val Glu Asn Ile Leu His
   1 5 10 15
   Tyr Arg Gln Leu Gly Lys Leu Gln Ser Thr Tyr Ile Glu Gly Leu Leu
   20 25 30
   Lys Val Val Arg Pro Asp Thr Gly Lys Val His Thr Met Phe Asn Gln
   35 40 45
   Ala Leu Thr Gln Thr Gly Arg Leu Ser Ser Ala Glu Pro Asn Leu Gln
   50 55 60
   Asn Ile Pro Ile Arg Leu Glu Glu Gly Arg Lys Ile Arg Gln Ala Phe
   65 70 75 80
<210> 89
   <211> 80
   <212> PRT
   <213> Artificial Sequence
<220>
   <223> Synthetic construct
<400> 89
   Leu Glu Lys Leu Ala Pro His His Glu Ile Val Glu Asn Ile Leu His
   1 5 10 15
   Tyr Arg Gln Leu Gly Lys Leu Gln Ser Thr Tyr Ile Glu Gly Leu Leu
   20 25 30
   Lys Val Val His Pro Asp Thr Gly Lys Val His Thr Arg Phe Asn Gln
   35 40 45
   Ala Leu Thr Gln Thr Gly Arg Leu Ser Ser Thr Asp Pro Asn Leu Gln
   50 55 60
   Asn Ile Pro Ile Arg Leu Glu Glu Gly Arg Lys Ile Arg Gln Ala Phe
   65 70 75 80
<210> 90
   <211> 80
   <212> PRT
   <213> Artificial Sequence
<220>
   <223> Synthetic construct
<400> 90
   Leu Glu Lys Leu Ala Pro Glu His Glu Ile Val Glu Asn Ile Leu His
   1               5                   10                  15
   Tyr Arg Gln Leu Gly Lys Leu Gln Ser Thr Tyr Ile Glu Gly Leu Leu
               20                  25                  30
   Lys Val Val Arg Thr Asp Thr Gly Lys Val His Thr Ile Phe Asn Gln
           35                  40                  45
   Ala Leu Thr Gln Thr Gly Arg Leu Ser Ser Ala Glu Pro Asn Leu Gln
       50                  55                  60
   Asn Ile Pro Ile Arg Leu Glu Glu Gly Arg Lys Ile Arg Gln Ala Phe
   65                  70                  75                  80
<210> 91
   <211> 80
   <212> PRT
   <213> Artificial Sequence
<220>
   <223> Synthetic construct
<400> 91
   Leu Glu Lys Leu Ala Pro Glu His Glu Ile Val Glu Asn Ile Leu His
   1               5                   10                  15
   Tyr Arg Gln Leu Gly Lys Leu Gln Ser Thr Tyr Ile Glu Gly Leu Leu
               20                  25                  30
   Lys Val Val Arg Thr Asp Thr Gly Lys Val His Thr Met Phe Asn Gln
           35                  40                  45
   Ala Leu Thr Gln Thr Gly Arg Leu Ser Ser Ala Asp Pro Asn Leu Gln
       50                  55                  60
   Asn Ile Pro Ile Arg Leu Glu Glu Gly Arg Lys Ile Arg Gln Ala Phe
   65                  70                  75                  80
<210> 92
   <211> 80
   <212> PRT
   <213> Artificial Sequence
<220>
   <223> Synthetic construct
<400> 92
   Leu Glu Lys Leu Ala Pro His His Glu Ile Val Glu Asn Ile Leu His
   1 5 10 15
   Tyr Arg Gln Leu Gly Lys Leu Gln Ser Thr Tyr Ile Glu Gly Leu Leu
   20 25 30
   Lys Val Val His Pro Asp Thr Gly Lys Val His Thr Met Phe Asn Gln
   35 40 45
   Ala Leu Thr Gln Thr Gly Arg Leu Ser Ser Thr Glu Pro Asn Leu Gln
   50 55 60
   Asn Ile Pro Ile Arg Leu Glu Glu Gly Arg Lys Ile Arg Gln Ala Phe
   65 70 75 80
<210> 93
   <211> 80
   <212> PRT
   <213> Artificial Sequence
<220>
   <223> Synthetic construct
<400> 93
   Leu Glu Lys Leu Ala Pro His His Glu Ile Val Glu Asn Ile Leu His
   1 5 10 15
   Tyr Arg Gln Leu Gly Lys Leu Gln Ser Thr Tyr Ile Glu Gly Leu Leu
   20 25 30
   Lys Val Val Arg Pro Asp Thr Gly Lys Val His Thr Met Phe Asn Gln
   35 40 45
   Ala Leu Thr Gln Thr Gly Arg Leu Ser Ser Thr Glu Pro Asn Leu Gln
   50 55 60
   Asn Ile Pro Ile Arg Leu Glu Glu Gly Arg Lys Ile Arg Gln Ala Phe
   65 70 75 80
<210> 94
   <211> 80
   <212> PRT
   <213> Artificial Sequence
<220>
   <223> Synthetic construct
<400> 94
   Leu Glu Lys Leu Ala Pro Glu His Glu Ile Val Glu Asn Ile Leu His
   1 5 10 15
   Tyr Arg Gln Leu Gly Lys Leu Gln Ser Thr Tyr Ile Glu Gly Leu Leu
   20 25 30
   Lys Val Val Asp Thr Asp Thr Gly Lys Val His Thr Met Phe Asn Gln
   35 40 45
   Ala Leu Thr Gln Thr Gly Arg Leu Ser Ser Ala Glu Pro Asn Leu Gln
   50 55 60
   Asn Ile Pro Ile Arg Leu Glu Glu Gly Arg Lys Ile Arg Gln Ala Phe
   65 70 75 80
<210> 95
   <211> 80
   <212> PRT
   <213> Artificial Sequence
<220>
   <223> Synthetic construct
<400> 95
   Leu Glu Lys Leu Ala Pro His His Glu Ile Val Glu Asn Ile Leu His
   1 5 10 15
   Tyr Arg Gln Leu Gly Lys Leu Gln Ser Thr Tyr Ile Glu Gly Leu Leu
   20 25 30
   Lys Val Val His Pro Asp Thr Gly Lys Val His Thr Ile Phe Asn Gln
   35 40 45
   Ala Leu Thr Gln Thr Gly Arg Leu Ser Ser Thr Glu Pro Asn Leu Gln
   50 55 60
   Asn Ile Pro Ile Arg Leu Glu Glu Gly Arg Lys Ile Arg Gln Ala Phe
   65 70 75 80
<210> 96
   <211> 80
   <212> PRT
   <213> Artificial Sequence
<220>
   <223> Synthetic construct
<400> 96
   Leu Glu Lys Leu Ala Pro His His Glu Ile Val Glu Asn Ile Leu His
   1               5                   10                  15
   Tyr Arg Gln Leu Gly Lys Leu Gln Ser Thr Tyr Ile Glu Gly Leu Leu
               20                  25                  30
   Lys Val Val His Pro Asp Thr Gly Lys Val His Thr Met Phe Asn Gln
           35                  40                  45
   Ala Leu Thr Gln Thr Gly Arg Leu Ser Ser Ala Glu Pro Asn Leu Gln
       50                  55                  60
   Asn Ile Pro Ile Arg Leu Glu Glu Gly Arg Lys Ile Arg Gln Ala Phe
   65                  70                  75                  80
<210> 97
   <211> 80
   <212> PRT
   <213> Artificial Sequence
<220>
   <223> Synthetic construct
<400> 97
   Leu Glu Lys Leu Ala Pro His His Glu Ile Val Glu Asn Ile Leu His
   1               5                   10                  15
   Tyr Arg Gln Leu Gly Lys Leu Gln Ser Thr Tyr Ile Glu Gly Leu Leu
               20                  25                  30
   Lys Val Val Asp Thr Asp Thr Gly Lys Val His Thr Met Phe Asn Gln
           35                  40                  45
   Ala Leu Thr Gln Thr Gly Arg Leu Ser Ser Ala Glu Pro Asn Leu Gln
       50                  55                  60
   Asn Ile Pro Ile Arg Leu Glu Glu Gly Arg Lys Ile Arg Gln Ala Phe
   65                  70                  75                  80
<210> 98
   <211> 80
   <212> PRT
   <213> Artificial Sequence
<220>
   <223> Synthetic construct
<400> 98
   Leu Glu Lys Leu Ala Pro His His Glu Ile Val Glu Asn Ile Leu His
   1               5                   10                  15
   Tyr Arg Gln Leu Gly Lys Leu Gln Ser Thr Tyr Ile Glu Gly Leu Leu
               20                  25                  30
   Lys Val Val His Pro Asp Thr Gly Lys Val His Thr Arg Phe Asn Gln
           35                  40                  45
   Ala Leu Thr Gln Thr Gly Arg Leu Ser Ser Thr Glu Pro Asn Leu Gln
       50                  55                  60
   Asn Ile Pro Ile Arg Leu Glu Glu Gly Arg Lys Ile Arg Gln Ala Phe
   65                  70                  75                  80
<210> 99
   <211> 80
   <212> PRT
   <213> Artificial Sequence
<220>
   <223> Synthetic construct
<400> 99
   Leu Glu Lys Leu Ala Pro Glu His Glu Ile Val Glu Asn Ile Leu His
   1               5                   10                  15
   Tyr Arg Gln Leu Gly Lys Leu Gln Ser Thr Tyr Ile Glu Gly Leu Leu
               20                  25                  30
   Lys Val Val Arg Thr Asp Thr Gly Lys Val His Thr Met Phe Asn Gln
           35                  40                  45
   Ala Leu Thr Gln Thr Gly Arg Leu Ser Ser Ala Glu Pro Asn Leu Gln
       50                  55                  60
   Asn Ile Pro Ile Arg Leu Glu Glu Gly Arg Lys Ile Arg Gln Ala Phe
   65                  70                  75                  80
<210> 100
   <211> 80
   <212> PRT
   <213> Artificial Sequence
<220>
   <223> Synthetic construct
<400> 100
   Val Pro Ser Glu Pro Asp Trp Leu Ile Phe Ala Ala Asp Tyr Ser Gln
   1               5                   10                  15
   Ile Glu Leu Arg Val Leu Ala His Ile Ala Asp Asp Asp Asn Leu Ile
               20                  25                  30
   Glu Ala Phe Gln Arg Asp Leu Asp Ile His Thr Lys Thr Ala Met Asp
           35                  40                  45
   Ile Phe His Val Ser Glu Glu Glu Val Thr Ala Asn Met Arg Arg Gln
       50                  55                  60
   Ala Lys Ala Val Asn Phe Gly Ile Val Tyr Gly Ile Ser Asp Tyr Gly
   65                  70                  75                  80
<210> 101
   <211> 80
   <212> PRT
   <213> Artificial Sequence
<220>
   <223> Synthetic construct
<400> 101
   Val Pro Ser Glu Pro Leu Trp Leu Ile Phe Ala Ala Asp Tyr Ser Gln
   1               5                   10                  15
   Ile Glu Leu Arg Val Leu Ala His Ile Ala Asp Asp Asp Asn Leu Ala
               20                  25                  30
   Glu Ala Phe Gln Arg Asp Leu Asp Ile His Thr Lys Thr Ala Met Asp
           35                  40                  45
   Ile Phe His Val Ser Glu Glu Glu Val Thr Ser Arg Met Arg Arg Gln
       50                  55                  60
   Ala Lys Ala Val Asn Phe Gly Ile Val Tyr Gly Ile Ser Asp Tyr Gly
   65                  70                  75                  80
<210> 102
   <211> 80
   <212> PRT
   <213> Artificial Sequence
<220>
   <223> Synthetic construct
<400> 102
   Val Pro Ser Glu Pro Leu Trp Leu Ile Phe Ala Ala Asp Tyr Ser Gln
   1               5                   10                  15
   Ile Glu Leu Arg Val Leu Ala His Ile Ala Asp Asp Asp Asn Leu Ala
               20                  25                  30
   Glu Ala Phe Arg Arg Asp Leu Asp Ile His Thr Lys Thr Ala Met Asp
           35                  40                  45
   Ile Phe His Val Ser Glu Glu Glu Val Thr Ala Arg Met Arg Arg Gln
       50                  55                  60
   Ala Lys Ala Val Asn Phe Gly Ile Val Tyr Gly Ile Ser Asp Tyr Gly
   65                  70                  75                  80
<210> 103
   <211> 80
   <212> PRT
   <213> Artificial Sequence
<220>
   <223> Synthetic construct
<400> 103
   Val Pro Ser Glu Pro Asp Trp Leu Ile Phe Ala Ala Asp Tyr Ser Gln
   1               5                   10                  15
   Ile Glu Leu Arg Val Leu Ala His Ile Ala Asn Asp Glu Asn Leu Ile
               20                  25                  30
   Glu Ala Phe Arg Arg Asp Leu Asp Ile His Thr Lys Thr Ala Met Asp
           35                  40                  45
   Ile Phe His Val Ser Glu Asp Glu Val Thr Ala Asn Met Arg Arg Gln
       50                  55                  60
   Ala Lys Ala Val Asn Phe Gly Ile Val Tyr Gly Ile Ser Asp Tyr Gly
   65                  70                  75                  80
<210> 104
   <211> 80
   <212> PRT
   <213> Artificial Sequence
<220>
   <223> Synthetic construct
<400> 104
   Val Pro Ser Glu Pro Asp Trp Leu Ile Phe Ala Ala Asp Tyr Ser Gln
   1               5                   10                  15
   Ile Glu Leu Arg Val Leu Ala His Ile Ala Asn Asp Asp Asn Leu Ile
               20                  25                  30
   Glu Ala Phe Arg Arg Asp Met Asp Ile His Thr Lys Thr Ala Met Asp
           35                  40                  45
   Val Phe His Val Ser Glu Asp Glu Val Thr Ser Asn Met Arg Arg Gln
       50                  55                  60
   Ala Lys Ala Val Asn Phe Gly Ile Val Tyr Gly Ile Ser Asp Tyr Gly
   65                  70                  75                  80
<210> 105
   <211> 80
   <212> PRT
   <213> Artificial Sequence
<220>
   <223> Synthetic construct
<400> 105
   Val Pro Ser Glu Pro Leu Trp Leu Ile Phe Ala Ala Asp Tyr Ser Gln
   1               5                   10                  15
   Ile Glu Leu Arg Val Leu Ala His Ile Ala Asn Asp Asp Asn Leu Ala
               20                  25                  30
   Glu Ala Phe Gln Arg Asp Met Asp Ile His Thr Lys Thr Ala Met Asp
           35                  40                  45
   Val Phe His Val Ser Glu Glu Glu Val Thr Ala Arg Met Arg Arg Gln
       50                  55                  60
   Ala Lys Ala Val Asn Phe Gly Ile Val Tyr Gly Ile Ser Asp Tyr Gly
   65                  70                  75                  80
<210> 106
   <211> 80
   <212> PRT
   <213> Artificial Sequence
<220>
   <223> Synthetic construct
<400> 106
   Val Pro Ser Glu Pro Asp Trp Leu Ile Phe Ala Ala Asp Tyr Ser Gln
   1 5 10 15
   Ile Glu Leu Arg Val Leu Ala His Ile Ala Asp Asp Asp Asn Leu Ile
   20 25 30
   Glu Ala Phe Arg Arg Asp Leu Asp Ile His Thr Lys Thr Ala Met Asp
   35 40 45
   Ile Phe His Val Ser Glu Glu Glu Val Thr Ala Asn Met Arg Arg Gln
   50 55 60
   Ala Lys Ala Val Asn Phe Gly Ile Val Tyr Gly Ile Ser Asp Tyr Gly
   65 70 75 80
<210> 107
   <211> 80
   <212> PRT
   <213> Artificial Sequence
<220>
   <223> Synthetic construct
<400> 107
   Val Pro Ser Glu Pro Asp Trp Leu Ile Phe Ala Ala Asp Tyr Ser Gln
   1 5 10 15
   Ile Glu Leu Arg Val Leu Ala His Ile Ala Asp Asp Asp Asn Leu Ile
   20 25 30
   Glu Ala Phe Arg Arg Asp Leu Asp Ile His Thr Lys Thr Ala Met Asp
   35 40 45
   Ile Phe His Val Ser Glu Asp Glu Val Thr Ala Asn Met Arg Arg Gln
   50 55 60
   Ala Lys Ala Val Asn Phe Gly Ile Val Tyr Gly Ile Ser Asp Tyr Gly
   65 70 75 80
<210> 108
   <211> 80
   <212> PRT
   <213> Artificial Sequence
<220>
   <223> Synthetic construct
<400> 108
   Val Pro Ser Glu Pro Asp Trp Leu Ile Phe Ala Ala Asp Tyr Ser Gln
   1 5 10 15
   Ile Glu Leu Arg Val Leu Ala His Ile Ala Asn Asp Asp Asn Leu Ile
               20                  25                  30
   Glu Ala Phe Arg Arg Asp Leu Asp Ile His Thr Lys Thr Ala Met Asp
           35                  40                  45
   Ile Phe His Val Ser Glu Asp Glu Val Thr Ala Asn Met Arg Arg Gln
       50                  55                  60
   Ala Lys Ala Val Asn Phe Gly Ile Val Tyr Gly Ile Ser Asp Tyr Gly
   65                  70                  75                  80
<210> 109
   <211> 80
   <212> PRT
   <213> Artificial Sequence
<220>
   <223> Synthetic construct
<400> 109
   Val Pro Ser Glu Pro Leu Trp Leu Ile Phe Ala Ala Asp Tyr Ser Gln
   1               5                   10                  15
   Ile Glu Leu Arg Val Leu Ala His Ile Ala Asn Asp Glu Asn Leu Ala
               20                  25                  30
   Glu Ala Phe Gln Arg Asp Leu Asp Ile His Thr Lys Thr Ala Met Asp
           35                  40                  45
   Val Phe His Val Ser Glu Glu Glu Val Thr Ser Arg Met Arg Arg Gln
       50                  55                  60
   Ala Lys Ala Val Asn Phe Gly Ile Val Tyr Gly Ile Ser Asp Tyr Gly
   65                  70                  75                  80
<210> 110
   <211> 80
   <212> PRT
   <213> Artificial Sequence
<220>
   <223> Synthetic construct
<400> 110
   Val Pro Ser Glu Pro Asp Trp Leu Ile Phe Ala Ala Asp Tyr Ser Gln
   1               5                   10                  15
   Ile Glu Leu Arg Val Leu Ala His Ile Ala Asp Asp Glu Asn Leu Ile
               20                  25                  30
   Glu Ala Phe Arg Arg Asp Leu Asp Ile His Thr Lys Thr Ala Met Asp
           35                  40                  45
   Ile Phe His Val Ser Glu Glu Glu Val Thr Ala Asn Met Arg Arg Gln
       50                  55                  60
   Ala Lys Ala Val Asn Phe Gly Ile Val Tyr Gly Ile Ser Asp Tyr Gly
   65                  70                  75                  80
<210> 111
   <211> 80
   <212> PRT
   <213> Artificial Sequence
<220>
   <223> Synthetic construct
<400> 111
   Val Pro Ser Glu Pro Asp Trp Leu Ile Phe Ala Ala Asp Tyr Ser Gln
   1               5                   10                  15
   Ile Glu Leu Arg Val Leu Ala His Ile Ala Asp Asp Asp Asn Leu Ile
               20                  25                  30
   Glu Ala Phe Arg Arg Asp Leu Asp Ile His Thr Lys Thr Ala Met Asp
           35                  40                  45
   Ile Phe His Val Ser Glu Glu Glu Val Thr Ala Asn Met Arg Arg Gln
       50                  55                  60
   Ala Lys Ala Val Asn Phe Gly Ile Val Tyr Gly Ile Ser Asp Tyr Gly
   65                  70                  75                  80
<210> 112
   <211> 80
   <212> PRT
   <213> Artificial Sequence
<220>
   <223> Synthetic construct
<400> 112
   Leu Ala Gln Asn Leu Asn Ile Thr Arg Lys Glu Ala Ala Glu Phe Ile
   1               5                   10                  15
   Glu Arg Tyr Phe Ala Ser Phe Pro Gly Val Lys Gln Tyr Met Glu Asn
               20                  25                  30
   Ile Val Gln Glu Ala Lys Gln Lys Gly Tyr Val Thr Thr Leu Leu His
           35                  40                  45
   Arg Arg Arg Tyr Leu Pro Asp Ile Thr Ser Arg Asn Phe Asn Val Arg
       50                  55                  60
   Ser Phe Ala Glu Arg Thr Ala Met Asn Thr Pro Ile Gln Gly Ser Ala
   65                  70                  75                  80
<210> 113
   <211> 80
   <212> PRT
   <213> Artificial Sequence
<220>
   <223> Synthetic construct
<400> 113
   Leu Ser Gln Asn Leu Asn Ile Thr Arg Lys Glu Ala Ala Glu Phe Ile
   1               5                   10                  15
   Glu Arg Tyr Phe Glu Ser Phe Pro Gly Val Lys Arg Tyr Met Glu Asn
               20                  25                  30
   Ile Val Gln Glu Ala Lys Gln Lys Gly Tyr Val Thr Thr Leu Leu His
           35                  40                  45
   Arg Arg Arg Tyr Leu Pro Asp Ile Thr Ser Arg Asn Phe Asn Val Arg
       50                  55                  60
   Ser Phe Ala Glu Arg Thr Ala Met Asn Thr Pro Ile Gln Gly Ser Ala
   65                  70                  75                  80
<210> 114
   <211> 80
   <212> PRT
   <213> Artificial Sequence
<220>
   <223> Synthetic construct
<400> 114
   Leu Ala Gln Asn Leu Asn Ile Thr Arg Lys Glu Ala Ala Glu Phe Ile
   1               5                   10                  15
   Glu Arg Tyr Phe Ala Ser Phe Pro Gly Val Lys Arg Tyr Met Glu Asp
               20                  25                  30
   Ile Val Gln Glu Ala Lys Gln Lys Gly Tyr Val Thr Thr Leu Leu His
           35                  40                  45
   Arg Arg Arg Tyr Leu Pro Asp Ile Thr Ser Arg Asn Phe Asn Val Arg
       50                  55                  60
   Ser Phe Ala Glu Arg Thr Ala Met Asn Thr Pro Ile Gln Gly Ser Ala
   65                  70                  75                  80
<210> 115
   <211> 80
   <212> PRT
   <213> Artificial Sequence
<220>
   <223> Synthetic construct
<400> 115
   Leu Ala Gln Asn Leu Asn Ile Thr Arg Lys Glu Ala Ala Glu Phe Ile
   1               5                   10                  15
   Glu Arg Tyr Phe Glu Ser Phe Pro Gly Val Lys Gln Tyr Met Glu Asn
               20                  25                  30
   Ile Val Gln Glu Ala Lys Gln Lys Gly Tyr Val Thr Thr Leu Leu His
           35                  40                  45
   Arg Arg Arg Tyr Leu Pro Asp Ile Thr Ser Arg Asn Phe Asn Val Arg
       50                  55                  60
   Ser Phe Ala Glu Arg Thr Ala Met Asn Thr Pro Ile Gln Gly Ser Ala
   65                  70                  75                  80
<210> 116
   <211> 80
   <212> PRT
   <213> Artificial Sequence
<220>
   <223> Synthetic construct
<400> 116
   Leu Ala Gln Asn Leu Asn Ile Lys Arg Lys Glu Ala Ala Glu Phe Ile
   1 5 10 15
   Glu Arg Tyr Phe Ala Ser Phe Pro Gly Val Lys Arg Tyr Met Glu Val
   20 25 30
   Ile Val Gln Glu Ala Lys Gln Lys Gly Tyr Val Thr Thr Leu Leu His
   35 40 45
   Arg Arg Arg Tyr Asp Pro Asp Ile Thr Ser Arg Asn Phe Asn Val Arg
   50 55 60
   Ser Phe Ala Glu Arg Met Ala Met Asn Thr Pro Ile Gln Gly Ser Ala
   65 70 75 80
<210> 117
   <211> 80
   <212> PRT
   <213> Artificial Sequence
<220>
   <223> Synthetic construct
<400> 117
   Leu Ser Gln Asn Leu Asn Ile Thr Arg Lys Glu Ala Ala Glu Phe Ile
   1 5 10 15
   Glu Arg Tyr Phe Glu Ser Phe Pro Gly Val Lys Gln Tyr Met Glu Asp
   20 25 30
   Ile Val Gln Glu Ala Lys Gln Lys Gly Tyr Val Thr Thr Leu Leu His
   35 40 45
   Arg Arg Arg Tyr Leu Pro Asp Ile Thr Ser Arg Asn Phe Asn Val Arg
   50 55 60
   Ser Phe Ala Glu Arg Thr Ala Met Asn Thr Pro Ile Gln Gly Ser Ala
   65 70 75 80
<210> 118
   <211> 80
   <212> PRT
   <213> Artificial Sequence
<220>
   <223> Synthetic construct
<400> 118
   Leu Ala Gln Asn Leu Gly Ile Thr Arg Lys Glu Ala Ala Glu Phe Ile
   1 5 10 15
   Glu Arg Tyr Phe Ala Ser Phe Pro Gly Val Lys Gln Tyr Met Glu Asp
   20 25 30
   Ile Val Gln Glu Ala Lys Gln Lys Gly Tyr Val Thr Thr Leu Leu His
   35 40 45
   Arg Arg Arg Tyr Leu Pro Glu Ile Thr Ser Arg Asn Phe Asn Leu Arg
   50 55 60
   Ser Phe Ala Glu Arg Thr Ala Met Asn Thr Pro Ile Gln Gly Ser Ala
   65 70 75 80
<210> 119
   <211> 80
   <212> PRT
   <213> Artificial Sequence
<220>
   <223> Synthetic construct
<400> 119
   Leu Ala Gln Asn Leu Gly Ile Thr Arg Lys Glu Ala Ala Glu Phe Ile
   1 5 10 15
   Glu Arg Tyr Phe Ala Ser Phe Pro Gly Val Lys Glu Tyr Met Glu Glu
   20 25 30
   Ile Val Gln Glu Ala Lys Gln Lys Gly Tyr Val Thr Thr Leu Leu His
   35 40 45
   Arg Arg Arg Tyr Leu Pro Asp Ile Thr Ser Arg Asn Phe Asn Val Arg
   50 55 60
   Ser Phe Ala Glu Arg Met Ala Met Asn Thr Pro Ile Gln Gly Ser Ala
   65 70 75 80
<210> 120
   <211> 80
   <212> PRT
   <213> Artificial Sequence
<220>
   <223> Synthetic construct
<400> 120
   Leu Ala Gln Asn Leu Asn Ile Thr Arg Lys Glu Ala Ala Glu Phe Ile
   1 5 10 15
   Glu Arg Tyr Phe Ala Ser Phe Pro Gly Val Lys Arg Tyr Met Glu Asn
   20 25 30
   Ile Val Gln Glu Ala Lys Gln Lys Gly Tyr Val Thr Thr Leu Leu His
   35 40 45
   Arg Arg Arg Tyr Leu Pro Asp Ile Thr Ser Arg Asn Phe Asn Val Arg
   50 55 60
   Ser Phe Ala Glu Arg Thr Ala Met Asn Thr Pro Ile Gln Gly Ser Ala
   65 70 75 80
<210> 121
   <211> 80
   <212> PRT
   <213> Artificial Sequence
<220>
   <223> Synthetic construct
<400> 121
   Leu Ala Gln Asn Leu Asn Ile Thr Arg Lys Glu Ala Ala Glu Phe Ile
   1 5 10 15
   Glu Arg Tyr Phe Glu Ser Phe Pro Gly Val Lys Arg Tyr Met Glu Asn
   20 25 30
   Ile Val Gln Glu Ala Lys Gln Lys Gly Tyr Val Thr Thr Leu Leu His
   35 40 45
   Arg Arg Arg Tyr Leu Pro Asp Ile Thr Ser Arg Asn Phe Asn Val Arg
   50 55 60
   Ser Phe Ala Glu Arg Thr Ala Met Asn Thr Pro Ile Gln Gly Ser Ala
   65 70 75 80
<210> 122
   <211> 80
   <212> PRT
   <213> Artificial Sequence
<220>
   <223> Synthetic construct
<400> 122
   Leu Ser Gln Asn Leu Asn Ile Thr Arg Lys Glu Ala Ala Glu Phe Ile
   1 5 10 15
   Glu Arg Tyr Phe Ala Ser Phe Pro Gly Val Lys Gln Tyr Met Glu Asn
   20 25 30
   Ile Val Gln Glu Ala Lys Gln Lys Gly Tyr Val Thr Thr Leu Leu His
   35 40 45
   Arg Arg Arg Tyr Leu Pro Asp Ile Thr Ser Arg Asn Phe Asn Val Arg
   50 55 60
   Ser Phe Ala Glu Arg Thr Ala Met Asn Thr Pro Ile Gln Gly Ser Ala
   65 70 75 80
<210> 123
   <211> 80
   <212> PRT
   <213> Artificial Sequence
<220>
   <223> Synthetic construct
<400> 123
   Leu Ala Gln Asn Leu Asn Ile Lys Arg Lys Glu Ala Ala Glu Phe Ile
   1 5 10 15
   Glu Arg Tyr Phe Ala Ser Phe Pro Gly Val Lys Gln Tyr Met Glu Val
   20 25 30
   Ile Val Gln Glu Ala Lys Gln Lys Gly Tyr Val Thr Thr Leu Leu His
   35 40 45
   Arg Arg Arg Tyr Asp Pro Asp Ile Thr Ser Arg Asn Phe Asn Val Arg
   50 55 60
   Ser Phe Ala Glu Arg Met Ala Met Asn Thr Pro Ile Gln Gly Ser Ala
   65 70 75 80
<210> 124
   <211> 80
   <212> PRT
   <213> Artificial Sequence
<220>
   <223> Synthetic construct
<400> 124
   Leu Ser Gln Asn Leu Gly Ile Thr Arg Lys Glu Ala Ala Glu Phe Ile
   1               5                   10                  15
   Glu Arg Tyr Phe Glu Ser Phe Pro Gly Val Lys Glu Tyr Met Glu Asp
               20                  25                  30
   Ile Val Gln Glu Ala Lys Gln Lys Gly Tyr Val Thr Thr Leu Leu His
           35                  40                  45
   Arg Arg Arg Tyr Leu Pro Glu Ile Thr Ser Arg Asn Phe Asn Leu Arg
       50                  55                  60
   Ser Phe Ala Glu Arg Thr Ala Met Asn Thr Pro Ile Gln Gly Ser Ala
   65                  70                  75                  80
<210> 125
   <211> 80
   <212> PRT
   <213> Artificial Sequence
<220>
   <223> Synthetic construct
<400> 125
   Leu Ser Gln Asn Leu Asn Ile Thr Arg Lys Glu Ala Ala Glu Phe Ile
   1               5                   10                  15
   Glu Arg Tyr Phe Ala Ser Phe Pro Gly Val Lys Arg Tyr Met Glu Glu
               20                  25                  30
   Ile Val Gln Glu Ala Lys Gln Lys Gly Tyr Val Thr Thr Leu Leu His
           35                  40                  45
   Arg Arg Arg Tyr Leu Pro Asp Ile Thr Ser Arg Asn Phe Asn Val Arg
       50                  55                  60
   Ser Phe Ala Glu Arg Thr Ala Met Asn Thr Pro Ile Gln Gly Ser Ala
   65                  70                  75                  80
<210> 126
   <211> 76
   <212> PRT
   <213> Artificial Sequence
<220>
   <223> Synthetic construct
<400> 126
   Ala Asp Ile Ile Lys Lys Ala Met Ile Asp Leu Ala Ala Arg Leu Lys
   1               5                   10                  15
   Glu Glu Gln Leu Gln Ala Arg Leu Leu Leu Gln Val His Asp Glu Leu
               20                  25                  30
   Ile Leu Glu Ala Pro Lys Glu Glu Ile Glu Arg Leu Cys Glu Leu Val
           35                  40                  45
   Pro Glu Val Met Glu Gln Ala Val Thr Leu Arg Val Pro Leu Lys Val
       50                  55                  60
   Asp Tyr His Tyr Gly Pro Thr Trp Tyr Asp Ala Lys
   65                  70                  75
<210> 127
   <211> 76
   <212> PRT
   <213> Artificial Sequence
<220>
   <223> Synthetic construct
<400> 127
   Ala Asp Ile Ile Lys Lys Ala Met Ile Asp Leu Ala Ala Arg Leu Lys
   1               5                   10                  15
   Glu Glu Arg Leu Gln Ala Arg Leu Leu Leu Gln Val His Asp Glu Leu
               20                  25                  30
   Ile Leu Glu Ala Pro Lys Glu Glu Ile Glu Arg Leu Cys Arg Leu Val
           35                  40                  45
   Pro Glu Val Met Glu Gln Ala Val Thr Leu Arg Val Pro Leu Lys Val
       50                  55                  60
   Asp Tyr His Tyr Gly Pro Thr Trp Tyr Asp Ala Lys
   65                  70                  75
<210> 128
   <211> 76
   <212> PRT
   <213> Artificial Sequence
<220>
   <223> Synthetic construct
<400> 128
   Ala Asp Ile Ile Lys Lys Ala Met Ile Asp Leu Ala Ala Arg Leu Lys
   1               5                   10                  15
   Glu Glu Arg Leu Gln Ala Arg Leu Leu Leu Gln Val His Asp Glu Leu
               20                  25                  30
   Ile Leu Glu Ala Pro Lys Glu Glu Ile Glu Arg Leu Cys Lys Leu Val
           35                  40                  45
   Pro Glu Val Met Glu Asn Ala Val Thr Leu Arg Val Pro Leu Lys Val
       50                  55                  60
   Asp Tyr His Tyr Gly Pro Thr Trp Tyr Asp Ala Lys
   65                  70                  75
<210> 129
   <211> 76
   <212> PRT
   <213> Artificial Sequence
<220>
   <223> Synthetic construct
<400> 129
   Ala Asp Ile Ile Lys Lys Ala Met Ile Asp Leu Ala Ala Arg Leu Lys
   1               5                   10                  15
   Glu Glu Gln Leu Gln Ala Arg Leu Leu Leu Gln Val His Asp Glu Leu
               20                  25                  30
   Ile Leu Glu Ala Pro Lys Glu Glu Met Glu Arg Leu Cys Val Leu Val
           35                  40                  45
   Pro Glu Val Met Glu Gln Ala Val Arg Leu Arg Val Pro Leu Lys Val
       50                  55                  60
   Asp Tyr His Tyr Gly Trp Thr Trp Tyr Asp Ala Lys
   65                  70                  75
<210> 130
   <211> 76
   <212> PRT
   <213> Artificial Sequence
<220>
   <223> Synthetic construct
<400> 130
   Ala Asp Ile Ile Lys Lys Ala Met Ile Asp Leu Ala Ala Arg Leu Lys
   1               5                   10                  15
   Glu Glu Arg Leu Gln Ala Arg Leu Leu Leu Gln Val His Asp Glu Leu
               20                  25                  30
   Ile Leu Glu Ala Pro Lys Glu Glu Met Glu Arg Leu Cys Arg Leu Val
           35                  40                  45
   Pro Glu Val Met Glu Gln Ala Val Thr Leu Arg Val Pro Leu Lys Val
       50                  55                  60
   Asp Tyr His Tyr Gly Pro Thr Trp Tyr Asp Ala Lys
   65                  70                  75
<210> 131
   <211> 76
   <212> PRT
   <213> Artificial Sequence
<220>
   <223> Synthetic construct
<400> 131
   Ala Asp Ile Ile Lys Lys Ala Met Ile Asp Met Ala Ala Arg Leu Lys
   1               5                   10                  15
   Glu Glu Gln Leu Gln Ala Arg Leu Leu Leu Gln Val His Asp Glu Leu
               20                  25                  30
   Ile Leu Glu Ala Pro Lys Glu Glu Met Glu Arg Leu Glu Glu Leu Val
           35                  40                  45
   Pro Glu Val Met Glu His Ala Val Thr Leu Arg Val Pro Leu Lys Val
       50                  55                  60
   Asp Tyr His Tyr Gly Trp Thr Trp Tyr Asp Ala Lys
   65                  70                  75
<210> 132
   <211> 76
   <212> PRT
   <213> Artificial Sequence
<220>
   <223> Synthetic construct
<400> 132
   Ala Asp Ile Ile Lys Lys Ala Met Ile Asp Met Ala Ala Arg Leu Lys
   1               5                   10                  15
   Glu Glu Gln Leu Gln Ala Arg Leu Leu Leu Gln Val His Asp Glu Leu
               20                  25                  30
   Ile Phe Glu Ala Pro Lys Glu Glu Ile Glu Arg Leu Glu Glu Leu Val
           35                  40                  45
   Pro Glu Val Met Glu Asn Ala Val Thr Leu Arg Val Pro Leu Lys Val
       50                  55                  60
   Asp Tyr His Tyr Gly Trp Thr Trp Tyr Asp Ala Lys
   65                  70                  75
<210> 133
   <211> 76
   <212> PRT
   <213> Artificial Sequence
<220>
   <223> Synthetic construct
<400> 133
   Ala Asp Ile Ile Lys Lys Ala Met Ile Asp Leu Ala Ala Arg Leu Lys
   1               5                   10                  15
   Glu Glu Arg Leu Gln Ala Arg Leu Leu Leu Gln Val His Asp Glu Leu
               20                  25                  30
   Ile Leu Glu Ala Pro Lys Glu Glu Ile Glu Arg Leu Cys Gln Leu Val
           35                  40                  45
   Pro Glu Val Met Glu Gln Ala Val Glu Leu Arg Val Pro Leu Lys Val
       50                  55                  60
   Asp Tyr His Tyr Gly Pro Thr Trp Tyr Asp Ala Lys
   65                  70                  75
<210> 134
   <211> 76
   <212> PRT
   <213> Artificial Sequence
<220>
   <223> Synthetic construct
<400> 134
   Ala Asp Ile Ile Lys Lys Ala Met Ile Asp Met Ala Ala Arg Leu Lys
   1               5                   10                  15
   Glu Glu Arg Leu Gln Ala Arg Leu Leu Leu Gln Val His Asp Glu Leu
               20                  25                  30
   Ile Phe Glu Ala Pro Lys Glu Glu Ile Glu Arg Leu Glu Lys Leu Val
           35                  40                  45
   Pro Glu Val Met Glu His Ala Val Glu Leu Arg Val Pro Leu Lys Val
       50                  55                  60
   Asp Tyr His Tyr Gly Pro Thr Trp Tyr Asp Ala Lys
   65                  70                  75
<210> 135
   <211> 76
   <212> PRT
   <213> Artificial Sequence
<220>
   <223> Synthetic construct
<400> 135
   Ala Asp Ile Ile Lys Lys Ala Met Ile Asp Leu Ala Ala Arg Leu Lys
   1               5                   10                  15
   Glu Glu Gln Leu Gln Ala Arg Leu Leu Leu Gln Val His Asp Glu Leu
               20                  25                  30
   Ile Phe Glu Ala Pro Lys Glu Glu Met Glu Arg Leu Glu Glu Leu Val
           35                  40                  45
   Pro Glu Val Met Glu His Ala Val Thr Leu Arg Val Pro Leu Lys Val
       50                  55                  60
   Asp Tyr His Tyr Gly Trp Thr Trp Tyr Asp Ala Lys
   65                  70                  75
<210> 136
   <211> 76
   <212> PRT
   <213> Artificial Sequence
<220>
   <223> Synthetic construct
<400> 136
   Ala Asp Ile Ile Lys Lys Ala Met Ile Asp Met Ala Ala Arg Leu Lys
   1               5                   10                  15
   Glu Glu Gln Leu Gln Ala Arg Leu Leu Leu Gln Val His Asp Glu Leu
               20                  25                  30
   Ile Phe Glu Ala Pro Lys Glu Glu Ile Glu Arg Leu Cys Glu Leu Val
           35                  40                  45
   Pro Glu Val Met Glu Asn Ala Val Thr Leu Arg Val Pro Leu Lys Val
       50                  55                  60
   Asp Tyr His Tyr Gly Trp Thr Trp Tyr Asp Ala Lys
   65                  70                  75
<210> 137
   <211> 76
   <212> PRT
   <213> Artificial Sequence
<220>
   <223> Synthetic construct
<400> 137
   Ala Asp Ile Ile Lys Lys Ala Met Ile Asp Leu Ala Ala Arg Leu Lys
   1               5                   10                  15
   Glu Glu Arg Leu Gln Ala Arg Leu Leu Leu Gln Val His Asp Glu Leu
               20                  25                  30
   Ile Leu Glu Ala Pro Lys Glu Glu Ile Glu Arg Leu Cys Gln Leu Val
           35                  40                  45
   Pro Glu Val Met Glu Gln Ala Val Thr Leu Arg Val Pro Leu Lys Val
       50                  55                  60
   Asp Tyr His Tyr Gly Pro Thr Trp Tyr Asp Ala Lys
   65                  70                  75
<210> 138
   <211> 76
   <212> PRT
   <213> Artificial Sequence
<220>
   <223> Synthetic construct
<400> 138
   Ala Asp Ile Ile Lys Lys Ala Met Ile Asp Leu Ala Ala Arg Leu Lys
   1 5 10 15
   Glu Glu Arg Leu Gln Ala Arg Leu Leu Leu Gln Val His Asp Glu Leu
   20 25 30
   Ile Leu Glu Ala Pro Lys Glu Glu Ile Glu Arg Leu Cys Lys Leu Val
   35 40 45
   Pro Glu Val Met Glu Gln Ala Val Glu Leu Arg Val Pro Leu Lys Val
   50 55 60
   Asp Tyr His Tyr Gly Pro Thr Trp Tyr Asp Ala Lys
   65 70 75
<210> 139
   <211> 46
   <212> DNA
   <213> Artificial Sequence
<220>
   <223> Synthetic construct
<400> 139
   cagccagccg cagcacgttc gctcatagga gatatggtag agccgc 46
<210> 140
   <211> 51
   <212> DNA
   <213> Artificial Sequence
<220>
   <223> Synthetic construct
<400> 140
   gagagaattt gtaccacctc ccaccgggca catagcagtc ctagggacag t 51
<210> 141
   <211> 22
   <212> DNA
   <213> Artificial Sequence
<220>
   <223> Synthetic construct
<400> 141
   ggcttggctc tgctaacacg tt 22
<210> 142
   <211> 22
   <212> DNA
   <213> Artificial Sequence
<220>
   <223> Synthetic construct
<400> 142
   ggacgtttgt aatgtccgct cc 22
<210> 143
   <211> 17
   <212> DNA
   <213> Artificial Sequence
<220>
   <223> Synthetic construct
<400> 143
   ctgcatacga cgtgtct 17
<210> 144
   <211> 20
   <212> DNA
   <213> Artificial Sequence
<220>
   <223> Synthetic construct
<400> 144
   accatctatg actgtacgcc 20
<210> 145
   <211> 24
   <212> DNA
   <213> Artificial Sequence
<220>
   <223> Synthetic construct
<400> 145
   cgccagggtt ttcccagtca cgac 24
<210> 146
   <211> 20
   <212> DNA
   <213> Artificial Sequence
<220>
   <223> Synthetic construct
<400> 146
   agaacgggaa gcttgtcatc 20
<210> 147
   <211> 18
   <212> DNA
   <213> Artificial Sequence
<220>
   <223> Synthetic construct
<400> 147
   cgaacatggg ggcatcag 18

## Description

A DNA polymerase from Geobacillus stearothermophilus has been described in Kong, et al., US Patent No. 5,814,506 (1998). This enzyme, which is a Bst DNA polymerase, belongs to DNA polymerase Family A and shares about 45% sequence identity with its better known relative Taq DNA polymerase. Whereas Taq DNA polymerase is from a hyperthermophilic organism and is able to survive the high temperatures of the polymerase chain reaction, the Bst DNA polymerase reported in Kong, et al., is from a thermophilic organism, is optimally active between 60-70°C, but does not survive the high temperatures of PCR. The full length (FL) Bst DNA polymerase is 876 amino acid residues and has 5'-3' endonuclease activity but not 3'-5' exonuclease activity. The large fragment (LF) of Bst DNA polymerase lacks both 5'-3' exonuclease activity and 3'-5' exonuclease activity and is only 587 amino acid residues with 289 amino acids being deleted from the N-terminal end. The FL Bst DNA polymerase and the LF Bst DNA polymerase have been found to be useful for isothermal amplification techniques and DNA sequencing.

US 2003/180737 describes a method of reverse transcription. WO 02/101004 describes low-temperature cycle extension of DNA with high priming specificity.

### SUMMARY OF EMBODIMENTS

Compositions and methods are described herein that relate to variants of DNA polymerases belonging to Family A DNA polymerases.

The embodiments of the invention are defined in the claims appended hereto.

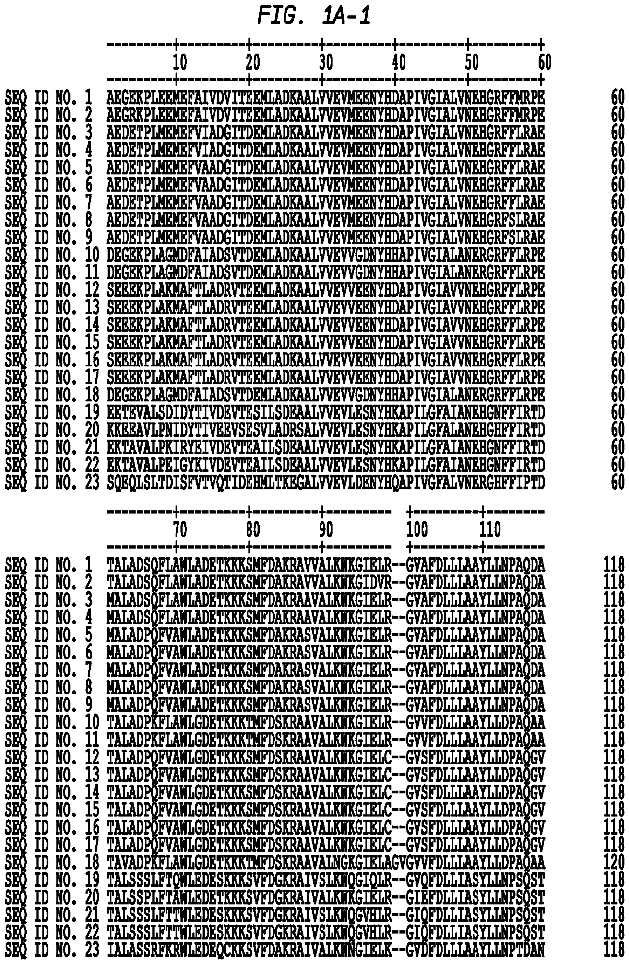

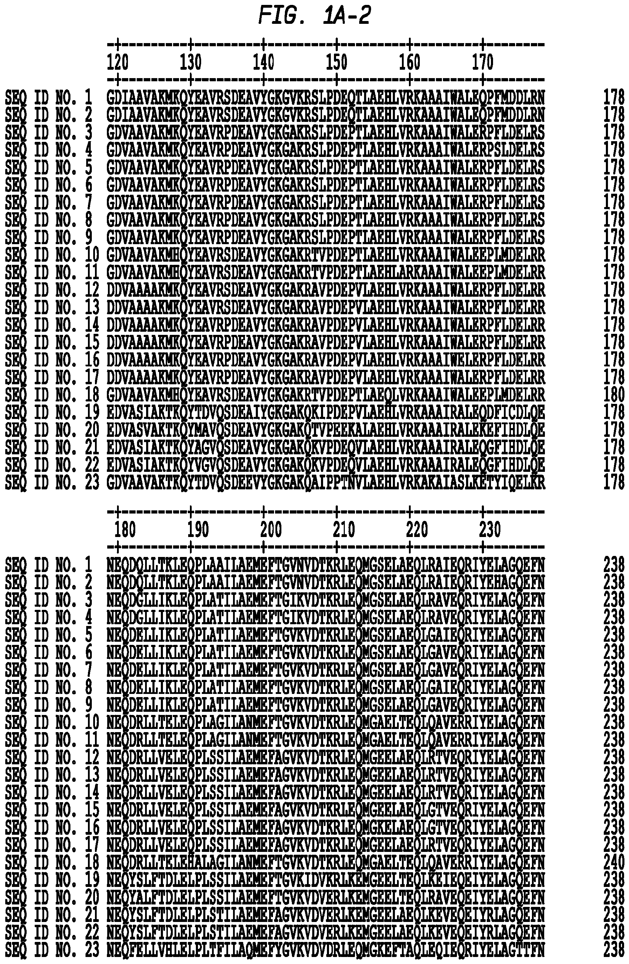

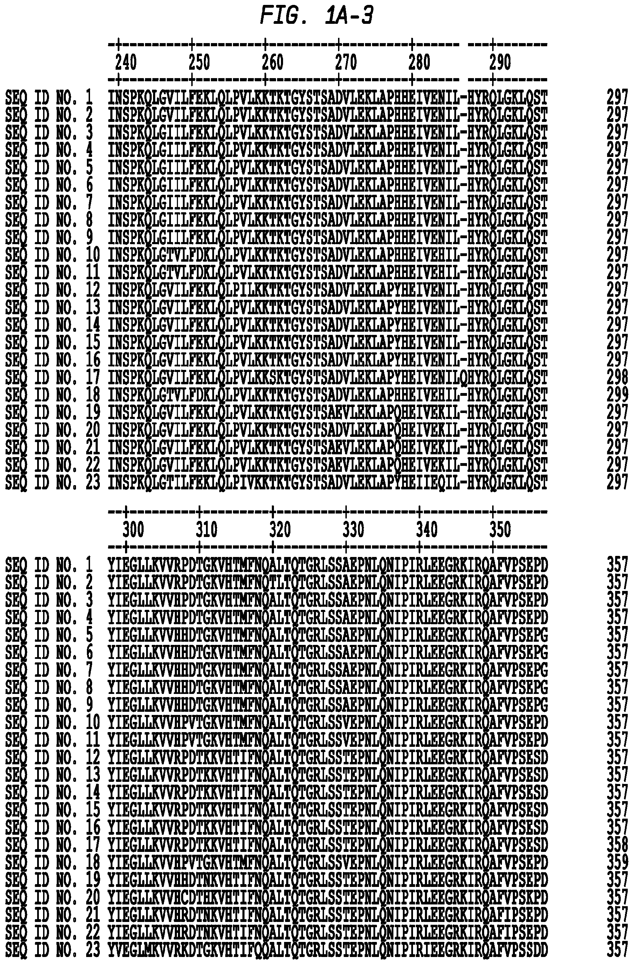

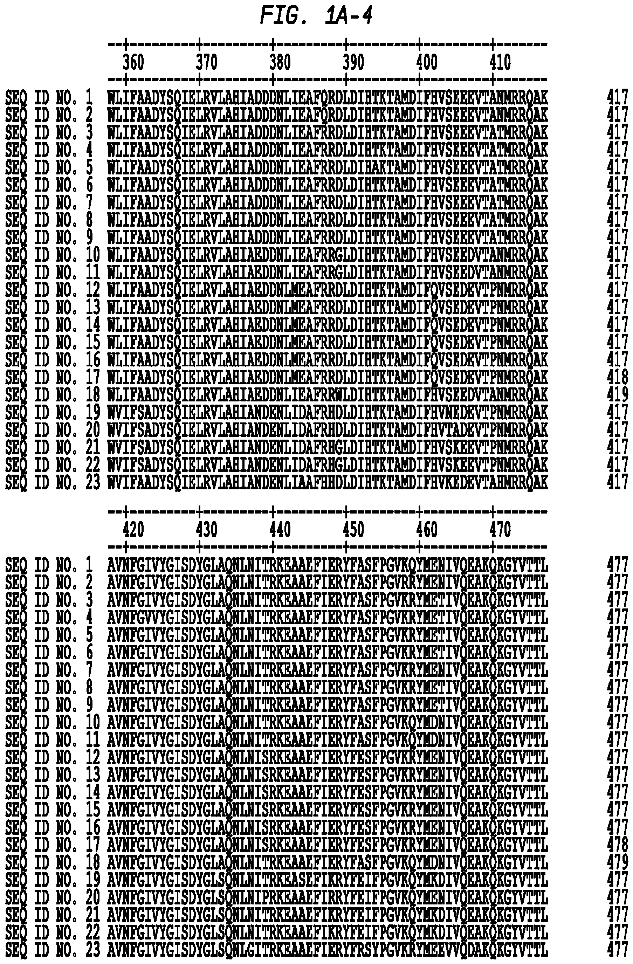

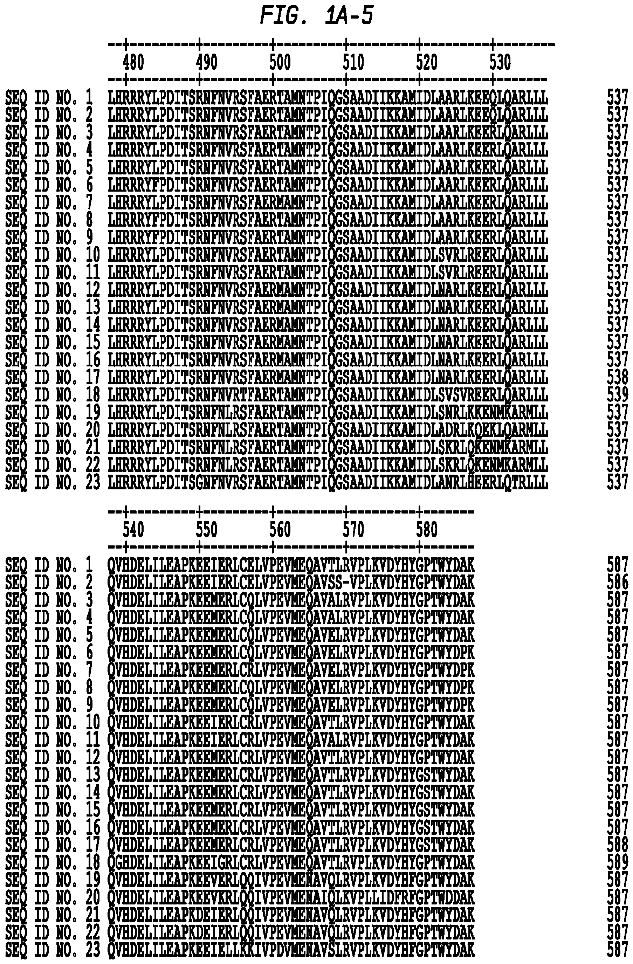

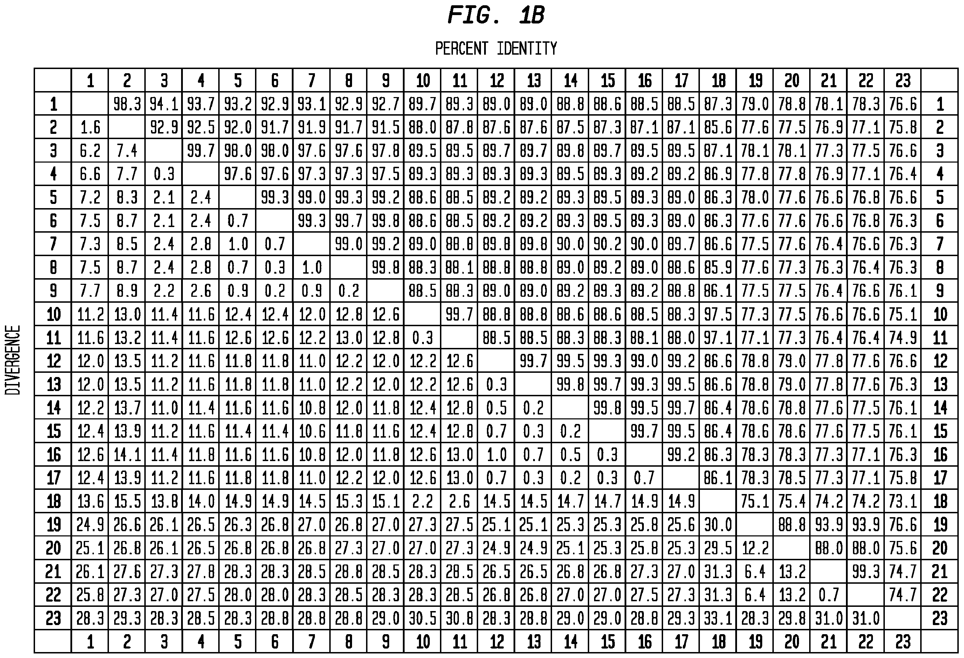

In embodiment 1 of the disclosure, a variant Family A DNA polymerase comprises two or more amino acid sequence motifs selected from 3..EEK..5, 15..ADE..17, 65..SPQ..67, 86..RAI..88, 185..LTE..187, 186..TEL..188, 222..LKE..224, 306..VHP..308, 314...HTR..316, 555..LCK..557, 556..CKL..558 and 567..VEL..569, where the number preceding the amino acid in the motif corresponds to the location of that amino acid in the amino acid sequence of Figure 1, wherein the two or more motifs confer improved reaction speed in an amplification reaction and/or improved stability compared to the reaction speed and/or stability of any of SEQ ID NOs:1-23.

In embodiment 2 of the disclosure, a variant polymerase of embodiment 1 has at least 75% but less than 100% identity to any of SEQ ID NOs:1-23.

In embodiment 3 of the disclosure, a variant polymerase of embodiment 1 or 2 comprises at least three or four or five or six or seven or eight or nine or ten or eleven or twelve of the motifs.

In embodiment 4 of the disclosure, a variant polymerase of any one of the preceding embodiments further comprises one or more mutations selected from the group of mutations consisting of (a)-(f) where the mutations in (a)-(f) are:
(a) A1E, G3(K, E or D), K5(L, A or V), E8(M or A), E9D, M10I, A13(D, E, T or V), I14D, V15A, V17(T, E or G), I18V;
(b) E20(M or D) M34(Q or L), E36D, I46F, L48(N or I), M57(L or I), P59(T or A), T61L, D65S, S66(F, E or P);
(c) Q67A, L69(V or K), A73E, M81V, A84R, V88(A or I), R99V, A102D, N113A, D117(T, S or A), A118D, G119(D or E), I121(A or V), V124K, E131H, S135(E or P), V144A, S147(P or A), L148(D or V), Q152(L or P);
(d) T153(A or V), Q170(R or E), M173(L or I), D175E, N178(E, K or R), Q183(L, E or R), L185F, T186(L or I), K187(E or D), Q190(L or M);
(e) A193(I or S), A194(L, S or T), N205(D or K), S216(L or E), R223(V, K or G), A224E, I225(Q or V), V247L, R307H, M316R; and
(f) A330T, D357L, D378N, D380E, I383A, Q387R, L390M; I400V, E406D, A410S, N411R, A433S, N437G, T439K, A452E, Q459(R or E), N463(V, E or D), L484D, D486E, V494L, T501M, Q530R, I552M, E557(K, Q or R) and T568(E or R).
In embodiment 5 of the disclosure, a variant polymerase according to embodiment 4, comprising at least one mutated amino acid selected from each of groups (a)-(f).

In embodiment 6 of the disclosure, a variant polymerase of embodiment 4 further comprises two, three, four, five, six, seven, eight, nine, ten, eleven, twelve, thirteen, fourteen, fifteen, sixteen, seventeen, eighteen, nineteen, or twenty mutant amino acids at the same position as the corresponding amino acids in (a)-(f) of SEQ ID NO:1.

In embodiment 7 of the disclosure, a variant polymerase of any one of the preceding embodiments is described wherein said sequence motif(s) confer one or more improved properties selected from at least one of specific activity; reaction speed; thermostability; storage stability; dUTP tolerance and salt tolerance; increased performance in isothermal amplification; non-interference of pH during sequencing; improved strand displacement; altered processivity; altered ribonucleotide incorporation; altered modified nucleotide incorporation; and altered fidelity when compared to the corresponding parent polymerase.

In embodiment 8 of the disclosure, a variant polymerase of any one of the preceding embodiments is described wherein a peptide is fused to one end of the variant polymerase directly or by means of a linker sequence.

In embodiment 9 of the disclosure, an enzyme preparation comprises a variant polymerase according to any one of the preceding embodiments and a buffer.

In embodiment 10 of the disclosure, an enzyme preparation according to embodiment 8 or 9 comprises a temperature dependent inhibitor of polymerase activity.

In embodiment 11 of the disclosure, an enzyme preparation according to any of embodiments 8 through 10, further comprises dNTPs.

In embodiment 12 of the disclosure, a DNA encodes a variant polymerase as described in any of preceding embodiments.

In embodiment 13 of the disclosure, a host cell comprises the DNA according to embodiment 12.

In embodiment 14 of the disclosure, a process for preparing a variant of a parent Family A DNA polymerase having improved polymerase activity compared with the parent polymerase, comprises synthesizing a polypeptide as defined in any one of embodiments 1-8; and characterizing the polymerase activity.

In embodiment 15 of the disclosure, the process of embodiment 14 is described wherein characterizing the polymerase activity, further comprises: determining in comparison with the parent polymerase, at last one of: thermostability; stability in storage; tolerance to salt; performance in isothermal amplification; strand displacement; kinetics; processivity; fidelity; altered ribonucleotide incorporation; altered dUTP incorporation; and altered modified nucleotide incorporation.

In embodiment 16 of the disclosure, a variant Family A DNA polymerase is obtainable by the process of embodiment 14 or embodiment 15.

In embodiment 17 of the disclosure, a variant polymerase of any of embodiments 1 through 7 wherein the one or more motifs or one or more mutations selected from the group of mutations consisting of (a)-(f) have improved reverse transcriptase (Rtx) activity.

In embodiment 18 of the disclosure, a method for reverse transcribing an RNA of interest, comprises combining an RNA with a DNA polymerase variant or preparation thereof according to embodiments 1-11 to form a complementary DNA (cDNA).

In embodiment 19 of the disclosure, a method according to embodiment 18 further comprises amplifying the cDNA by means of the DNA polymerase variant or preparation thereof according to claims 1-11, to produce amplified DNA.

In embodiment 20 of the disclosure, a method for amplifying DNA comprises combining a target DNA with a DNA polymerase variant or preparation thereof according to embodiments 1-11, to produce amplified DNA.

In embodiment 21 of the disclosure, a variant protein comprises: an amino acid sequence with at least 75% or 80% or 85% or 90% or 95% but less than 100% sequence identity to any of SEQ ID NOs:1-23, wherein the variant protein further comprises at least one mutated amino acid having a position corresponding to SEQ ID NO:1 selected from the group of mutated amino acids consisting of (a)-(f) where the mutations in (a)-(f) are:
(a) A1E, G3(K, E or D), K5(L, A or V), E8(M or A), E9D, M10I, A13(D, E, T or V), I14D, V15A, V17(T, E or G), I18V;
(b) E20(M or D) M34(Q or L), E36D, I46F, L48(N or I), M57(L or I), P59(T or A), T61L, D65S, S66(F, E or P);
(c) Q67A, L69(V or K), A73E, M81V, A84R, V88(A or I), R99V, A102D, N113A, D117(T, S or A), A118D, G119(D or E), I121(A or V), V124K, E131H, S135(E or P), V144A, S147(P or A), L148(D or V), Q152(L or P);
(d) T153(A or V), Q170(R or E), M173(L or I), D175E, N178(E, K or R), Q183(L, E or R), L185F, T186(L or I), K187(E or D), Q190(L or M);
(e) A193(I or S), A194(L, S or T), N205(D or K), S216(L or E), R223(V, K or G), A224E, I225(Q or V), V247L, R307H, M316R; and
(f) A330T, D357L, D378N, D380E, I383A, Q387R, L390M; I400V, E406D, A410S, N411R, A433S, N437G, T439K, A452E, Q459(R or E), N463(V, E or D), L484D, D486E, V494L, T501M, Q530R, I552M, E557(K, Q or R) and T568(E or R).

In embodiment 22 of the disclosure, the variant may contain at least one amino acid corresponding to a mutated amino acid in SEQ ID NO:1 selected from each of groups (a) through (f).

In embodiment 23 of the disclosure, the variant protein according to embodiment 21, further comprises at least one amino acid motif or at least two amino acid motifs selected from the group consisting of: from 3..EEK..5, 15..ADE..17, 65..SPQ..67, 86..RAI..88, 185..LTE..187, 186..TEL..188, 222..LKE..224, 306..VHP..308, 314...HTR..316, 555..LCK..557, 556..CKL..558 and 567..VEL..569.

In embodiment 24 of the disclosure, a variant protein according to any of embodiments 21-23, further comprises two, three, four, five, six, seven, eight, nine, ten, eleven, twelve, thirteen, fourteen, fifteen, sixteen, seventeen, eighteen, nineteen, or twenty amino acids at the same positions as the corresponding mutant amino acids in (a)-(f) of SEQ ID NO:1.

In embodiment 25 of the disclosure, the variant protein of embodiment 24 is described wherein the amino acid sequence is at least 80% identical to any one of SEQ ID NOs:1-23.

In embodiment 26 of the disclosure, a variant protein according to embodiment 25 is described, wherein the amino acid sequence is at least 90% identical to any one of SEQ ID NOs:1-23.

In embodiment 27 of the disclosure, a variant protein according to embodiment 26, is described wherein the amino acid sequence is at least 95% identical to any one of SEQ ID NOs:1-23.

In embodiment 28 of the disclosure, a non-naturally occurring synthetic protein comprises: a fragment 1, a fragment 2, a fragment 3, a fragment 4, a fragment 5, a fragment 6, a fragment 7 and a fragment 8 wherein the fragments are covalently linked in numerical order, and wherein:
the fragment 1 is selected from Segment 1 having an amino acid sequence selected from the group consisting of SEQ ID NOs:24-39;
the fragment 2 is selected from Segment 2 having an amino acid sequence selected from the group consisting of SEQ ID NOs:40-56;
the fragment 3 is selected from Segment 3 having an amino acid sequence selected from the group consisting of SEQ ID NOs:57-72;
the fragment 4 is selected from Segment 4 having an amino acid sequence selected from the group consisting of SEQ ID NOs:73-87;
the fragment 5 selected from Segment 5 having an amino acid sequence selected from the group consisting of SEQ ID NOs: 88-99;
the fragment 6 selected from Segment 6 having an amino acid sequence selected from the group consisting of SEQ ID NOs:100-111;
the fragment 7 selected from Segment 7 having an amino acid sequence selected from the group consisting of SEQ ID NOs:112-125;
the fragment 8 selected from Segment 8 having an amino acid sequence selected from the group consisting of SEQ ID NOs:126-138; and;
wherein the covalently linked fragments has an amino acid sequence that does not have 100% identity to SEQ ID NOs: 1-23.

In embodiment 29 of the disclosure, a synthetic protein according to embodiment 28 is described, wherein the amino acid sequence of the synthetic protein comprises at least one amino acid sequence motif selected from the group consisting of: 3..EEK..5, 15..ADE..17, 65..SPQ..67, 86..RAI..88, 185..LTE..187, 186..TEL..188, 222..LKE..224, 306..VHP..308, 314...HTR..316, 555..LCK..557, 556..CKL..558 and 567..VEL..569.

In embodiment 30 of the disclosure, the synthetic protein of embodiment 28 is described, wherein the amino acid sequence comprises at least two or three or four or five or six or seven or eight or nine or ten or eleven or twelve of the amino acid sequence motifs selected from the group consisting of: 3..EEK..5, 15..ADE..17, 65..SPQ..67, 86..RAI..88, 185..LTE..187, 186..TEL..188, 222..LKE..224, 306..VHP..308, 314...HTR..316, 555..LCK..557, 556..CKL..558 and 567..VEL..569.

In embodiment 31 of the disclosure, a protein comprises at least 75% or 80% or 85% or 90% or 95% sequence identity with SEQ ID NOs:1 and further comprises one or more mutations (such as, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, or 79 mutations) selected from the group consisting of A1 E, G3(K, E or D), K5(L, A or V), E8(M or A), E9D, M101, A13(D, E, T or V), I14D, V15A, V17(T, E or G), I18V, E20(M or D) M34(Q or L), E36D, I46F, L48(N or I), M57(L or I), P59(T or A), T61L, D65S, S66(F, E or P), Q67A, L69(V or K), A73E, M81V, A84R, V88(A or I), R99V, A102D, N113A, D117(T, S or A), A118D, G119(D or E), I121(A or V), V124K, E131H, S135(E or P), V144A, S147(P or A), L148(D or V), Q152(L or P), T153(A or V), Q170(R or E), M173(L or I), D175E, N178(E, K or R), Q183(L, E or R), L185F, T186(L or I), K187(E or D), Q190(L or M), : A193(I or S), A194(L, S or T), N205(D or K), S216(L or E), R223(V, K or G), A224E, I225(Q or V), V247L, R307H, M316R, A330T, D357L, D378N, D380E, I383A, Q387R, L390M, I400V, E406D, A410S, N411R, A433S, N437G, T439K, A452E, Q459(R or E), N463(V, E or D), L484D, D486E, V494L, T501M, Q530R, I552M, E557(K, Q or R) and T568(E or R); and optionally a sequence motif at a specified position in SEQ ID NO:1 selected from the group consisting of: 3..EEK..5, 15..ADE..17, 65..SPQ..67, 86..RAI..88, 185..LTE..187, 186..TEL..188, 222..LKE..224, 306..VHP..308, 314...HTR..316, 555..LCK..557, 556..CKL..558 and 567..VEL..569.

In embodiment 32 of the disclosure, a variant protein or a synthetic protein according to any of embodiments of 21-31 is described, wherein a peptide is fused to one end of the variant protein. For example, the peptide may be fused to one end of the variant protein either directly or by means of a linker.

In embodiment 33 of the disclosure, an enzyme preparation comprises a variant protein or a synthetic protein according to any of embodiments 21-31 and a buffer.

In embodiment 34 of the disclosure, an enzyme preparation according to embodiment 33 further comprises a plurality of proteins.

In embodiment 35 of the disclosure, an enzyme preparation according to embodiment 33 or 34 further comprises a reversible inhibitor of polymerase activity.

In embodiment 36 of the disclosure, an enzyme preparation according to embodiment 33 or 34 further comprises dNTPs.

In embodiment 37 of the disclosure, DNA encodes a variant protein or synthetic protein described in any of embodiments 21-36.

In embodiment 38 of the disclosure, a host cell comprises the DNA according to embodiment 37.

In embodiment 39 of the disclosure, a method for obtaining a variant of a parent protein has improved polymerase activity compared with the parent protein, comprises synthesizing a protein from any of embodiments 21-36; and characterizing the polymerase activity.

In embodiment 40 of the disclosure, which is a method according to embodiment 39, characterizing the polymerase activity further comprises: determining in comparison with the parent protein, at least one of: thermostability; stability in storage; tolerance to salt; performance in isothermal amplification; strand displacement; kinetics; processivity; fidelity; altered ribonucleotide incorporation; altered dUTP incorporation; and altered modified nucleotide incorporation. Additionally, characterizing the polymerase activity includes detecting an increase in Rtx activity.

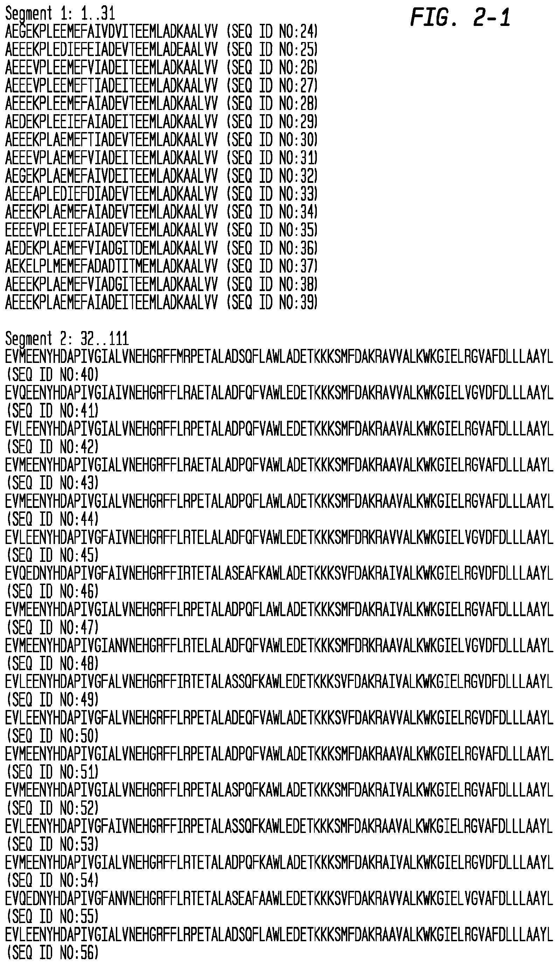

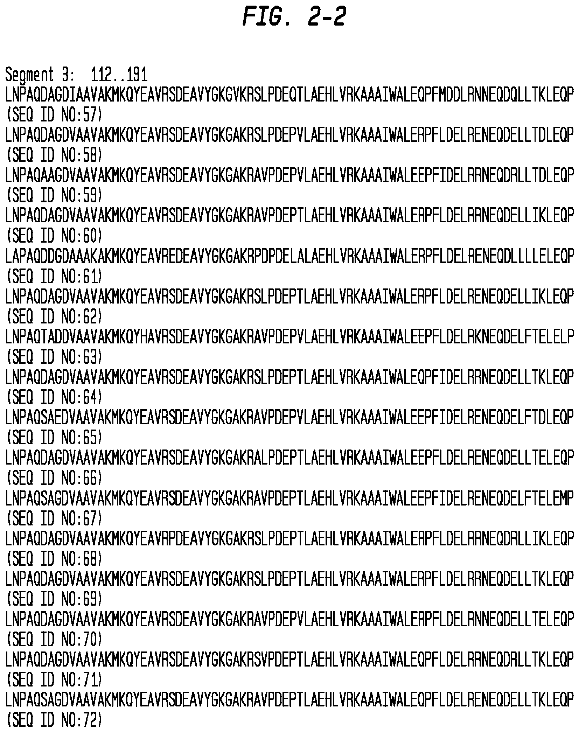

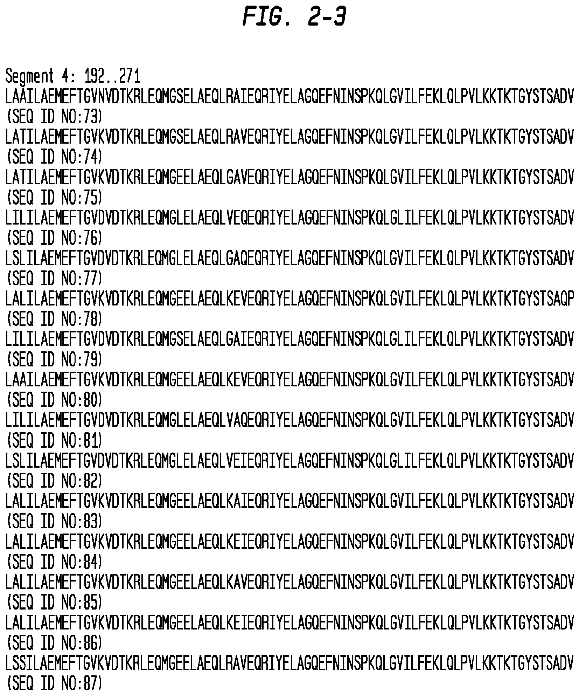

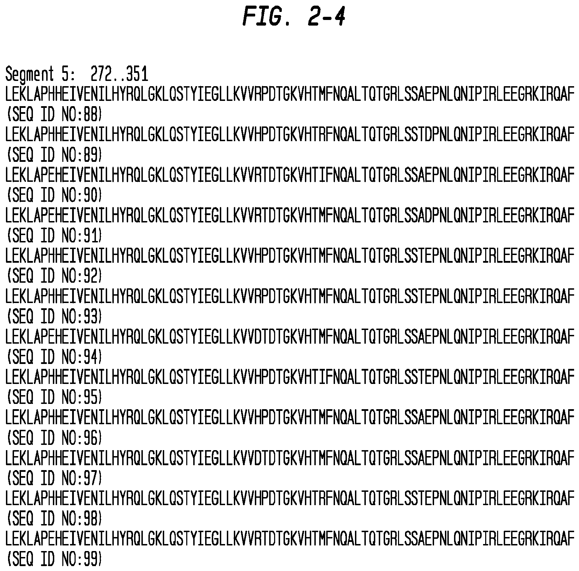

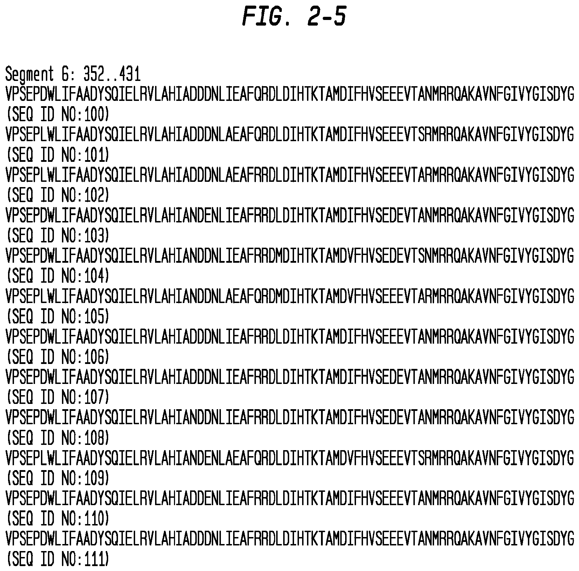

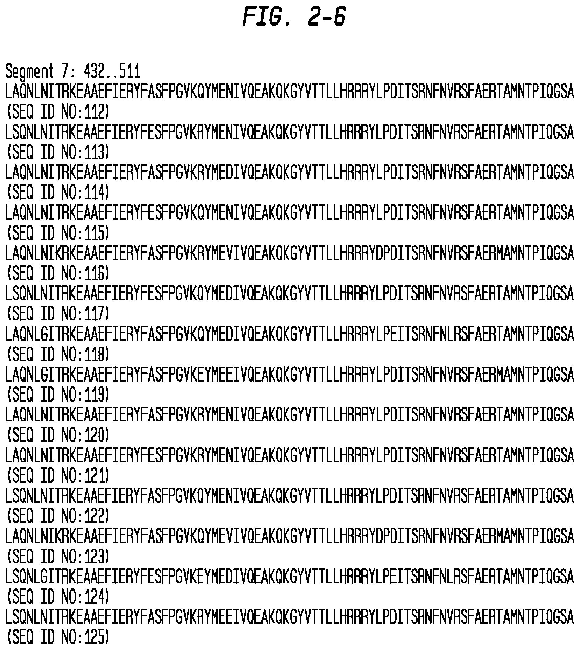

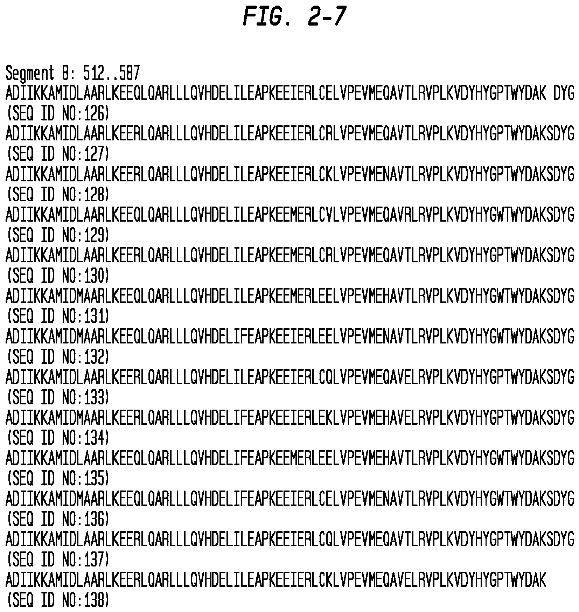

In embodiment 41 of the disclosure, a method comprises: (a) synthesizing a protein wherein the protein has an amino acid sequence which is capable of being generated from single selected protein fragments obtainable from 8 different segments described in Figure 2; and (b) assaying the synthetic protein for polymerase activity.

In embodiment 42 of the disclosure, a method according to embodiment 41 is provided, wherein the protein is synthesized by cloning a DNA sequence encoding the protein.

In embodiment 43 of the disclosure, a method comprises: (a) selecting a protein variant or synthetic protein according to any of claims embodiments 21-36 having an amino acid sequence; and (b) expressing the protein variant or synthetic protein as a fusion protein with an additional peptide at an end of the amino acid sequence.

In embodiment 44 of the disclosure, a method of isothermal amplification comprises: (a) providing a preparation comprising a variant protein or synthetic protein according to any of claims 21-36; (b) combining a target DNA with the preparation; and (c) amplifying the target DNA at a temperature less than 90°C.

In embodiment 45 of the disclosure, a method according to embodiment 44 is described, wherein the amplification reaction results in a quantitative measure of the amount of target DNA in the preparation.

In embodiment 46 of the disclosure, a DNA polymerase having one or more improved properties for isothermal amplification compared with SEQ ID NO:1, where the one or more improved properties are selected from the group consisting of: (a) an increased reaction speed where the increase is at least 10% and as much as 200%; 500% or 1000%; (b) an increased temperature stability in the range of 50°C to 100°C, 50°C to 90°C, or 60°C to 90°C; (c) an increased salt tolerance in the range of 10 mM - 1M, or 20 mM - 200 mM or 500 mM monovalent salt; (d) an increase in storage stability at 25°C, retaining at least 50% activity over 45 weeks, over 1 year or over 2 years; (e) an enhanced dUTP tolerance of the range of an increase of 50% to 100% dUTP; and (f) an increased reverse transcriptase activity by at least 2 fold; wherein the DNA polymerase is a non-naturally occurring mutant of a wild type Bst DNA polymerase.

In embodiment 47 of the disclosure, a DNA polymerase according to embodiment 46 is described having at least two or three or four or five or six of the improved properties.

In embodiment 48 of the disclosure, a DNA polymerase according to embodiments 46 or 47 having at least 80% amino acid sequence identity but less than 100% amino acid sequence identity with any of SEQ ID NOs:1-23 and containing at least 12 artificially introduced single amino acid mutations that occur within a three amino acid motif that differs from a three amino acid motif in the corresponding site of a naturally occurring Bst polymerase.

In embodiment 49 of the disclosure, a DNA polymerase according to embodiment 48 is described wherein at least one of the three amino acid motifs is selected from the group consisting of 3..EEK..5, 15..ADE..17, 65..SPQ..67, 86..RAI..88, 185..LTE..187, 186..TEL..188, 222..LKE..224, 306..VHP..308, 314...HTR..316, 555..LCK..557, 556..CKL..558 and 567..VEL..569.

In general in one aspect, the composition includes a variant protein, having an amino acid sequence with at least 75% or 80% or 85% or 90% or 95% but less than 100% identity to any of SEQ ID NOs:1-23. The variant protein may include at least one amino acid identified by a position in its amino acid sequence and an identity corresponding to any of the mutated amino acids in the corresponding position in SEQ ID NO:1 and listed in (a)-(f) as provided below, wherein the at least one amino acid is selected from the group consisting of:
(a) A1E, G3(K, E or D), K5(L, A or V), E8(M or A), E9D, M10I, A13(D, E, T or V), I14D, V15A, V17(T, E or G), I18V;
(b) E20(M or D) M34(Q or L), E36D, I46F, L48(N or I), M57(L or I), P59(T or A), T61L, D65S, S66(F, E or P);
(c) Q67A, L69(V or K), A73E, M81V, A84R, V88(A or I), R99V, A102D, N113A, D117(T, S or A), A118D, G119(D or E), 1121 (A or V), V124K, E131H, S135(E or P), V144A, S147(P or A), L148(D or V), Q152(L or P);
(d) T153(A or V), Q170(R or E), M173(L or I), D175E, N178(E, K or R), Q183(L, E or R), L185F, T186(L or I), K187(E or D), Q190(L or M);
(e) A193(I or S), A194(L, S or T), N205(D or K), S216(L or E), R223(V, K or G), A224E, I225(Q or V), V247L, R307H, M316R; and
(f) A330T, D357L, D378N, D380E, I383A, Q387R, L390M; I400V, E406D, A410S, N411R, A433S, N437G, T439K, A452E, Q459(R or E), N463(V, E or D), L484D, D486E, V494L, T501M, Q530R, I552M, E557(K, Q or R) and T568(E or R).

In another aspect, the variant may contain at least one amino acid corresponding to a mutated amino acid in SEQ ID NO:1 and selected from each of groups (a) through (f).

In another aspect, the variant protein may include in addition to the amino acids specified above, two, three, four, five, six, seven, eight, nine, ten, eleven, twelve, thirteen, fourteen, fifteen, sixteen, seventeen, eighteen, nineteen, or twenty amino acids at the same positions and identities as the corresponding mutant amino acids in (a)-(f) of SEQ ID NO:1.

In another aspect, the variant protein may include at least one or two or three or four or five or six or seven or eight or nine or ten or eleven or twelve amino acid sequence motifs selected from 3..EEK..5, 15..ADE..17, 65..SPQ..67, 86..RAI..88, 185..LTE..187, 186..TEL..188, 222..LKE..224, 306..VHP..308, 314...HTR..316, 555..LCK..557, 556..CKL..558 and 567..VEL..569, where the number preceding the amino acid in the motif corresponds to the location of that amino acid in the amino acid sequence as determined from Figure 1. The variant protein may include at least one or two or three or four or five or six or seven or eight or nine or ten or eleven or twelve of these motifs in addition to one or more mutations in (a)-(f).

In another aspect, the variant protein has an amino acid sequence that is at least 80%, or at least 85% or at least 90% or at least 95% but less than 100% identical to any one of SEQ ID NOs:1-23.

In another aspect, the variant protein of the sort described above has an amino acid sequence that is at least 80%, or at least 85% or at least 90% or at least 95% but less than 100% identical to any one of SEQ ID NOs:1-23.

In another aspect, a DNA polymerase is provided that comprises or consists of a plurality of peptide fragments selected from segments 1-8 covalently linked to form a single polypeptide that has less than 100% amino acid sequence identity with any of SEQ ID NOs:1-23.

In another aspect, a non-naturally occurring synthetic protein is provided that includes 8 fragments wherein the fragments include a Fragment 1 selected from Segment 1 and having an amino acid sequence selected from the group consisting of SEQ ID NOs:24-39; a Fragment 2 selected from Segment 2 and having an amino acid sequence selected from the group consisting of SEQ ID NOs:40-56, a Fragment 3 selected from Segment 3 and having an amino acid sequence selected from the group consisting of SEQ ID NOs:57-72, a Fragment 4 selected from Segment 4 and having an amino acid sequence selected from the group consisting of SEQ ID NOs:73-87, a Fragment 5 selected from Segment 5 and having an amino acid sequence selected from the group consisting of SEQ ID NOs:88-99; a Fragment 6 selected from Segment 6 and having an amino acid sequence selected from the group consisting of SEQ ID NOs:100-111; a Fragment 7 selected from Segment 7 and having an amino acid sequence selected from the group consisting of SEQ ID NOs:112-125; and a Fragment 8 selected from Segment 8 and having an amino acid sequence selected from the group consisting of SEQ ID NOs:126-138. Fragments 1-8 are covalently linked preferably in numerical order so as to form a single protein wherein the single protein is not any of SEQ ID NOs:1-23.

In another aspect, the amino acid sequence of the synthetic protein comprises at least one or at least two or at least three or at least four or at least five or at least six or at least seven or at least eight or at least nine or at least ten or at least eleven amino acid sequence motifs selected from the group consisting of: 3..EEK..5, 15..ADE..17, 65..SPQ..67, 86..RAI..88, 185..LTE..187, 186..TEL..188, 222..LKE..224, 306..VHP..308, 314...HTR..316, 555..LCK..557, 556..CKL..558 and 567..VEL..569.

In another aspect, a non-naturally occurring protein is provided that comprises or consists of an amino acid sequence having at least 80% sequence identity with SEQ ID NO:1. The non-naturally occurring protein further comprises one or more mutations (such as, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, or 79 mutations) selected from the group consisting of A1 E, G3(K, E or D), K5(L, A or V), E8(M or A), E9D, M10I, A13(D, E, T or V), I14D, V15A, V17(T, E or G), I18V, E20(M or D) M34(Q or L), E36D, I46F, L48(N or I), M57(L or I), P59(T or A), T61L, D65S, S66(F, E or P), Q67A, L69(V or K), A73E, M81V, A84R, V88(A or I), R99V, A102D, N113A, D117(T, S or A), A118D, G119(D or E), I121(A or V), V124K, E131H, S135(E or P), V144A, S147(P or A), L148(D or V), Q152(L or P), T153(A or V), Q170(R or E), M173(L or I), D175E, N178(E, K or R), Q183(L, E or R), L185F, T186(L or I), K187(E or D), Q190(L or M), A193(I or S), A194(L, S or T), N205(D or K), S216(L or E), R223(V, K or G), A224E, I225(Q or V), V247L, R307H, M316R, A330T, D357L, D378N, D380E, I383A, Q387R, L390M, I400V, E406D, A410S, N411R, A433S, N437G, T439K, A452E, Q459(R or E), N463(V, E or D), L484D, D486E, V494L, T501M, Q530R, I552M, E557(K, Q or R) and T568(E or R); and optionally a sequence motif at a specified position in SEQ ID NO:1 selected from the group consisting of: 3..EEK..5, 15..ADE..17, 65..SPQ..67, 86..RAI..88, 185..LTE..187, 186..TEL..188, 222..LKE..224, 306..VHP..308, 314...HTR..316, 555..LCK..557, 556..CKL..558 and 567..VEL..569.

In another aspect, a variant or synthetic protein as described herein may additionally comprise a peptide fused to the N-terminal end or the C-terminal end of the protein directly or via a linker.

In embodiments of the invention, an enzyme preparation is provided which contains a variant protein as defined in the claims and a buffer. The enzyme preparation may contain a plurality of proteins described herein and/or a reversible inhibitor of polymerase activity and/or dNTPs.

In embodiments of the invention, a polynucleotide is provided that encodes a variant protein as defined in the claims. The polynucleotide may be expressed in a transformed host organism.

In general, methods are provided for synthesizing a variant or synthetic protein of the type described above having polymerase activity which in one aspect includes synthesizing a protein of the sort described above; and optionally determining whether the protein has a desired property associated with polymerase activity, the polymerase activity being selected from the group consisting of increased thermostability; stability in storage; improved tolerance to salt; increased performance in isothermal amplification; does not alter the pH of a solution during sequencing; improved strand displacement; improved kinetics; altered processivity; altered ribonucleotide incorporation, altered non-standard deoxyribonucleotide incorporation; altered dUTP incorporation; higher fidelity; and increased Rtx activity as compared with the protein of any of SEQ ID NOs:1-23.

In another aspect, the method includes (a) synthesizing a protein wherein the protein has an amino acid sequence which is capable of being generated from single selected protein fragments obtainable from 8 different segments described in Figure 2; and (b) assaying the synthetic protein for polymerase activity and properties associated therewith. The protein may be synthesized by cloning a DNA sequence encoding the protein.

In another aspect, a method is provided that includes selecting a protein variant or synthetic DNA polymerase protein from those described above; and expressing the protein as a fusion protein with an additional peptide at one or both ends of the DNA polymerase amino acid sequence.

In another aspect, a method is provided for isothermal amplification that includes: (a) providing a preparation comprising of a variant protein or synthetic protein selected from those described above; (b) combining a target DNA with the preparation; and (c) amplifying the target DNA at a 7 temperature less than 90°C to obtain an amplified target and optionally obtaining a quantitative measure of the amount of amplified DNA in the preparation.

In another aspect there is provided a DNA polymerase having one or more improved properties for isothermal amplification compared with SEQ ID NO:1, wherein the improved properties are selected from the group consisting of: (a) an increased reaction speed in the range where the increase is at least 10% and as much as 20%; 500% or 1000%; (b) an increased temperature stability in the range of 50°C to 100°C, 50°C to 90°C or 60°C to 90°C; (c) an increased salt tolerance in the range of 10 mM - 1M, or 20 mM - 200 mM or 500 mM monovalent salt; (d) an increased storage stability at 25°C, retaining at least 50% activity over 45 weeks, over 1 year or over 2 years; (e) an enhanced dUTP tolerance of the range of an increase of 50% to 100% dUTP; and (d) an increased reverse transcriptase activity by at least 2 fold.

In the aforementioned aspect the DNA polymerase: (a) may have at least two or three or four or five or six of the improved properties; (b) may have at least 80% amino acid sequence identity but less than 100% amino acid sequence identity with any of SEQ ID NOs:1-23 and containing at least 12 artificially introduced single amino acid mutations that occur in a three amino acid motif that differs from an amino acid in the corresponding site of a naturally occurring Bst polymerase; or (c) may be such that at least one of the three amino acid motifs is selected from the group consisting of 3..EEK..5, 15..ADE..17, 65..SPQ..67, 86..RAI..88, 185..LTE..187, 186..TEL..188, 222..LKE..224, 306..VHP..308, 314...HTR..316, 555..LCK..557, 556..CKL..558 and 567..VEL..569.

### BRIEF DESCRIPTION OF THE FIGURES

Figure 1A shows an alignment of 23 wild type Bst DNA polymerase (LF) sequences. Not shown is a methionine optionally added at the N-terminal end of each of SEQ ID NOs:1-23 to facilitate expression of the polymerase in a host cell.
Figure 1B shows sequence pair distances of the sequences in Figure 1A using the software program Lasergene® MegAlign™ (DNASTAR, Madison, WI).
Figure 2 shows a 115 fragments arrayed in 8 segments where a fragment selected from each segment joined in order to the neighboring fragment forms an intact synthetic protein having DNA reagent properties.
Figure 3A and 3B show melt peaks for a parent Bst DNA polymerase FL or LF and variant DNA polymerases.

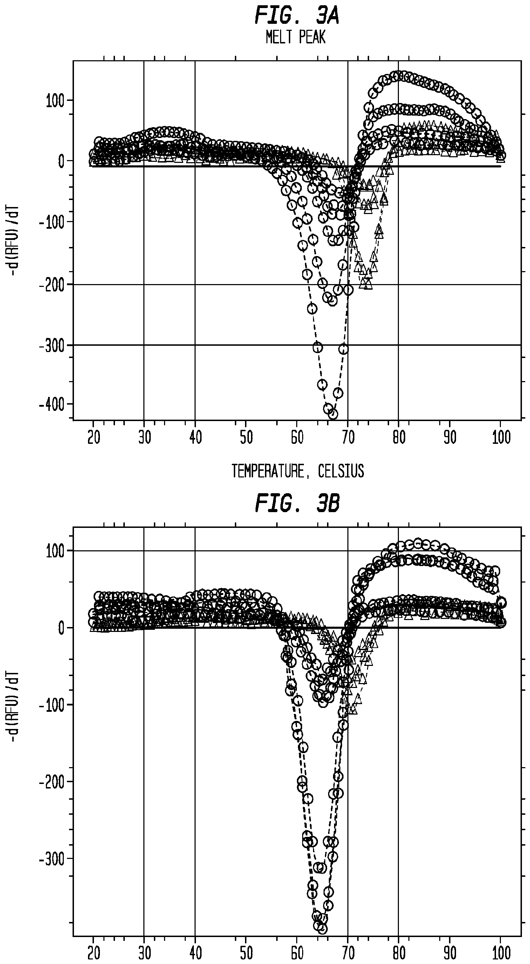

Figure 3A shows the melt peaks for the variant DNA polymerase (FL) which has a melting temperature (Tm) = 73.5°C (Δ) and the parent Bst DNA polymerase (FL) has a Tm = 68°C (O).
Figure 3B shows the melt peaks for a parent Bst DNA polymerase LF (O) which has a Tm = 65°C while the variant DNA polymerase (Δ) has a Tm = 70°C.
   The reactions were performed in 1x Detergent-free ThermoPol™ Buffer (New England Biolabs, Ipswich, MA) and 1x SYPRO® Orange (Life Technologies, Carlsbad, CA).

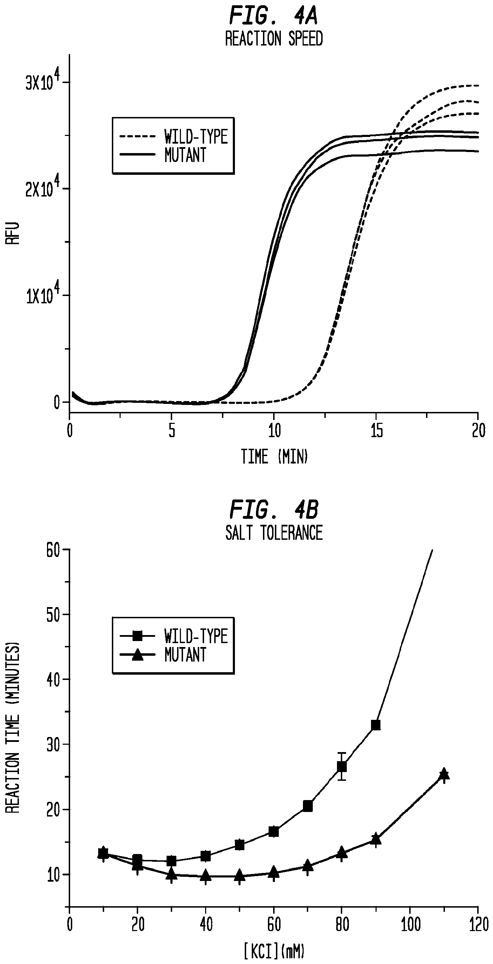

Figures 4A-E show how the properties of a variant DNA polymerase can be screened for significant beneficial properties using an isothermal amplification protocol (Notomi, et al., Nucleic Acids Research, 28:E63 (2000)) and lambda DNA.
Figure 4A shows an analysis of reaction speed. The variant DNA polymerase shows faster DNA amplification than the parent Bst DNA polymerase.
Figure 4B shows the results of an assay to determine salt tolerance. The time in which the amplification reaction took to reach a threshold level of product was graphed against increasing KCI concentration in the reaction. The variant DNA polymerase was more tolerant to changes in salt concentration than the parent Bst DNA polymerase.

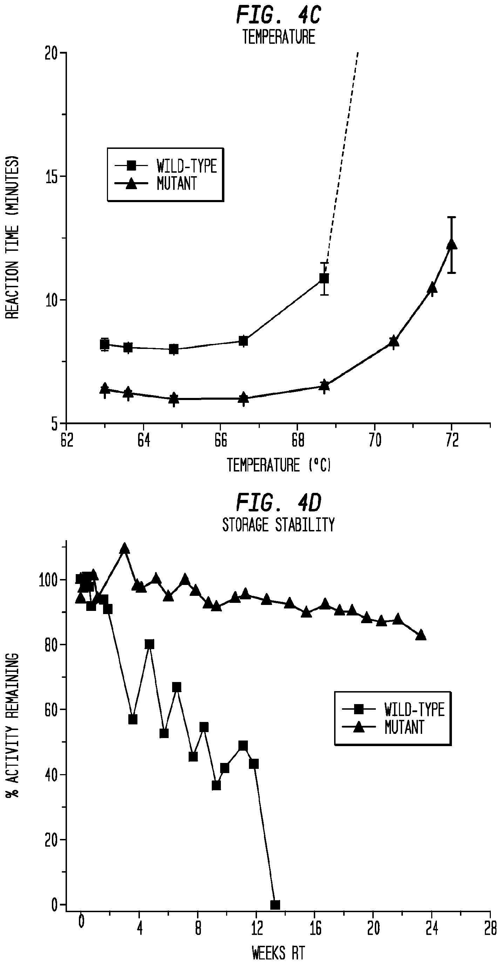

Figure 4C shows the results of an assay to determine an increase in thermostability of a variant DNA polymerase by at least 3°C compared with the parent Bst DNA polymerase. The time in which the amplification reaction took to reach a threshold level of product was graphed against increasing reaction temperature. The variant DNA polymerase was able to amplify DNA at a higher temperature than the parent Bst DNA polymerase.
Figure 4D shows the results of an assay for storage stability in which a variant polymerase remains stable for at least 28 weeks at room temperature (22°C) versus about 13 weeks for the parent Bst DNA polymerase (8000U/ml for each enzyme was used).

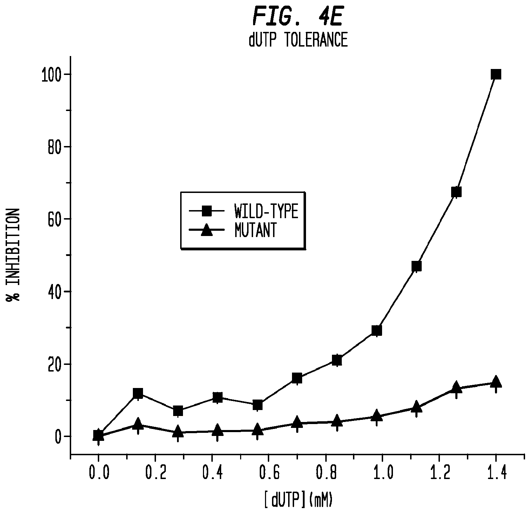

Figure 4E shows the results of an assay for dUTP tolerance in which a parent Bst DNA polymerase is significantly inhibited by increasing amounts of dUTP while the variant DNA polymerase activity is relatively stable as dUTP levels increase (1.4 mM dUTP corresponds to complete substitution of dTTP with dUTP). The ability to incorporate dUTP without inhibition of the polymerase is a useful feature of a DNA polymerase for various applications including strand modification and differentiation. Thermophilic archaeal DNA polymerases do not amplify DNA effectively in the presence of dUTP. Taq DNA polymerase can incorporate dUTP into substrate but Taq DNA polymerase is not suitable for isothermal amplification because it is not capable of the requisite amount of strand displacement.

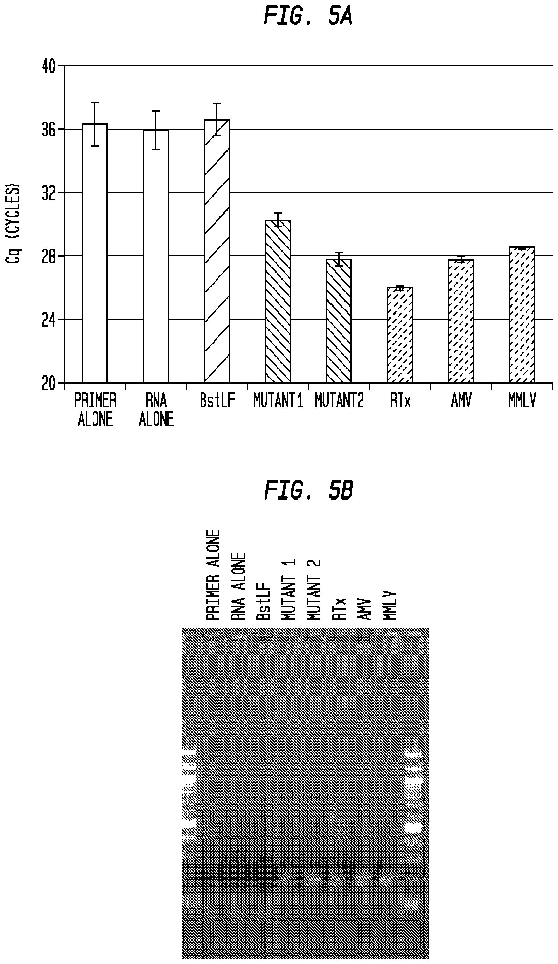

Figure 5A and 5B shows that the DNA polymerase mutants described herein with improved polymerase activity also have improved reverse transcriptase activity.
Figure 5A shows the results of determining Rtx activity using RT-qPCR. The lower the value of cycles (Cq) the greater the activity of the Rtx. From left to right, the bar chart shows Primer alone, RNA alone, Bst polymerase large fragment (BstLF), 2 mutants of the DNA polymerase described herein, Rtx, Avian Myeloblastosis Virus Reverse Transcriptase (AMV) and Moloney Murine Leukemia Virus Reverse Transcriptase (MMLV).
Figure 5B shows gel electrophoresis of amplified DNA resulting from an RNA template and BstLF DNA polymerase or mutants. The lanes are labeled left to right as follows: primer alone, RNA alone, BstLF, Mutant 1 and 2, Rtx, AMV and MMLV.

### DETAILED DESCRIPTION

As used herein, the term "synthetic" with respect to proteins or peptides refers to a non-naturally occurring amino acid sequence that is generated either by expression of a gene encoding the non-naturally occurring amino acid sequence or is generated by chemical synthesis. The gene encoding the non-naturally occurring amino acid sequence may be generated, for example, by mutagenesis of a naturally occurring gene sequence or by total chemical synthesis.

A "variant" protein refers to a protein that differs from a parent protein by at least one amino acid that is the product of a mutation. A variant polymerase is intended to include a "synthetic" protein and vice versa as the context permits. The examples utilize a variant DNA polymerase but it will be understood to a person of ordinary skill in the art that the assays described in the examples are applicable to analyzing synthetic proteins also.

"Non-naturally occurring" refers to a sequence or protein that at the date in which the embodiments of the invention are presented herein, no naturally occurring amino acid sequence corresponding to the alleged non-naturally occurring amino acid has been described in the publically available databases.

"Isothermal amplification" refers to a DNA amplification protocol that is conducted at a temperature below 90°C after an initial denaturation step, where an initial denaturation step is required.

The term "stability" as used in the claims includes thermostability and storage stability as illustrated in Figure 4 and in the examples.

We have developed a set of variant proteins that are mutants of a highly conserved family of DNA polymerases belonging to Family A DNA polymerases. One or more of the amino acid mutations and/or amino acid motifs described herein are capable of enhancing the properties of these polymerases such as those properties determined by the assays described in the examples.

The Family A DNA polymerases are highly conserved so that it will be readily appreciated that with the teaching herein, a person of ordinary skill in the art could select a naturally occurring DNA polymerase sequence (such as from GenBank) having at least 80% sequence identity with SEQ ID NOs:1-23 and introduce one or more of the specified mutations and/or motifs described herein to obtain polymerases with improved properties such as the type described in the examples.

In one embodiment of the disclosure, the DNA polymerase mutant proteins comprise or consist of an amino acid sequence that has at least 75% amino acid sequence identity, at least 80% amino acid sequence identity, or at least 85% amino acid sequence identify and as much as 90% amino acid sequence identity or 95% amino acid sequence identity to the parent DNA polymerase provided in the sequences described in SEQ ID NOs:1-23 wherein the amino acid sequence is less than 100% identical to the amino acid sequence of any of SEQ ID NOs:1-23.

Percentage sequence identity may be calculated by any method known in the art such as for example, using the BLOSUM62 matrix and the methods described in Henikoff, et al., PNAS, 89 (22):10915-10919 (1992)).

The at least one amino acid mutation in the variants is identified using the numbering scheme described in Figure 1 with a reference amino acid as it occurs in SEQ ID NO:1 replaced by a desired amino at the specified position.

Accordingly, a parent polymerases having amino acid sequences with at least 75%, 80%, 85%, 90%, or 95% sequence identity to any of SEQ ID NOs:1-23 may be altered by at least one mutation selected from the group consisting of: A1E, G3(K, E or D), K5(L, A or V), E8(M or A), E9D, M10I, A13(D, E, T or V), I14D, V15A, V17(T, E or G), I18V, E20(M or D) M34(Q or L), E36D, I46F, L48(N or I), M57(L or I), P59(T or A), T61L, D65S, S66(F, E or P), Q67A, L69(V or K), A73E, M81V, A84R, V88(A or I), R99V, A102D, N113A, D117(T, S or A), A118D, G119(D or E), I121(A or V), V124K, E131H, S135(E or P), V144A, S147(P or A), L148(D or V), Q152(L or P), T153(A or V), Q170(R or E), M173(L or I), D175E, N178(E, K or R), Q183(L, E or R), L185F, T186(L or I), K187(E or D), Q190(L or M), A193(I or S), A194(L, S or T), N205(D or K), S216(L or E), R223(V, K or G), A224E, I225(Q or V), V247L, R307H, M316R, A330T, D357L, D378N, D380E, I383A, Q387R, L390M, I400V, E406D, A410S, N411R, A433S, N437G, T439K, A452E, Q459(R or E), N463(V, E or D), L484D, D486E, V494L, T501M, Q530R, I552M, E557(K, Q or R) and T568(E or R).

The variant may optionally include one or more motifs selected from the group consisting of: 3..EEK..5, 15..ADE..17, 65..SPQ..67, 86..RAI..88, 185..LTE..187, 186..TEL..188, 222..LKE..224, 306..VHP..308, 314...HTR..316, 555..LCK..557, 556..CKL..558 and 567..VEL..569.

The DNA polymerase protein variants described above may be screened using at least one method described in Examples 1-6 so as to identify those variants having at least one of the functional properties that are at least typical of a Family A DNA polymerase, such as, Bst DNA Polymerase with an amino acid sequence corresponding to SEQ ID NO:1. The DNA polymerase may additionally have improved properties as compared with the wild type Family A DNA polymerases such as those including one of specific activity, reaction speed, thermostability, storage stability, dUTP tolerance, salt tolerance and reverse transcriptase activity.

In another embodiment of the disclosure, a synthetic protein is described that contains sequences from single fragments selected from each of 8 segments assembled in order of the 8 numbered segments (see Figure 2). The synthetic protein may be synthesized either as a single DNA or protein sequence or as a set of polynucleotides or peptides that are ligated together using techniques known in the art (see for example Gibson Assembly™ Master Mix (New England Biolabs, Ipswich, MA), US Patent No. 7,435,572 or US Patent No. 6,849,428):
a Fragment 1 selected from Segment 1 having an amino acid sequence selected from the group consisting of SEQ ID NOs:24-39;
a Fragment 2 selected from Segment 2 having an amino acid sequence selected from the group consisting of SEQ ID NOs:40-56;
a Fragment 3 selected from Segment 3 having an amino acid sequence selected from the group consisting of SEQ ID NOs:57-72;
a Fragment 4 selected from Segment 4 having an amino acid sequence selected from the group consisting of SEQ ID NOs:73-87;
a Fragment 5 selected from Segment 5 having an amino acid sequence selected from the group consisting of SEQ ID NOs:88-99;
a Fragment 6 selected from Segment 6 having an amino acid sequence selected from the group consisting of SEQ ID NOs:100-111;
a Fragment 7 selected from Segment 7 having an amino acid sequence selected from the group consisting of SEQ ID NOs:112-125;
a Fragment 8 selected from Segment 8 having an amino acid sequence selected from the group consisting of SEQ ID NOs:126-138.

A proviso for creating a synthetic protein is that the synthetic protein has a sequence that differs from any SEQ ID NOs:1-23.

Preferably, a synthetic protein comprising segments 1-8 has at least one, two, three, four, five, six, seven, eight, nine or 10 sequence motifs selected from 3..EEK..5, 15..ADE..17, 65..SPQ..67, 86..RAI..88, 185..LTE..187, 186..TEL..188, 222..LKE..224, 306..VHP..308, 314...HTR..316, 555..LCK..557, 556..CKL..558 and 567..VEL..569.

The synthetic proteins described herein and characterized by a non-natural amino acid sequence generally retain DNA binding properties making these synthetic proteins useful for example as DNA detection reagents. The variants may be screened using at least one method described in Examples 1-6, or by other screening methods common used in the art, so as to identify those variants having at least one of the functional properties that are at least typical of a Family A DNA polymerase and/or have one or more improved properties selected from at least one of specific activity; reaction speed; thermostability; storage stability; dUTP tolerance and salt tolerance; increased performance in isothermal amplification; non-interference of pH during sequencing; improved strand displacement; altered processivity; altered ribonucleotide incorporation; altered modified nucleotide incorporation; and altered fidelity when compared to the corresponding parent polymerase. The improved properties of these mutant enzymes have been demonstrated to enhance the performance of sequencing platforms (for example, the Ion Torrent™ sequencer (Life Technologies, Carlsbad, CA)). The improved properties of these mutant enzymes enhance their use in isothermal amplification for diagnostic applications.

The DNA polymerase variants and synthetic proteins described herein may be expressed in suitable non-native host cells such as *E. coli* according to standard methods known in the art. To facilitate expression, the variant DNA polymerase may additionally have a methionine in front of the first amino acid at the N-terminal end. Host cells may be transformed with DNA encoding the variant optionally contained in a suitable expression vector (see New England Biolabs catalog 2019-10 or 2011-12 for expression vectors known in the art for this purpose). Transformation is achieved using methods well known in the art.

The DNA polymerase variants and synthetic proteins characterized herein may further be modified by additions and/or deletions of peptides at their N-terminal and/or C-terminal ends. For example, fusion of a peptide to a synthetic protein may include fusion of one or more of a DNA binding domain (such as Sso7d from archaea), an exonuclease domain (such as amino acids 1-289 of Bst DNA polymerase), a peptide lacking exonuclease activity (for example, a mutated exonuclease domain similar to amino acids 1-289 of Bst DNA polymerase), an affinity binding domain such as a Histidine tag, chitin binding domain, or intein, and a solubility tag such as maltose binding domain (MBP). The addition of a peptide fused to an end of the amino acid sequence of the DNA polymerase may be used to enhance one or more of the functional features described in Examples 1-6. Aptamers may be fused to one end of the mutant DNA polymerase.

The variants may be stored in a storage or reaction buffer that includes a detergent such as a non-ionic detergent, a zwitterionic detergent, an anionic detergent or a cationic detergent. The storage or reaction buffer may further include one or more of: a polynucleotide, for example, an aptamer for facilitating a hot start; polynucleotide primers, dNTPs, target polynucleotides; additional polymerases including additional DNA polymerases; RNA polymerases and/or reverse transcriptases; crowding agents such as polyethylene glycol; and/or other molecules known in the art for enhancing the activity of the DNA polymerase variants.

The DNA polymerase variant and synthetic proteins may be used for DNA synthesis, DNA repair, cloning and sequencing (see for example US Patent No. 7,700,283 and US Application Publication No. US 2011/0201056) and such as illustrated in the examples and also for temperature dependent amplification methods. Examples of isothermal amplification methods in addition to loop-mediated isothermal amplification (LAMP) used in the present examples include helicase dependent amplification (HDA) (see for example US Patent No. 7,829,284, US Patent No. 7,662,594, and US Patent No. 7,282,328); strand displacement amplification (SDA); nicking enzyme amplification reaction; recombinase polymerase amplification; padlock amplification; rolling circle amplification; and multiple displacement amplification (see for example Gill, et al., Nucleosides, Nucleotides and Nucleic Acids, 27:224-243 (2008)). The variant and synthetic DNA polymerases described herein may also be used in sample preparation for sequencing by synthesis techniques known in the art. The variant and/or synthetic polymerases may also be used in quantitative amplification techniques known in the art that may be performed at a temperature at which the variant or synthetic protein effectively polymerizes nucleotides.

### EXAMPLES

The examples below illustrate assays and properties of Bst DNA polymerase variants described above.

### Example 1: Assay for determining the properties of a variant DNA polymerase

### (a) Loop-mediated isothermal amplification (LAMP)

The properties of a variant polymerase can be determined using an isothermal amplification procedure such as a LAMP protocol (Nagamine, et al., Mol. Cell. Probes, 16:223-229(2002); Notomi, et al., Nucleic Acids Research, 28:E63 (2000)).

The LAMP reaction used bacteriophage λ genomic DNA (New England Biolabs, Ipswich, MA) as the template. The LAMP primers used here were:
FIP (5'-CAGCCAGCCGCAGCACGTTCGCTCATAGGAGATATGGTAGAGCCGC-3') (SEQ ID NO:139),
BIP (5'GAGAGAATTTGTACCACCTCCCACCGGGCACATAGCAGTCCTAGGGAC AGT-3') (SEQ ID NO:140),
F3 (5'-GGCTTGGCTCTGCTAACACGTT-3') (SEQ ID NO:141),
B3 (5'-GGACGTTTGTAATGTCCGCTCC-3') (SEQ ID NO:142),
LoopF (5'-CTGCATACGACGTGTCT-3')(SEQ ID NO:143),
LoopB (5'-ACCATCTATGACTGTACGCC-3') (SEQ ID NO:144).

The LAMP reaction used 0.4 U-0.2 U variant Polymerase/µL, 1.6 µM FIP/BIP, 0.2 µM F3/B3, 0.4 µM LoopF/LoopB, and 5 ng lambda DNA in a buffer containing 1x ThermoPol Detergent-free, 0.1% Tween 20, 6-8mM MgSO₄ and 1.4µMdNTP. The reaction was followed by monitoring turbidity in real time using the Loopamp® Realtime Turbidimeter LA-320c (SA Scientific, San Antonio, TX) or with a CFX96™ Real-Time fluorimeter (Bio-Rad, Hercules, CA). The reaction conditions were varied to determine the optimum range that the variant DNA polymerase could perform LAMP. This was compared with the parent Bst DNA polymerase. The parent Bst DNA polymerase was typically used at 65°C in these LAMP reaction conditions. However, the temperature was varied to determine the optimum temperature for a particular variant. Different salt conditions and rates of reaction were tested and variants identified which were 10%-50% faster than the parent polymerase and had an increased salt tolerance to as much as 200mM KCI. The results are shown in Figure 4.

### (b) DNA polymerase activity assay using modified nucleotides in a comparison of the activity of a fusion variant protein with exonuclease activity, with full length parent Bst polymerase.

This assay was used to determine the activity of the variant polymerase having exonuclease activity as a result of an additional 289 amino acid sequence at the N-terminal end that has been described in detail for parent DNA Bst polymerase. The activity was measured by incorporation of a radioactive ³H-dTTP in a DNA substrate using various concentrations of a variant polymerase. A DNA polymerase reaction cocktail (40 µl) was prepared by mixing 30 nM single-stranded M13mp18, 82 nM primer #1224 (5'-CGCCAGGGTTTTCCCAGTCACGAC-3') (SEQ ID NO:145), 200 µM dATP, 200 µM dCTP, 200 µM dGTP, and 100 or 200 µM dTTP including 0.6 to 0.8 µCi [3H]-dTTP. The DNA polymerase reaction cocktail was mixed with DNA polymerase (2.2 to 8.7 ng for the parent Bst DNA polymerase (FL), 0.27 to 1 ng for the fusion variant, or 2.5 to 20 ng for the parent BstLF), or water for the no enzyme control, and incubated at 65°C for 5 minutes. Reactions were halted and precipitated by acid precipitation as follows. A 30 µl aliquot of each reaction was spotted onto 3 mm Whatman discs and immediately submerged into cold 10% Trichloroacetic acid (TCA) in 1L beaker in an ice bucket. A total counts control was spotted as described but not washed. Filters were washed three times with cold 10% TCA for 10 minutes with vigorous shaking and twice with room temperature 95% isopropanol for 5 minutes. Filters were dried under a heat lamp for 10 minutes and counted using a scintillation counter. The pmoles of dNTPs incorporated were calculated for each sample from the fraction of radioactive counts incorporated, multiplied by the total amount of dNTPs and the volume of the reaction.

A tenfold increase in specific activity of the fusion variant polymerase was found compared with the parent FL Bst polymerase where the fusion variant DNA polymerase was present in the mixture at 506,000 U/mg while the parent Bst DNA polymerase was present at 48,000 U/mg. (1 unit = incorporation of 10 nmol dNTP in 30 minutes at 65°C).

A 15% increase in activity of the variant polymerase compared with the parent Bst large fragment DNA polymerases was observed in which the variant DNA polymerase was present in the mixture at 370,000 U/mg and the parent BstLF was present at 260,000 U/mg.

### Example 2: Variant DNA polymerase thermostability

The thermostability of the variant DNA polymerase was assessed by incubating the polymerase at differing temperatures followed by performing either one or both of the DNA polymerase assay described in Example 1. The results are shown in Figure 4C.

### Example 3: Inhibitor resistance of the variant DNA polymerase

The resistance of a variant DNA polymerase to inhibitors such as blood is determined by adding increasing concentrations of the inhibitor into the DNA polymerase assay and determining the change, if any, in the apparent specific activity of the protein. The DNA polymerase assay was performed as described in Example 1 at 65°C.

Another inhibitor of DNA polymerase is dUTP which is used to prevent carryover contamination in isothermal amplification by replacing dTTP. In this case it is desirable for the polymerase to be insensitive to dUTP inhibition so as to utilize dUTP as a substrate for LAMP. Figure 4E shows that the mutant polymerase can efficiently utilize dUTP while the wild type Bst polymerase is inhibited by substituting dTTP with dUTP in the amplification reaction.

### Example 4: Increased resistance to high salt concentration

The resistance of a variant DNA polymerase to increased salt concentration was determined by adding increasing concentrations of salt (for example, KCI or NaCl) to the DNA polymerase assay described in Example 1 and determining the activity of the protein at 65°C and comparing its activity to parent Bst DNA polymerase (see Figure 4B).

### Example 5: Increased stability in storage

The stability of a variant DNA polymerase during storage was determined by incubating the enzyme in storage buffer (10 mMTris-HCl pH 7.5, 50 mM KCI, 1 mM Dithiothreitol, 0.1 mM EDTA, 50% Glycerol, 0.1% Triton X-100) at a temperature ranging from 4°C to 65°C for a time period ranging from 1 day to 28 weeks, and assaying DNA polymerase activity remaining after storage using the LAMP method described in Example 1. The remaining activity was compared to a sample stored at -20°C for the same amount of time. The stability of the variant was then compared to the stability of parent Bst DNA polymerase (See Figure 4D). When this period was extended to 60 weeks, no detectable loss of activity of the mutants was observed even in the absence of glycerol.

Example 6: Assay for determining the melting temperature of a variant polymerase for comparison with a parent DNA polymerase using a SYPRO Orange assay. The assay was performed as follows: Each 50 µl reaction contains 1x ThermoPol Buffer, detergent-free (20 mM Tris-HCl pH 8.8, 10 mM (NH₄)₂SO₄, 10 mM KCI, 2 mM MgSO4, 1x SYPRO Orange protein gel stain, and DNA polymerase concentrations ranging from 2.2 to 17.5 µg (parent BstLF mutant) or 0.6 to 4.8 µg (parent Bst FL mutant). The reactions were placed in a CFX96 Real-Time System. The temperature was raised 1°C per second from 20 to 100°C, and the fluorescence (in the FRET channel) was read at each temperature. Here, the Tm is the inflection point of the sigmodial curve of fluorescence plotted against temperature. The inverted first derivative of the fluorescence emission in Figure 3A and 3B is shown in relation to temperature, where the location of the minima corresponded to the value of the Tm (see Figure 3).

### Example 7: Whole genome amplification using a variant Bst DNA polymerase.

The variant DNA polymerase can be tested for suitability in whole genome amplification using the methods termed hyperbranched strand displacement amplification (Lage, et al., Genome Research, 13 (2):294-307 (2003)) or multiple-strand displacement amplification (Aviel-Ronen, et al., BMC Genomics, 7:312 (2006)).

### Example 8: DNA sequencing on a semiconductor device using a variant DNA polymerase

The variant DNA polymerase can be tested for its suitability in DNA sequencing, for example, as described in Rothberg, et al., Nature, 475(7356):348-352(2011), an integrated semiconductor device enabling non-optical genome sequencing.

Example 9: Solid-phase DNA amplification using a variant polymerase. Variant DNA polymerase can be tested for its suitability in solid-phase DNA amplification, for example as described in (Adessi, et al., Nucleic Acids Research, 28:E87 (2000), which describes a method for the amplification of target sequences with surface bound oligonucleotides.

### Example 10: Enhanced reverse transcriptase activity

The reverse activity of the mutant Bst DNA polymerase was determined using a two-step RT-qPCR assay (Sambrook, et al., Molecular Cloning -A Laboratory Manual, 3rd ed., Cold Harbor Laboratory Press (2001)). The first step was for cDNA synthesis using the mutant enzymes and various traditional reverse transcriptases. The second measures the amount of synthesized cDNA by qPCR. The RT step was performed using 6uM Hexamer (Random Primer Mix, New England Biolabs, Ipswich, MA) as primers in Isothermal Amplification Buffer (New England Biolabs, Ipswich, MA) supplemented with 6mM Mg and 200uM dNTP with 0.1 ug Jurkat Total RNA (Life Technologies, Carlsbad, CA) and incubated at 65°C for 20 minutes. 1 ul of the RT product was added to qPCR reaction for GAPDH gene with 200nM of forward (5'-AGAACGGGAAGCTTGTCATC) (SEQ ID NO:146) and reverse primer (5'-CGAACATGGGGGCATCAG) (SEQ ID NO:147), 200uM dNTP, 1.25 unit of Taq DNA polymerase in 25ul of 1x Standard Taq Buffer (New England Biolabs, Ipswich, MA) containing 2 uM of dsDNA-binding fluorescent dye SYTO®9 (Life Technologies, Carlsbad, CA). The PCR cycles were: 95°C for 1 minute, then 50 cycles at 95°C for 10 seconds, 61°C for 15 seconds and 68°C for 30 seconds, and a final step of 68°C for 5 minutes. The PCR was performed on a CFX96 Real-Time PCR machine and the Cq value was obtained as an indication of the amount of specific cDNA being synthesized (Figure 5A). Mutant 1 and mutant 2 (4^{th} and 5^{th} bar from left in bar chart) make abundant cDNA as indicated by having Cq values similar to that of traditional RTs (6^{TH}, 7^{th}, and 8^{th} bar from left) in qPCR. Wild type BstLF (3rd bar from the left) is the same as controls (1st and 2^{nd} bar from left) without RT. After completion of the PCR reaction, 10ul of PCR product was analyzed by electrophoresis in a 1.5% agarose gel (Figure 5B) to verify the size of the PCR product. The lanes from left to right are primer alone, RNA alone, BstLF, mutant 1, mutant 2, Rtx, AMV and MMLV. Mutant 1, mutant 2 and all RTs (Rtx, AMV and MMLV) lanes gave a band of expected size (207 base pairs) but no specific band with wild type BstLF or controls. These results demonstrate that mutant 1 and mutant 2 has much improved Rtx activity compared to wild type BstLF.

## Claims

1. A variant protein, comprising an amino acid sequence with at least 90% but less than 100% sequence identity to any of SEQ ID NOs: 1-23, wherein the variant protein further comprises a mutated amino acid having a position corresponding to M316 of SEQ ID NO: 1, where the amino acid at this position is mutated to R;
and wherein the variant protein has one or more improved properties for isothermal amplification compared with SEQ ID NO: 1, wherein the one or more improved properties of the variant protein comprises an increased salt tolerance in the range of 20 mM - 200 mM monovalent salt.

2. A variant protein according to Claim 1, further comprising at least one mutated amino acid having a position corresponding to SEQ ID NO: 1 selected from the group of mutated amino acids consisting of (a)-(f), where the mutations in (a)-(f) are:
(a) A1 E, G3(K, E or D), K5(L, A or V), E8(M or A), E9D, M10I, A13(D, E, T or V), I14D, V15A, V17(T, E or G), I18V;
(b) E20(M or D) M34(Q or L), E36D, I46F, L48(N or I), M57(L or I), P59(T or A), T61L, D65S, S66(F, E or P);
(c) Q67A, L69(V or K), A73E, M81V, A84R, V88(A or I), R99V, A102D, N113A, D117(T, S or A), A118D, G119(D or E), I121(A or V), V124K, E131H, S135(E or P), V144A, S147(P or A), L148(D or V), Q152(L or P);
(d) T153(A or V), Q170(R or E), M173(L or I), D175E, N178(E, K or R), Q183(L, E or R), L185F, T186(L or I), K187(E or D), Q190(L or M);
(e) A193(I or S), A194(L, S or T), N205(D or K), S216(L or E), R223(V, K or G), A224E, I225(Q or V), V247L, R307H; and
(f) A330T, D357L, D378N, D380E, 1383A, Q387R, L390M; I400V, E406D, A410S, N411R, A433S, N437G, T439K, A452E, Q459(R or E), N463(V, E or D), L484D, D486E, V494L, T501M, Q530R, I552M, E557(K, Q or R) and T568(E or R).

3. A variant protein according to Claim 1 or Claim 2, comprising at least one amino acid motif or at least two amino acid motifs selected from the group consisting of: 3..EEK..5, 15..ADE..17, 65..SPQ..67, 86..RAI..88, 185..LTE..187, 186..TEL..188, 222..LKE..224, 306..VHP..308, 314...HTR..316, 555..LCK..557, 556..CKL..558 and 567..VEL..569.

4. A variant protein according to Claim 1 containing at least 12 artificially introduced single amino acid mutations that occur in a three amino acid motif that differs from an amino acid in the corresponding site of a naturally occurring Bst polymerase.

5. A variant protein according to any one of Claims 1-4, wherein a peptide is fused to one end of the variant protein either directly or by means of a linker.

6. An enzyme preparation comprising a variant protein according to any one of Claims 1-5, and a buffer;
optionally wherein the enzyme preparation further comprises a reversible inhibitor of polymerase activity or wherein the enzyme preparation further comprises dNTPs.

7. A DNA encoding the variant protein according to any one of Claims 1-5.

8. A host cell comprising the DNA according to Claim 7.

9. A method for synthesizing a variant protein according to any one of Claims 1-5, comprising: expressing the DNA of Claim 7 in a transformed host cell.

10. A method for preparing a fusion protein, comprising:
(a) selecting a variant protein according to any one of Claims 1-4 having an amino acid sequence; and
(b) expressing the variant protein as a fusion protein with an additional peptide at an end of the amino acid sequence.

11. A method of isothermal amplification of a target DNA, comprising:
(a) combining the target DNA with a preparation comprising a variant protein according to any one of Claims 1-5; and
(b) amplifying the target DNA at a temperature less than 90°C.

12. A method for reverse transcribing an RNA of interest, comprising combining an RNA with a variant protein according to any one of Claims 1-5 or a preparation according to Claim 6 to form a cDNA.

13. A method for amplifying DNA, comprising combining a target DNA with a variant protein according to any one of Claims 1-5 or a preparation according to Claim 6, to produce amplified DNA.

14. A method according to Claim 13, wherein the target DNA is a cDNA formed in the reverse transcription method of Claim 12.

## Patentansprüche

1. Variantes Protein, umfassend eine Aminosäuresequenz mit einer mindestens 90%igen, aber weniger als 100%igen Sequenzidentität mit jedweder von SEQ ID NOs: 1 bis 23, wobei das variante Protein weiter eine mutierte Aminosäure mit einer Position umfasst, die M316 von SEQ ID NO: 1 entspricht, wobei die Aminosäure an dieser Position in R mutiert ist;
und wobei das variante Protein eine oder mehr verbesserte Eigenschaft(en) zur isothermalen Amplifikation verglichen mit SEQ ID NO: 1 aufweist, wobei die eine oder mehr verbesserte(n) Eigenschaft(en) des varianten Proteins eine erhöhte Salztoleranz im Bereich von 20 mM bis 200 mM monovalentem Salz umfasst/umfassen.

2. Variantes Protein nach Anspruch 1, weiter umfassend mindestens eine mutierte Aminosäure mit einer Position, die SEQ ID NO: 1 entspricht, ausgewählt aus der Gruppe von mutierten Aminosäuren, bestehend aus (a) bis (f), wobei die Mutationen in (a) bis (f) wie folgt sind:
a) A1E, G3(K, E oder D), K5(L, A oder V), E8(M oder A), E9D, M10I, A13(D, E, T oder V), I14D, V15A, V17(T, E oder G), I18V;
b) E20(M oder D) M34(Q oder L), E36D, I46F, L48(N oder I), M57(L oder I), P59(T oder A), T61L, D65S, S66(F, E oder P);
c) Q67A, L69(V oder K), A73E, M81V, A84R, V88(A oder I), R99V, A102D, N113A, D117(T, S oder A), A118D, G119(D oder E), I121(A oder V), V124K, E131H, S135(E oder P), V144A, S147(P oder A), L148(D oder V), Q152(L oder P);
d) T153(A oder V), Q170(R oder E), M173(L oder I), D175E, N178(E, K oder R), Q183(L, E oder R), L185F, T186(L oder I), K187(E oder D), Q190(L oder M);
e) A193(I oder S), A194(L, S oder T), N205(D oder K), S216(L oder E), R223(V, K oder G), A224E, 1225(Q oder V), V247L, R307H; und
f) A330T, D357L, D378N, D380E, I383A, Q387R, L390M; I400V, E406D, A410S, N411R, A433S, N437G, T439K, A452E, Q459(R oder E), N463(V, E oder D), L484D, D486E, V494L, T501M, Q530R, I552M, E557(K, Q oder R) und T568(E oder R).

3. Variantes Protein nach Anspruch 1 oder Anspruch 2, umfassend mindestens ein Aminosäuremotiv oder mindestens zwei Aminosäuremotive, ausgewählt aus der Gruppe, bestehend aus: 3..EEK..5, 15..ADE..17, 65..SPQ..67, 86..RAI..88, 185..LTE..187, 186..TEL..188, 222..LKE..224, 306..VHP..308, 314..HTR..316, 555..LCK..557, 556..CKL..558 und 567..VEL..569.

4. Variantes Protein nach Anspruch 1, enthaltend mindestens 12 artifiziell eingeführte einzelne Aminosäuremutationen, die in einem 3-Aminosäuren-Motiv auftreten, das sich von einer Aminosäure an der entsprechenden Stelle von einer natürlich vorkommenden Bst-Polymerase unterscheidet.

5. Variantes Protein nach einem der Ansprüche 1 bis 4, wobei ein Peptid an ein Ende des varianten Proteins entweder direkt oder mittels eines Linkers fusioniert ist.

6. Enzympräparation, umfassend ein variantes Protein nach einem der Ansprüche 1 bis 5 und einen Puffer;
gegebenenfalls wobei die Enzympräparation weiter einen reversiblen Inhibitor von Polymeraseaktivität umfasst oder wobei die Enzympräparation weiter dNTPs umfasst.

7. DNA, kodierend für das variante Protein nach einem der Ansprüche 1 bis 5.

8. Wirtszelle, umfassend die DNA nach Anspruch 7.

9. Verfahren zum Synthetisieren eines varianten Proteins nach einem der Ansprüche 1 bis 5, umfassend: Expression der DNA nach Anspruch 7 in einer transformierten Wirtszelle.

10. Verfahren zur Präparation eines Fusionsproteins, umfassend:
a) Auswahl eines varianten Proteins nach einem der Ansprüche 1 bis 4 mit einer Aminosäuresequenz; und
b) Expression des varianten Proteins als ein Fusionsprotein mit einem zusätzlichen Peptid an einem Ende der Aminosäuresequenz.

11. Verfahren zur isothermalen Amplifikation einer Ziel-DNA, umfassend:
a) Kombination der Ziel-DNA mit einer Präparation, umfassend ein variantes Protein nach einem der Ansprüche 1 bis 5; und
b) Amplifikation der Ziel-DNA bei einer Temperatur von weniger als 90 °C.

12. Verfahren zur reversen Transkription einer RNA von Interesse, umfassend die Kombination einer RNA mit einem varianten Protein nach einem der Ansprüche 1 bis 5 oder eine Präparation nach Anspruch 6 zur Bildung einer cDNA.

13. Verfahren zur Amplifikation von DNA, umfassend die Kombination einer Ziel-DNA mit einem varianten Protein nach einem der Ansprüche 1 bis 5 oder einer Präparation nach Anspruch 6, zur Herstellung von amplifizierter DNA.

14. Verfahren nach Anspruch 13, wobei die Ziel-DNA eine mittels des reversen Transkriptionsverfahrens nach Anspruch 12 gebildete cDNA ist.

## Revendications

1. Protéine variante, comprenant une séquence d'acides aminés ayant une identité de séquence d'au moins 90 % mais de moins de 100 % avec l'une quelconque des SÉQ. ID n^{os} 1 à 23, la protéine variante comprenant en outre un acide aminé ayant subi une mutation ayant une position correspondant à M316 de la SÉQ. ID n° 1, la mutation entraînant un remplacement de l'acide aminé à cette position par R ;
et la protéine variante présentant une ou plusieurs propriétés améliorées pour l'amplification isotherme par comparaison avec la SÉQ. ID n° 1, lesdites une ou plusieurs propriétés améliorées de la protéine variante comprenant une tolérance accrue au sel dans la plage de 20 mM à 200 mM de sel monovalent.

2. Protéine variante selon la revendication 1, comprenant en outre au moins un acide aminé ayant subi une mutation ayant une position correspondant à la SÉQ. ID n° 1 sélectionné dans le groupe d'acides aminés ayant subi une mutation consistant en (a)-(f), les mutations en (a)-(f) tétant :
a) A1E, G3(K, E ou D), K5(L, A ou V), E8(M ou A), E9D, M10I, A13(D, E, T ou V), I14D, V15A, V17(T, E ou G), I18V ;
b) E20(M ou D), M34(Q ou L), E36D, I46F, L48(N ou I), M57(L ou I), P59 (T ou A), T61L, D65S, S66(F, E ou P) ;
c) Q67A, L69(V ou K), A73E, M81V, A84R, V88(A ou I), R99V, A102D, N113A, D117(T, S ou A), A118D, G119(D ou E), I121(A ou V), V124K, E131H, S135(E ou P), V144A, S147(P ou A), L148(D ou V), Q152(L ou P) ;
d) T153(A ou V), Q170(R ou E), M173(L ou I), D175E, N178(E, K ou R), Q183(L, E ou R), L185F, T186(L ou I), K187(E ou D), Q190(L ou M) ;
e) A193(I ou S), A194(L, S ou T), N205(D ou K), S216(L ou E), R223(V, K ou G), A224E, 1225(Q ou V), V247L, R307H ; et
f) A330T, D357L, D378N, D380E, I383A, Q387R, L390M ; I400V, E406D, A410S, N411R, A433S, N437G, T439K, A452E, Q459(R ou E), N463(V, E ou D), L484D, D486E, V494L, T501M, Q530R, I552M, E557(K, Q ou R) et T568(E ou R).

3. Protéine variante selon la revendication 1 ou la revendication 2, comprenant au moins un motif d'acides aminés ou au moins deux motifs d'acides aminés sélectionnés dans le groupe constitué de : 3..EEK..5, 15..ADE..17, 65..SPQ..67, 86..RA1..88, 185..LTE..187, 186..TEL..188, 222..LKE..224, 306..VHP..308, 314...HTR..316, 555..LCK..557, 556..CKL..558 et 567..VEL..569.

4. Protéine variante selon la revendication 1, contenant au moins 12 mutations simples d'acides aminés introduites artificiellement qui ont lieu dans un motif à trois acides aminés qui diffère d'un acide aminé dans le site correspondant d'une Bst polymérase naturelle.

5. Protéine variante selon l'une quelconque des revendications 1 à 4, dans laquelle un peptide est fusionné à une extrémité de la protéine variante, soit directement, soit au moyen d'un lieur.

6. Préparation enzymatique comprenant une protéine variante selon l'une quelconque des revendications 1 à 5 et un tampon ;
la préparation enzymatique comprenant optionnellement en outre un inhibiteur réversible de l'activité de la polymérase, ou la préparation enzymatique comprenant en outre des dNTP.

7. ADN codant la protéine variante selon l'une quelconque des revendications 1 à 5.

8. Cellule hôte comprenant l'ADN selon la revendication 7.

9. Procédé de synthèse d'une protéine variante selon l'une quelconque des revendications 1 à 5, comprenant l'expression de l'ADN selon la revendication 7 dans une cellule hôte transformée.

10. Procédé de préparation d'une protéine de fusion, comprenant :
a) la sélection d'une protéine variante selon l'une quelconque des revendications 1 à 4 ayant une séquence d'acides aminés ; et
b) l'expression de la protéine variante en tant que protéine de fusion comportant un peptide additionnel à une extrémité de la séquence d'acides aminés.

11. Procédé d'amplification isotherme d'un ADN cible, comprenant :
a) la combinaison de l'ADN cible avec une préparation comprenant une protéine variante selon l'une quelconque des revendications 1 à 5 ; et
b) l'amplification de l'ADN cible à une température inférieure à 90 °C.

12. Procédé de transcription inverse d'un ARN d'intérêt, comprenant la combinaison d'un ARN avec une protéine variante selon l'une quelconque des revendications 1 à 5 ou une préparation selon la revendication 6 pour former un ADNc.

13. Procédé d'amplification d'ADN, comprenant la combinaison d'un ADN cible avec une protéine variante selon l'une quelconque des revendications 1 à 5 ou une préparation selon la revendication 6, pour produire un ADN amplifié.

14. Procédé selon la revendication 13, dans lequel l'ADN cible est un ADNc formé dans le procédé de transcription inverse selon la revendication 12.
